(19) Europäisches Patentamt / European Patent Office / Office européen des brevets

(11) EP 3 868 889 A1

(12) **EUROPEAN PATENT APPLICATION**
published in accordance with Art. 153(4) EPC

(43) Date of publication:
**25.08.2021 Bulletin 2021/34**

(21) Application number: **19873862.7**

(22) Date of filing: **17.10.2019**

(51) Int Cl.:
*C12N 15/88* (2006.01) *A61K 9/127* (2006.01)
*A61K 9/14* (2006.01) *A61K 31/7088* (2006.01)
*A61K 35/17* (2015.01) *A61K 39/395* (2006.01)
*A61K 45/00* (2006.01) *A61K 47/68* (2017.01)
*A61K 48/00* (2006.01) *A61P 43/00* (2006.01)
*C12N 5/0783* (2010.01) *A61P 35/00* (2006.01)
*C07K 16/28* (2006.01) *C12N 15/113* (2010.01)
*C12N 15/12* (2006.01)

(86) International application number:
**PCT/JP2019/040937**

(87) International publication number:
**WO 2020/080475 (23.04.2020 Gazette 2020/17)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **18.10.2018 JP 2018197069**
**03.07.2019 JP 2019124629**

(71) Applicant: **Takeda Pharmaceutical Company Limited**
**Osaka-shi, Osaka 541-0045 (JP)**

(72) Inventors:
- **KUWAE, Shinobu**
  **Fujisawa-shi, Kanagawa 251-0012 (JP)**
- **MATSUMOTO, Satoru**
  **Fujisawa-shi, Kanagawa 251-0012 (JP)**
- **HAYASHI, Akira**
  **Fujisawa-shi, Kanagawa 251-0012 (JP)**
- **KASSAI, Yoshiaki**
  **Fujisawa-shi, Kanagawa 251-0012 (JP)**
- **NAKAYAMA, Kazuhide**
  **Fujisawa-shi, Kanagawa 251-0012 (JP)**

(74) Representative: **Jones, Nicholas Andrew**
**Withers & Rogers LLP**
**4 More London Riverside**
**London, SE1 2AU (GB)**

# (54) METHOD FOR ACTIVATION/PROLIFERATION OF T CELLS

(57) The present invention provides a method for activating/proliferating T cells, including a step of contacting a cell population containing T cells with a nucleic acid delivery carrier having at least one kind of T cell activating ligand added to its surface, and a method for delivering a nucleic acid into T cells, the methods including a step of contacting a cell population containing T cells simultaneously with (a) a nucleic acid delivery carrier having at least one kind of T cell activating ligand added to its surface and containing a nucleic acid inside, or (b) at least one kind of T cell activating ligand, and a nucleic acid delivery carrier containing a nucleic acid inside and free of a T cell activating ligand added to its surface, a method for producing a medicament containing T cells and the like.

EP 3 868 889 A1

## Description

[Technical Field]

The present invention relates to a nucleic acid delivery carrier with a T cell activating ligand added to the surface, a method for activating and/or proliferating T cells by using the nucleic acid delivery carrier, a method for delivering a nucleic acid into T cells, and the like. The present invention also relates to a method for activating and/or proliferating T cells and a method for delivering a nucleic acid into T cells, each characteristically including bringing a T cell activating ligand and a nucleic acid delivery carrier into simultaneous contact with T cells.

(Background of the Invention)

The research and development of cancer immunotherapy using CAR-T cells or TCR-T cells introduced with a gene of chimeric antigen receptor (CAR) or T-cell receptor (TCR) derived from cancer antigen-specific killer T cell is progressing rapidly. Current CAR-T cell therapy, such as Kymriah (trade name) and Yescarta (trade name), which were approved in the U.S., generally includes producing CAR-T cells by introducing CAR genes into T cells collected from patients ex vivo using virus vectors such as lentivirus vector, and administering the CAR-T cells to the patients. However, this method has the problem that the production cost becomes high due to the cost of cell culture and preparation of virus vectors because multiple steps are necessary over a long period of time such as activation/proliferation of T cells, preparation of virus vectors, gene transfer into T cells, and the like.

As a method for introducing CAR into T cells without using a virus vector, ex vivo or in vivo transfection of CAR into T cells has been reported which uses nanoparticles containing aggregates of CAR-encoding plasmid DNA and a cationic polymer that are coated with a non-cationic polymer conjugated with anti-CD3 antibody fragments (patent document 1, non-patent document 1), or nanocarrier containing mesoporous silica encapsulating CAR-encoding DNA in the pores and coated with a lipid having a surface modified with an anti-CD3 antibody (patent document 2).

Apart therefrom, techniques have been reported for delivering siRNA to a target cell by encapsulating the siRNA of interest in "lipid nanoparticles (LNP)", which do not have an internal pore structure and are composed of a cationic lipid, a non-cationic helper lipid, and a ligand for delivery to the target cell. For example, ex vivo or in vivo transfection of siRNA for CD45 into T cells by using an anti-CD4 antibody fragment as a targeted ligand has been reported (patent document 3, non-patent document 2).

In addition, patent document 4 describes a cationic lipid for introducing an active ingredient such as a nucleic acid or the like into various cells including T cell, tissues and organs.

On the other hand, as a method for activating/proliferating T cells, a method for activating and/or proliferating T cells using beads on which anti-CD3/CD28 antibody is immobilized or nano-sized matrix beads has been reported (patent documents 5 and 6).

However, no technique has been reported heretofore in which the step of activating/proliferating T cells and the step of introducing a gene into T cells can be performed simultaneously in one pod.

[Document List]

[Patent documents]

patent document 1: US 2017/0296676
patent document 2: US 2016/0145348
patent document 3: WO 2016/189532
patent document 4: WO 2016/021683
patent document 5: US 6,352,694
patent document 6: US 2014/0087462


[Non-patent documents]

non-patent document 1: Nature Nanotechnology 12, 813-820 (2017)
non-patent document 2: ACS Nano, 2015, 9(7), 6706-6716

[Summary of Invention]

[Technical Problem]

An object of the present invention is to shorten and simplify the production process of an agent for immune cell therapy such as CAR-T therapy and the like, to provide an agent for immune cell therapy in a short period of time with a low production cost, and to provide a safer production process of an agent for immune cell therapy that eliminates a potential risk of carcinogenicity due to a virus vector.

[Solution to Problem]

The present inventors have conducted intensive studies in an attempt to achieve the above-mentioned object and succeeded in simultaneously performing a step of activating/proliferating T cells and a step of introducing a gene into T cells in one pod by using a nucleic acid delivery carrier having a T cell activating ligand added to its surface. Furthermore, the present inventors have surprisingly found that activation/proliferation of T cells and introduction of nucleic acid into T cells can be efficiently achieved by simply bringing the T cell activating ligand and the nucleic acid delivery carrier into contact with the T cells at the same time, and completed the present invention.
Accordingly, the present invention provides the following.

[1] A method for activating/proliferating T cells, comprising a step of contacting a cell population containing T cells with a nucleic acid delivery carrier having at least one kind of T cell activating ligand added to its surface.
[2] The method of [1], wherein the aforementioned T cell activating ligand includes an antibody to CD3 and/or an antibody to CD28.
[3] The method of [1] or [2], wherein the aforementioned nucleic acid delivery carrier has two or more kinds of T cell activating ligands added to a surface thereof.
[4] The method of any of [1] to [3], wherein the aforementioned nucleic acid delivery carrier is a lipid nanoparticle or a liposome.
[5] The method of any of [1] to [4], wherein the aforementioned nucleic acid delivery carrier comprises, in its inside, a nucleic acid that suppresses expression of a T cell activation inhibitory factor and/or a nucleic acid encoding a T cell activation promoting factor.
[6] The method of any of [1] to [5], wherein the aforementioned nucleic acid delivery carrier comprises a nucleic acid encoding CAR or TCR.
[7] The method of any of [1] to [6], wherein the method is performed ex vivo.
[8] A method for delivering a nucleic acid into T cells, comprising a step of contacting a cell population containing T cells with a nucleic acid delivery carrier having at least one kind of T cell activating ligand added to its surface and containing a nucleic acid inside.
[9] The method of [8], wherein the aforementioned T cell activating ligand includes an antibody to CD3 and/or an antibody to CD28.
[10] The method of [8] or [9], wherein the aforementioned nucleic acid delivery carrier has two or more kinds of T cell activating ligands added to a surface thereof.
[11] The method of any of [8] to [10], wherein the aforementioned nucleic acid delivery carrier is a lipid nanoparticle or a liposome.
[12] The method of any of [8] to [11], wherein the aforementioned nucleic acid comprises a nucleic acid suppressing expression of a T cell activation inhibitory factor and/or a nucleic acid encoding a T cell activation promoting factor.
[13] The method of any of [8] to [12], wherein the aforementioned nucleic acid comprises a nucleic acid encoding CAR or TCR.
[14] The method of any of [8] to [13], wherein the method is performed ex vivo.
[15] A method for delivering a nucleic acid into T cells, comprising a step of contacting a cell population containing T cells simultaneously with at least one kind of T cell activating ligand, and a nucleic acid delivery carrier containing a nucleic acid inside and free of a T cell activating ligand added to its surface.
[16] The method of [15], wherein the aforementioned T cell activating ligand includes an antibody to CD3 and/or an antibody to CD28.
[17] The method of [15] or [16], wherein two or more kinds of T cell activating ligands are contacted.
[18] The method of any of [15] to [17], wherein the aforementioned nucleic acid delivery carrier is a lipid nanoparticle or a liposome.
[19] The method of any of [15] to [18], wherein the aforementioned nucleic acid includes a nucleic acid suppressing expression of a T cell activation inhibitory factor and/or a nucleic acid encoding a T cell activation promoting factor.
[20] The method of any of [15] to [19], wherein the aforementioned nucleic acid comprises a nucleic acid encoding

CAR or TCR.

[21] The method of any of [15] to [20], wherein the method is performed ex vivo.

[22] A method for producing a medicament comprising T cells, comprising a step of contacting a cell population containing T cells with a nucleic acid delivery carrier having at least one kind of T cell activating ligand added to its surface and containing a nucleic acid inside.

[23] The method of [22], wherein the aforementioned T cell activating ligand includes an antibody to CD3 and/or an antibody to CD28.

[24] The method of [22] or [23], wherein the aforementioned nucleic acid delivery carrier has two or more kinds of T cell activating ligands added to a surface thereof.

[25] The method of any of [22] to [24], wherein the aforementioned nucleic acid delivery carrier is a lipid nanoparticle or a liposome.

[26] The method of any of [22] to [25], wherein the aforementioned nucleic acid delivery carrier comprises a nucleic acid suppressing expression of a T cell activation inhibitory factor and/or a nucleic acid encoding a T cell activation promoting factor.

[27] The method of any of [22] to [26], wherein the aforementioned nucleic acid comprises a nucleic acid encoding CAR or TCR.

[28] The method of any of [22] to [27], wherein the method is performed ex vivo.

[29] A method for producing a medicament comprising T cells, comprising a step of contacting a cell population containing T cells simultaneously with at least one kind of T cell activating ligand, and a nucleic acid delivery carrier containing a nucleic acid inside and free of a T cell activating ligand added to its surface.

[30] The method of [29], wherein the aforementioned T cell activating ligand includes an antibody to CD3 and/or an antibody to CD28.

[31] The method of [29] or [30], wherein two or more kinds of T cell activating ligands are contacted.

[32] The method of any of [29] to [31], wherein the aforementioned nucleic acid delivery carrier is a lipid nanoparticle or a liposome.

[33] The method of any of [29] to [32], wherein the aforementioned nucleic acid delivery carrier comprises a nucleic acid suppressing expression of a T cell activation inhibitory factor and/or a nucleic acid encoding a T cell activation promoting factor.

[35] The method of any of [29] to [34], wherein the method is performed ex vivo.

[36] A nucleic acid delivery carrier having at least one kind of T cell activating ligand added to its surface.

[37] The nucleic acid delivery carrier of [36], wherein the aforementioned T cell activating ligand includes an antibody to CD3 and/or an antibody to CD28.

[38] The nucleic acid delivery carrier of [36] or [37], wherein the aforementioned nucleic acid delivery carrier has two or more kinds of T cell activating ligands added to a surface thereof.

[39] The nucleic acid delivery carrier of any of [36] to [38], wherein the aforementioned nucleic acid delivery carrier is a lipid nanoparticle or a liposome.

[40] The nucleic acid delivery carrier of any of [36] to [39], comprising, in the inside, a nucleic acid suppressing expression of a T cell activation inhibitory factor and/or a nucleic acid encoding a T cell activation promoting factor.

[41] The nucleic acid delivery carrier of any of [36] to [40], comprising, in the inside, a nucleic acid encoding CAR or TCR.

[42] A medicament comprising the nucleic acid delivery carrier of any of [36] to [41].

[43] A T cell into which a nucleic acid has been delivered by the method of any of [15] to [21].

[44] A medicament comprising the T cell of [43].

[45] A cell culture comprising a cell population containing T cells, at least one kind of T cell activating ligand, a nucleic acid delivery carrier without a T cell activating ligand added to the surface, and a medium.

[46] A composition for delivering a nucleic acid to T cells, comprising at least one kind of T cell activating ligand, and a nucleic acid delivery carrier without a T cell activating ligand added to the surface.

[47] A kit for delivering a nucleic acid into T cells, comprising at least one kind of T cell activating ligand, and a nucleic acid delivery carrier without a T cell activating ligand added to the surface.

[48] A T cell into which a nucleic acid has been delivered by the method of any of [8] to [14].

[49] A medicament comprising the T cell of [48].

[50] A cell culture comprising a cell population containing T cells, a nucleic acid delivery carrier having at least one kind of T cell activating ligand added to its surface and containing a nucleic acid inside, and a medium.

[51] A composition for delivering a nucleic acid into T cells, comprising a nucleic acid delivery carrier having at least one kind of T cell activating ligand added to its surface and containing a nucleic acid inside.

[52] A kit for delivering a nucleic acid into T cells, comprising a nucleic acid delivery carrier having at least one kind of T cell activating ligand added to its surface and containing a nucleic acid inside.

[Advantageous Effects of Invention]

According to the present invention, a step of activating/proliferating T cells and a step of introducing a gene into T cells can be performed simultaneously in one pod without using a virus vector. As a result, an agent for immune cell therapy can be provided in a short period of time with a low production cost.

[Brief Description of Drawings]

Fig. 1 shows a comparison of efficiency of gene transfer into T cells by lipid nanoparticles having an anti-CD3 antibody added to surface thereof, and containing various cationic lipids (compounds 7, 11, 12, 21, 31 and 35).
Fig. 2 shows a comparison of efficiency of gene transfer into T cells by lipid nanoparticles having an anti-CD3 antibody and/or an anti-CD28 antibody added to surface thereof.
Fig. 3 shows that gene transfer (I) into T cells and activation (II) of T cells are simultaneously achieved by lipid nanoparticles having an anti-CD3 antibody and an anti-CD28 antibody added to surface thereof. In (I) and (II), the numerical value in the upper panel shows a concentration ($\mu$g/ml) of encapsulated mRNA, and the numerical value in the lower panel shows a concentration ($\mu$g/ml) of the antibody. For comparison, (III) shows efficiency of T cell activation by beads having conventionally-known anti-CD3 antibody and anti-CD28 antibody added to surface thereof.
Fig. 4 shows that luc mRNA is efficiently introduced into human peripheral blood CD3-positive pan-T cells by co-addition of an activation stimulant and lipid nanoparticles.
Fig. 5 shows the survival and proliferation rate of T cells transfected with luc mRNA by lipid nanoparticles.
Fig. 6 shows that luc mRNA is efficiently introduced into human CD4/CD8-positive T cells by co-addition of an activation stimulant and lipid nanoparticles (left), and that the survival and proliferation rate of T cells is maintained at a high level (right).

(Detailed Description of the Invention)

1. Nucleic acid delivery carrier of the present invention

The present invention provides a nucleic acid delivery carrier having at least one kind of T cell activating ligand added to its surface (hereinafter to be also referred to as "the nucleic acid delivery carrier of the present invention").
As used herein, the "nucleic acid delivery carrier" means a carrier capable of supporting a nucleic acid and delivering the nucleic acid into a cell. Being "capable of delivering the nucleic acid into a cell" means that a nucleic acid being carried can be delivered at least into the cytoplasm of a cell.

1-1. Nucleic acid delivery carrier

The nucleic acid delivery carrier to be used in the present invention is not particularly limited in terms of the structure thereof, component molecules, and nucleic acid carrying form as long as it can support a nucleic acid and can deliver the nucleic acid into a cell, as described above. A representative drug delivery system (DDS) of nucleic acid is, for example, a complex using positively-charged cationic liposomes, cationic polymers, and the like as carriers, and formed based on the electrostatic interaction between them and nucleic acid. The complex binds to a negatively-charged cell membrane and is then incorporated into the cell by adsorptive endocytosis.
More specifically, examples of the nucleic acid delivery carrier to be used in the present invention include, but are not limited to, lipid nanoparticles (LNP), liposomes (e.g., cationic liposome, PEG-modified liposome, etc.), and cationic polymers (e.g., polyethyleneimine, polylysine, polyornithine, chitosan, atelocollagen, protamine etc.), those in which a cationic polymer is encapsulated in liposomes, and the like. Alternatively, exosome, which is a component derived from living organisms, can also be used. Preferred is lipid nanoparticle or liposome, more preferred is lipid nanoparticle.

1-1-1. Lipid nanoparticle (LNP)

In the present specification, the "lipid nanoparticle (LNP)" means a particle with an average diameter of less than 1 $\mu$m and free of a large pore structure (e.g., liposome) or a small pore structure (e.g., mesoporous material) inside the outer shell of a lipid aggregate containing cationic lipid and non-cationic lipid.
The components of the lipid nanoparticle are described below.

(a) Cationic lipid

In the present specification, the "cationic lipid" means a lipid that has a net positive charge in a low pH environment such as in physiological pH, endosome and the like. The cationic lipids used in the lipid nanoparticle used in the present invention are not particularly limited. For example, cationic lipids and the like described in WO 2016/021683, WO 2015/011633, WO 2011/153493, WO 2013/126803, WO 2010/054401, WO 2010/042877, WO 2016/104580, WO 2015/005253, WO 2014/007398, WO 2017/117528, WO 2017/075531, WO 2017/00414, WO 2015/199952, US 2015/0239834, WO2019/131839, and the like can be mentioned.

Alternatively, the synthetic cationic lipids (e.g., K-E12, H-A12, Y-E12, G-O12, K-A12, R-A12, cKK-E12, cPK-E12, PK1K-E12, PK500-E12, cQK-E12, cKK-A12, KK-A12, PK-4K-E12, cWK-E12, PK500-012, PK1K-O12, cYK-E12, cDK-E12, cSK-E12, cEK-E12, cMK-E12, cKK-O12, cIK-E12, cKK-E10, cKK-E14, and cKK-E16, preferably, cKK-E12, cKK-E14) described in Dong et al. (Proc Natl Acad Sci U S A. 2014 Apr 15; 111(15):5753), and the synthetic cationic lipids (e.g., C14-98, C18-96, C14-113, C14-120, C14-120, C14-110, C16-96 and C12-200, preferably C14-110, C16-96 and C12-200) described in Love KT et al. (Proc Natl Acad Sci U S A. 2010 May 25; 107(21):9915) can be mentioned.

In one preferred embodiment, a cationic lipid represented by the following general formula and described in WO 2016/021683 can be mentioned.

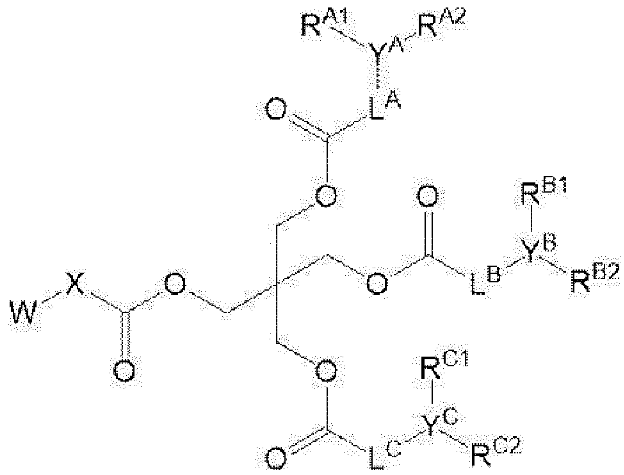

wherein

W is the formula $-NR^1R^2$ or the formula $-N^+R^3R^4R^5(Z^-)$,
$R^1$ and $R^2$ are each independently a $C_{1-4}$ alkyl group or a hydrogen atom,
$R^3$, $R^4$ and $R^5$ are each independently a $C_{1-4}$ alkyl group,
$Z^-$ is an anion,
X is an optionally substituted $C_{1-6}$ alkylene group,
$Y^A$, $Y^B$ and $Y^C$ are each independently an optionally substituted methine group,
$L^A$, $L^B$ and $L^C$ are each independently an optionally substituted methylene group or a bond, and
$R^{A1}$, $R^{A2}$, $R^{B1}$, $R^{B2}$, $R^{C1}$ and $R^{C2}$ are each independently an optionally substituted $C_{4-10}$ alkyl group, or a salt thereof.

More preferably, cationic lipids represented by the following structural formulas can be mentioned.

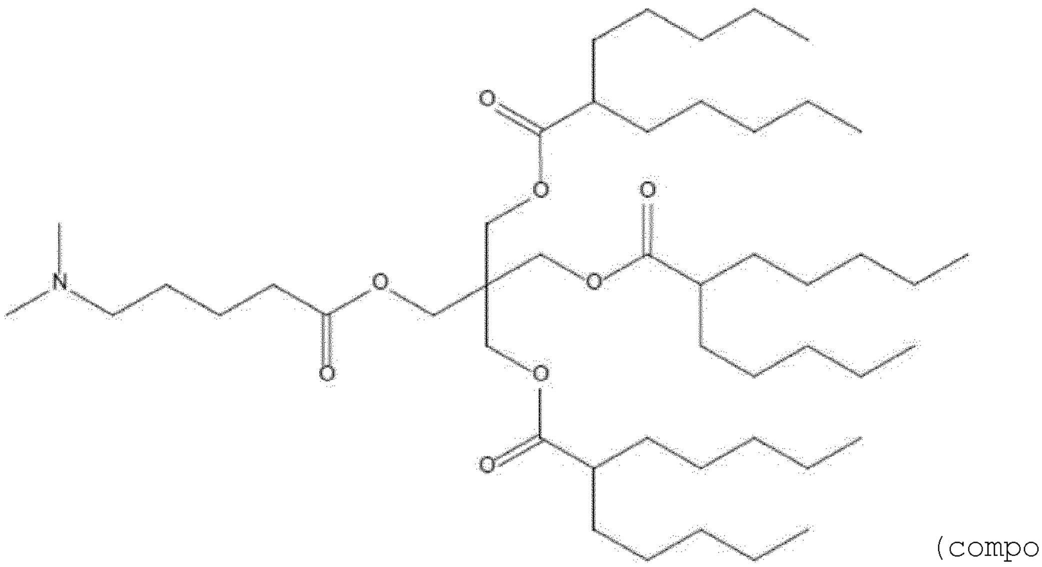

(compound 1)

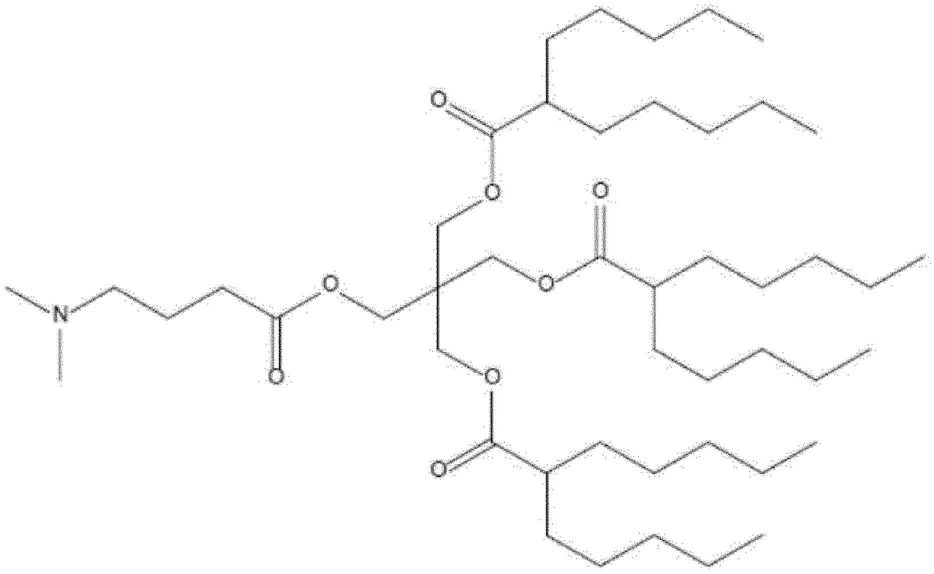

(compound 2)

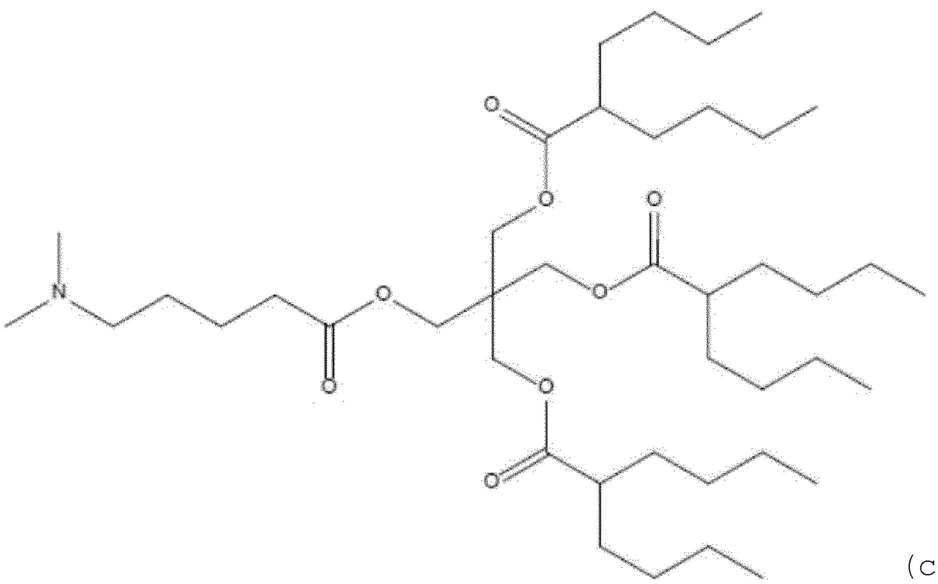

(compound 3)

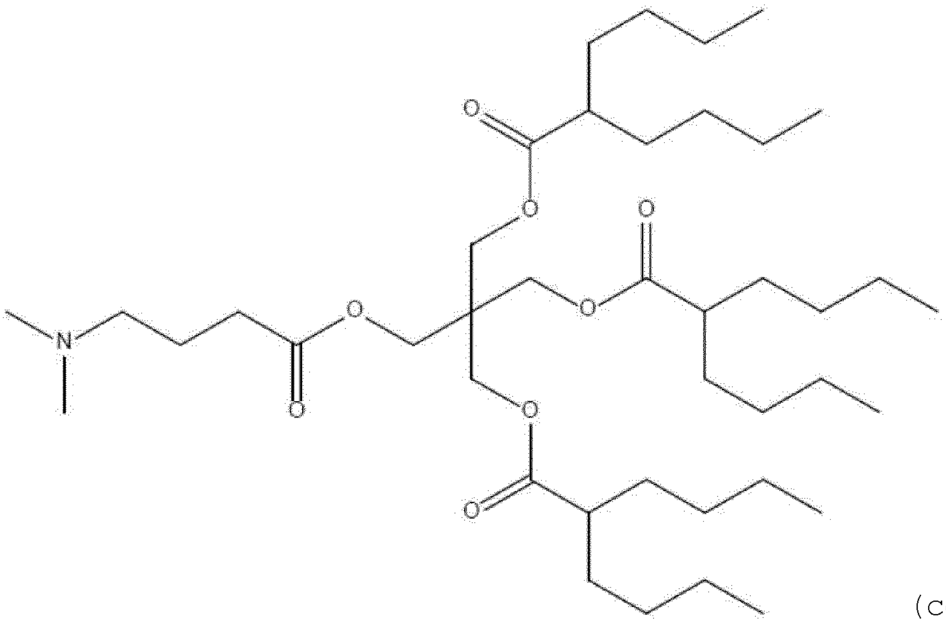

(compound 4)

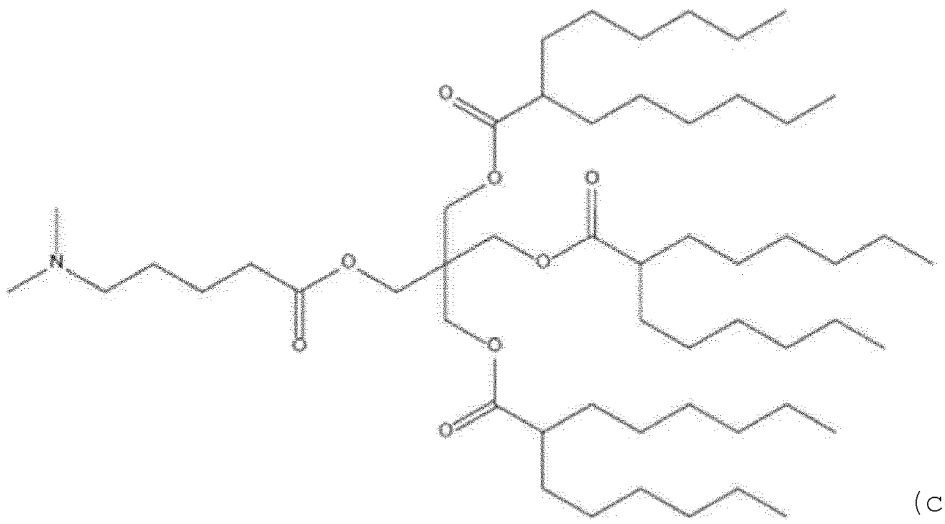

(compound 5)

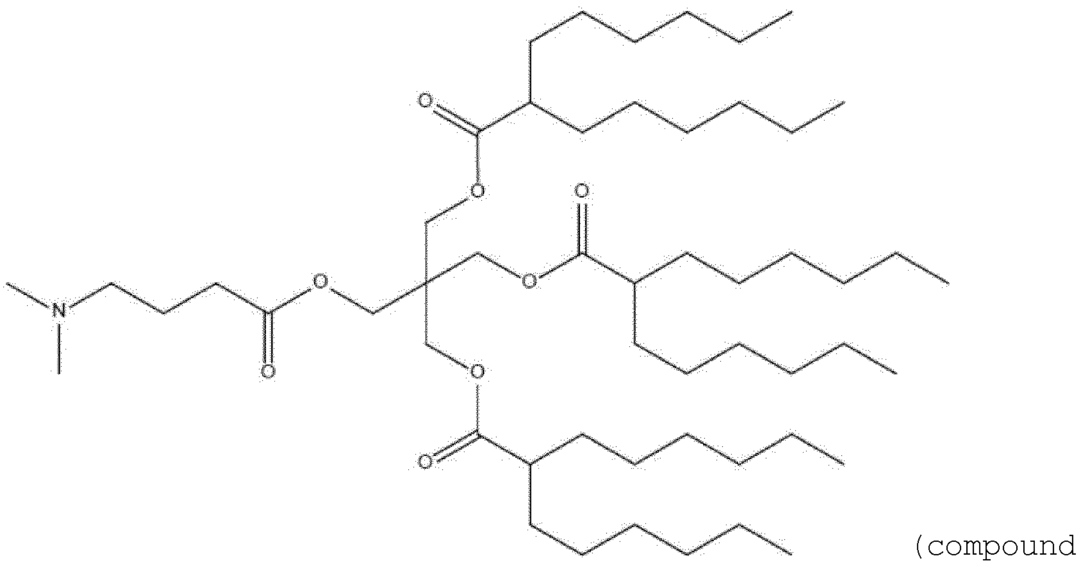

(compound 6)

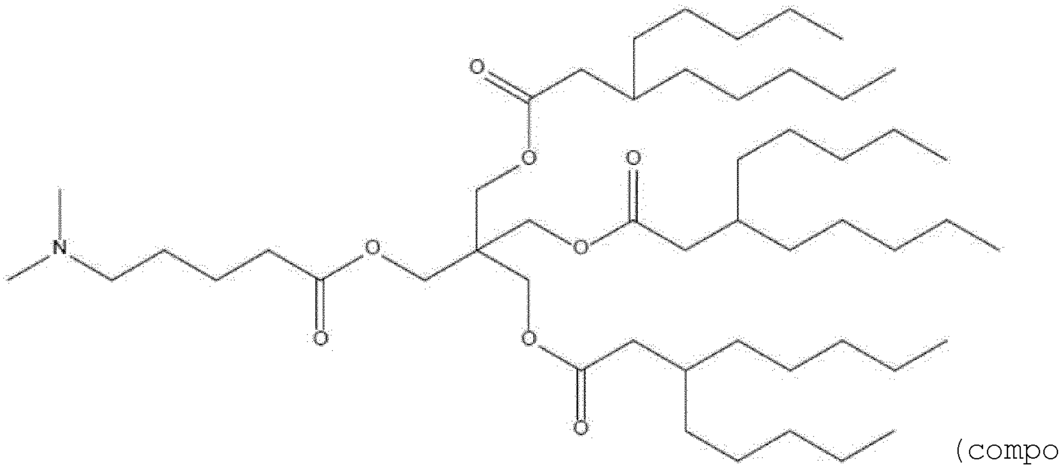

(compound 7)

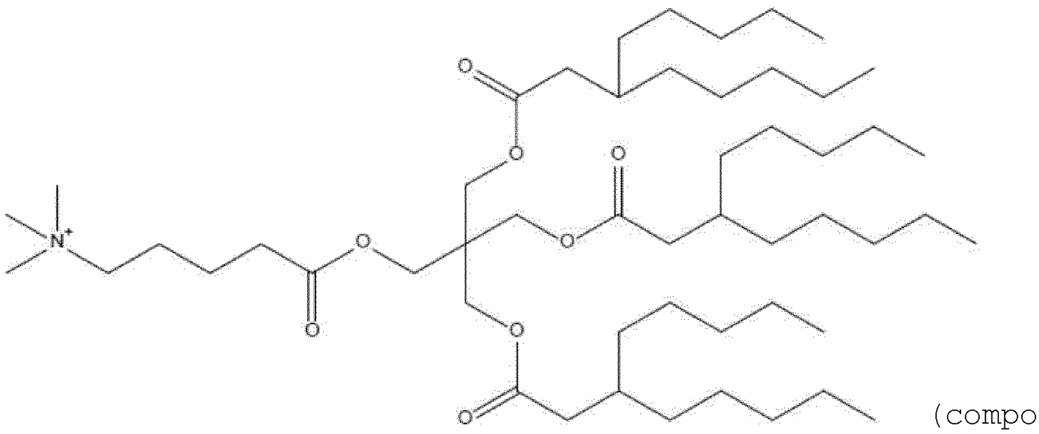

(compound 8)

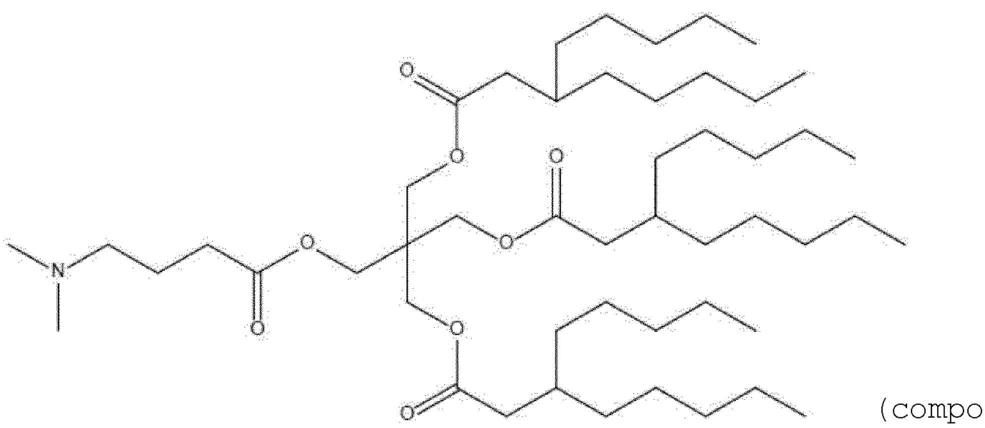

(compound 9)

and

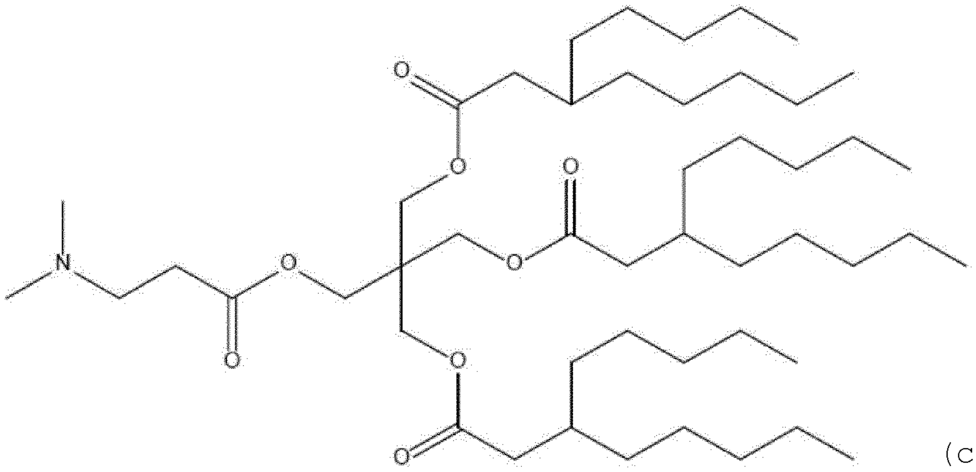

(compound 10)

and salts thereof.

Among the above-mentioned cationic lipids, more preferred cationic lipids are represented by the following structural formulas.

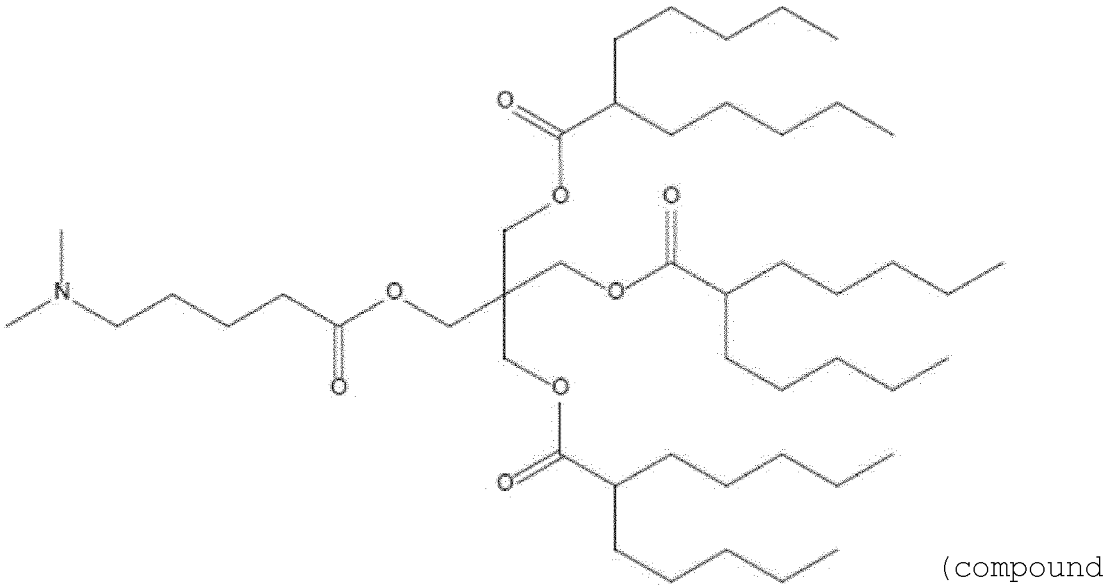

(compound 1)

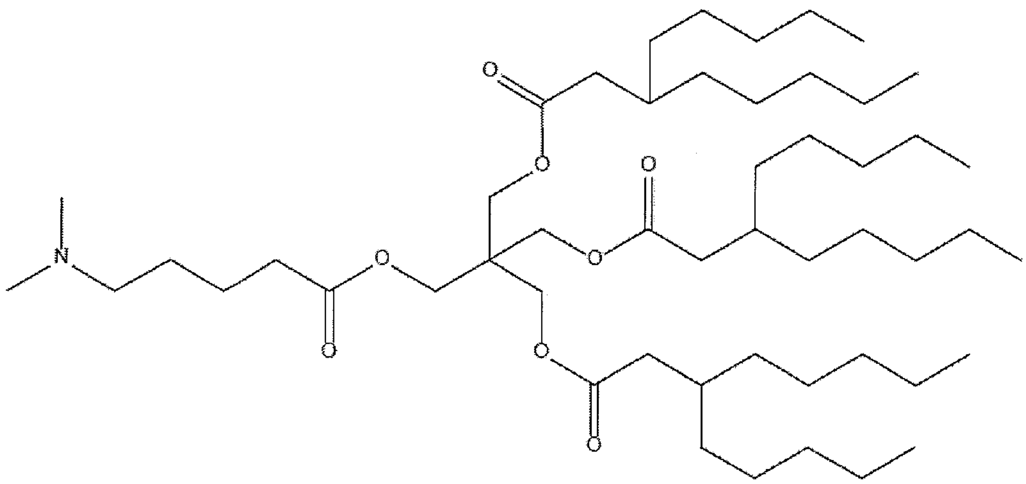

(compound 7)

and

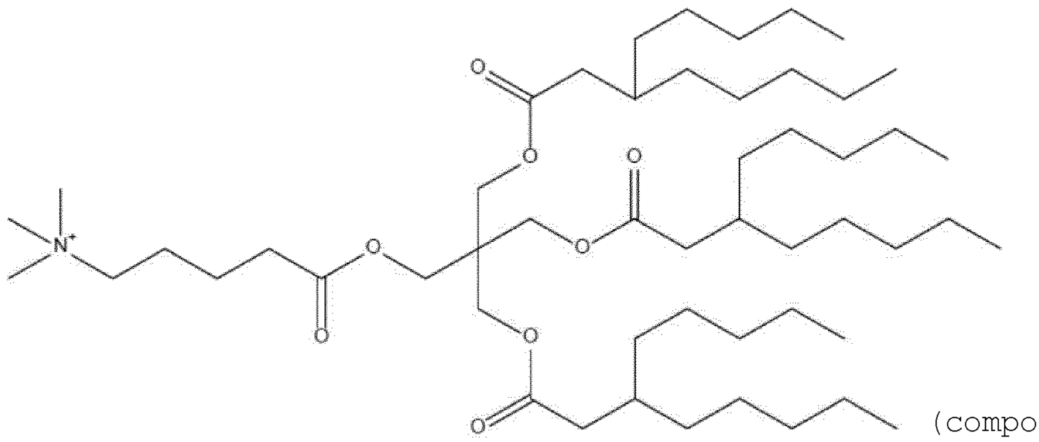

(compound 8)

and salts thereof.

In another preferred embodiment, a cationic lipid represented by the following structural formula and described in WO 2019/131839 can be mentioned.

A compound represented by

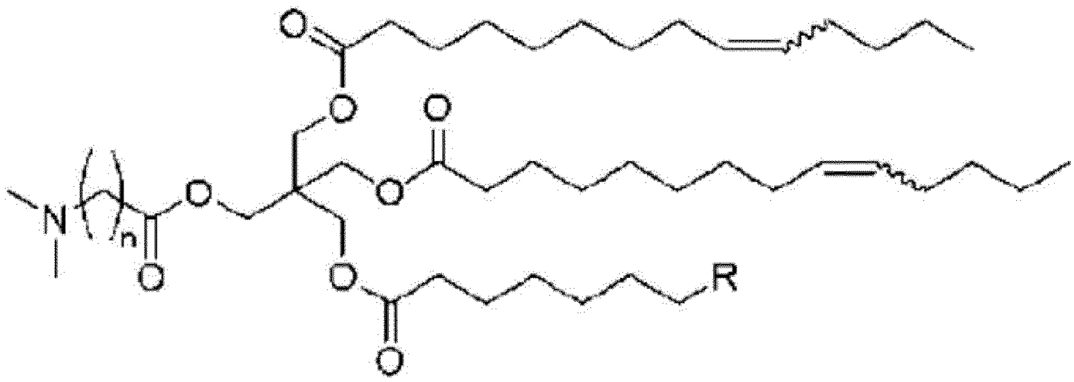

wherein

n is an integer of 2 to 5,
R is a linear $C_{1-5}$ alkyl group, a linear $C_{7-11}$ alkenyl group or a linear $C_{11}$ alkadienyl group, and
wavy lines are each independently shows a cis-type or trans-type bond,

or a salt thereof.

More preferably, cationic lipids represented by the following structural formulas can be mentioned.

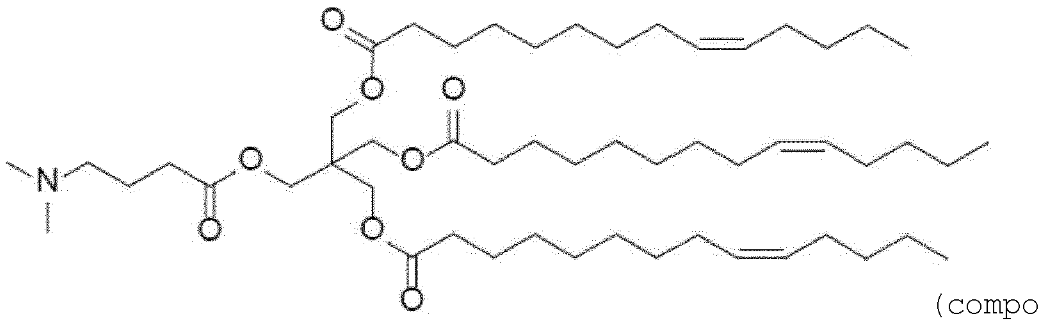

(compound 11)

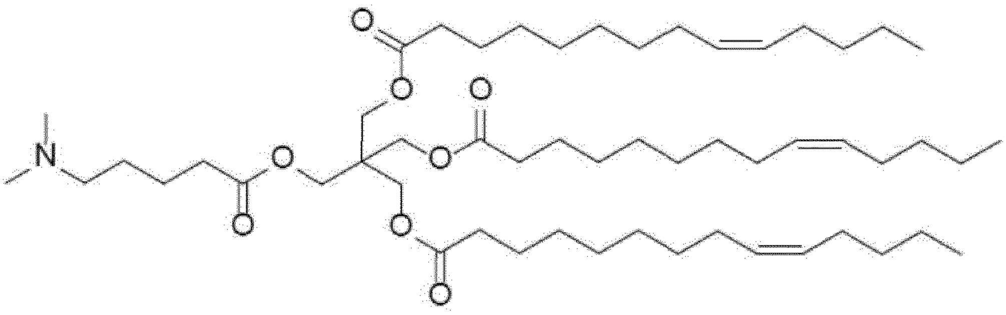

(compound 12)

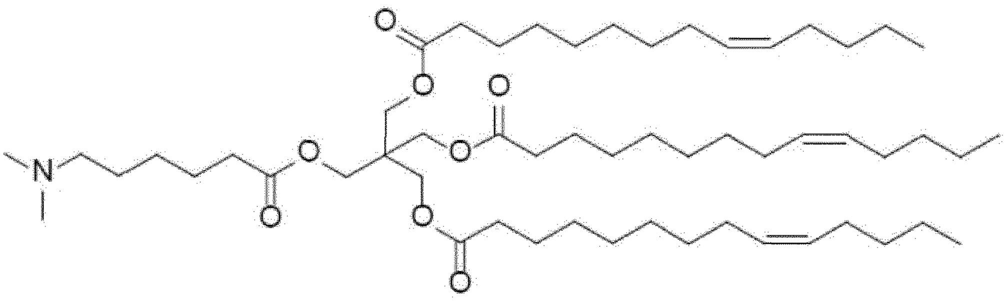

(compound 13)

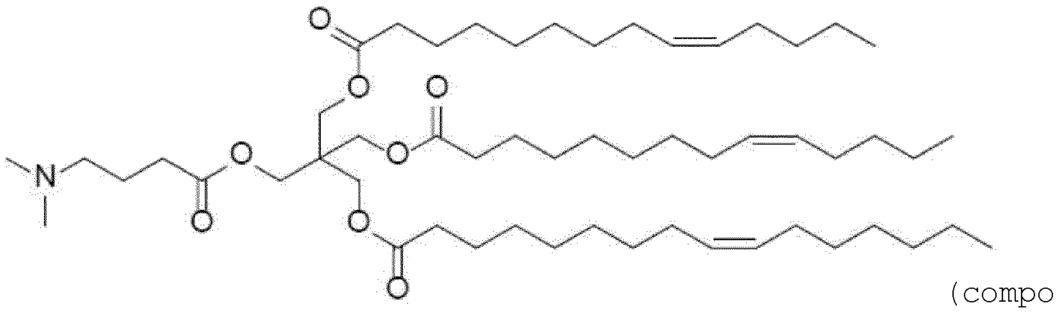

(compound 14)

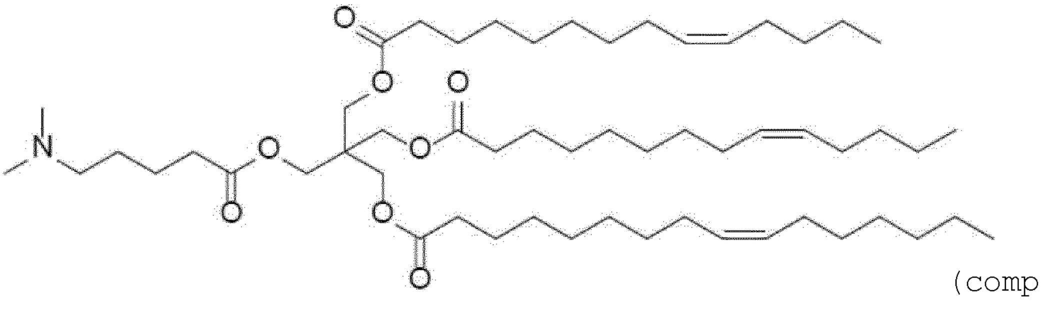

(compound 15)

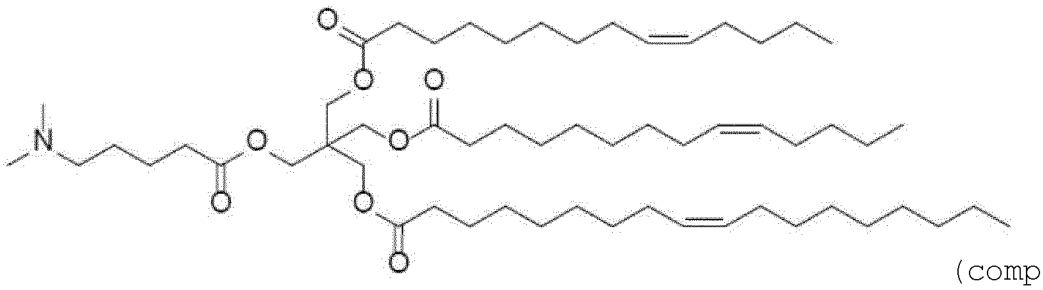

(compound 16)

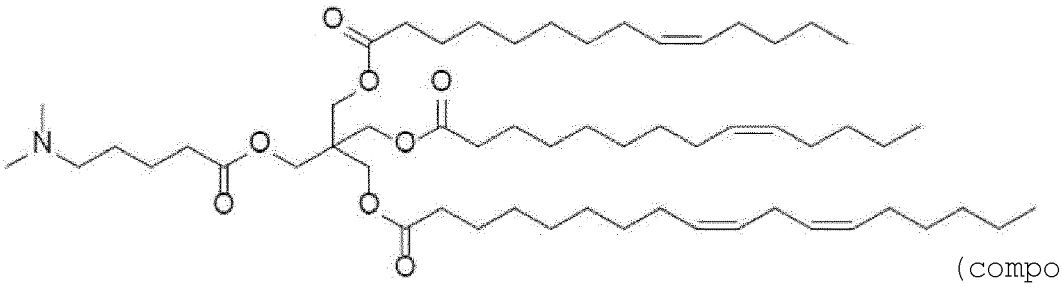

(compound 17)

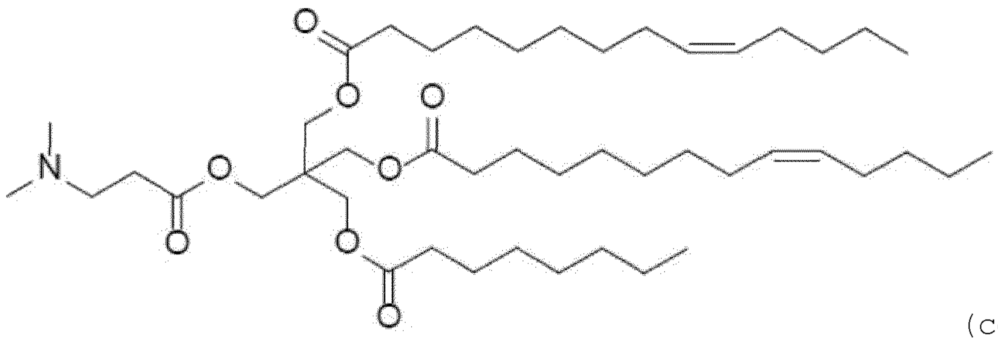

(compound 18)

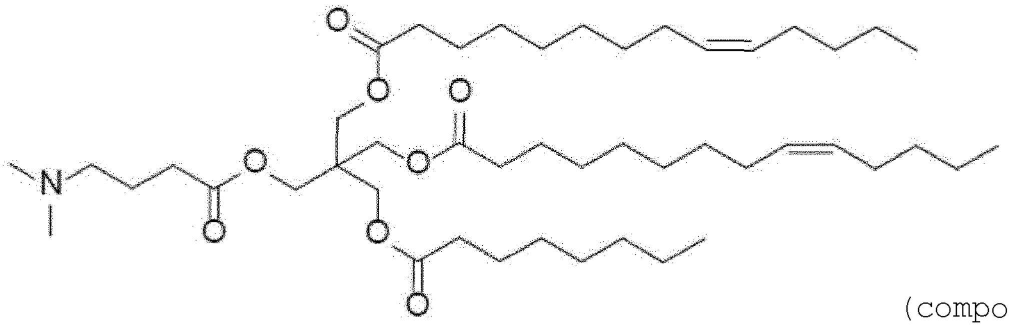

(compound 19)

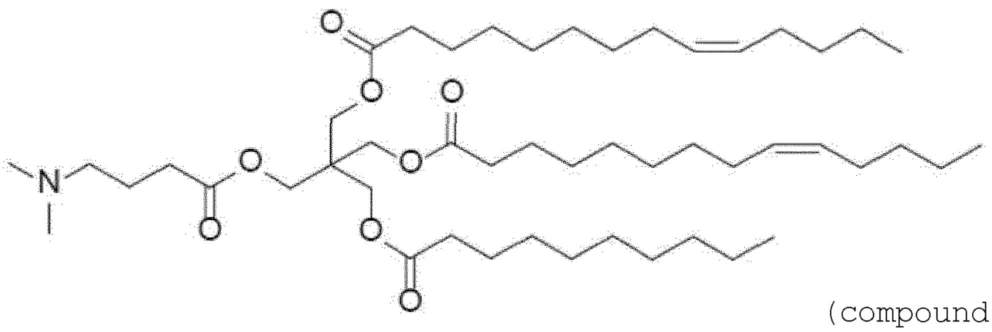

(compound 20)

and

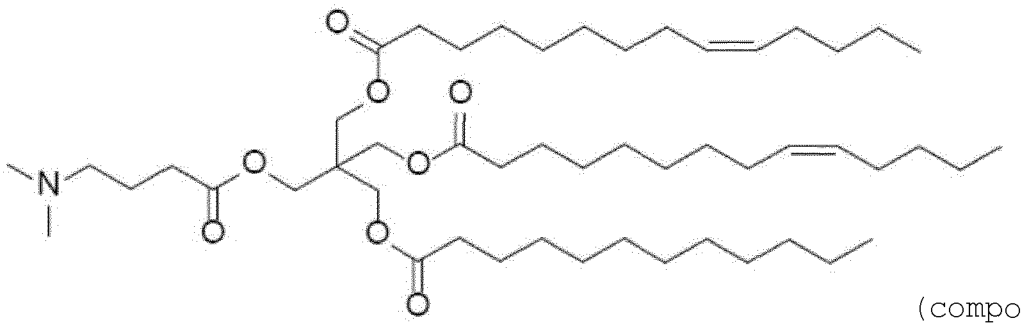

(compound 21)

and salts thereof.

Among the above-mentioned cationic lipids, more preferred cationic lipids are represented by the following structural formulas.

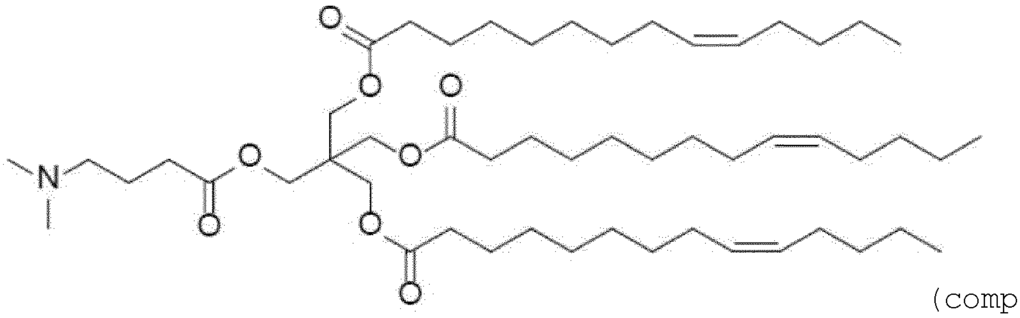

(compound 11)

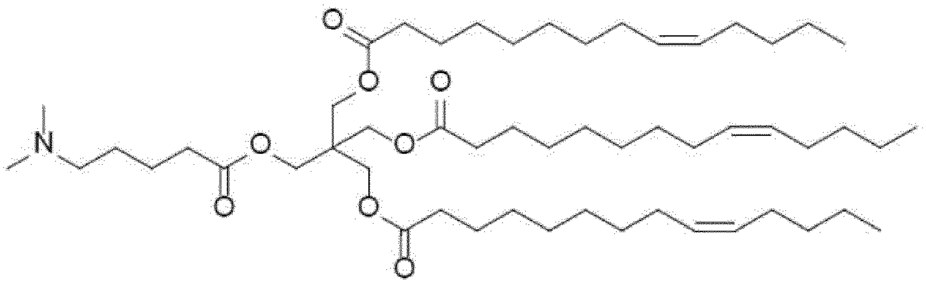

(compound 12)

and

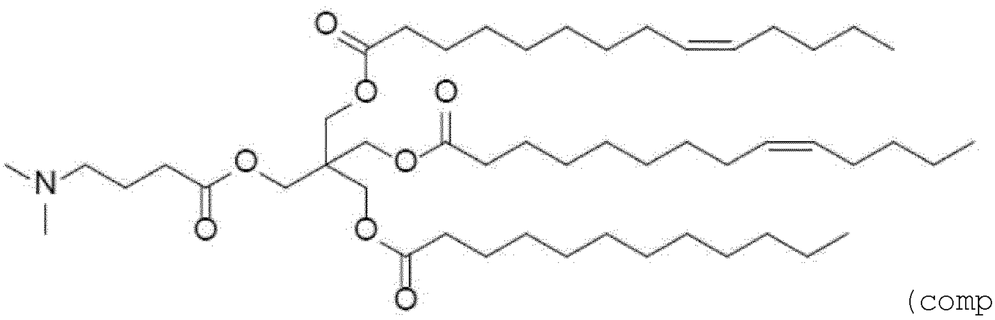

(compound 21)

and salts thereof.

In another preferred embodiment, a cationic lipid represented by the following general formula (III) can be mentioned.

A compound represented by

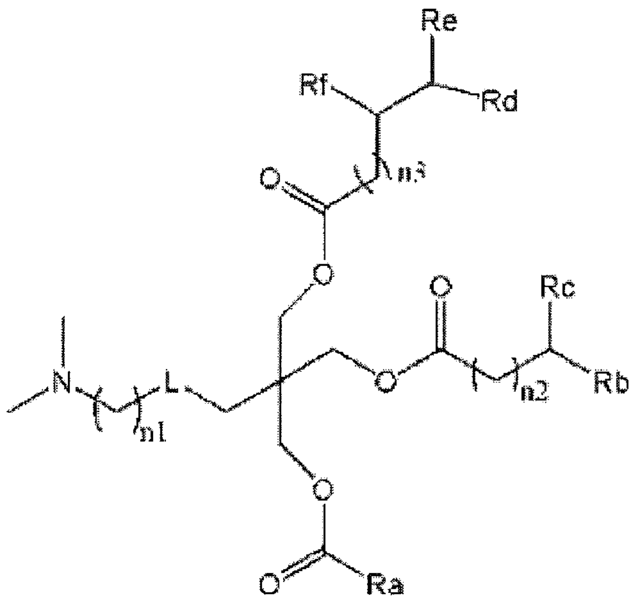

(III)

wherein

n1 is an integer of 2 - 6,
n2 is an integer of 0 - 2,
n3 is an integer of 0 - 2,
L is -C(O)O- or -NHC(O)O-,
Ra is a linear $C_{5-13}$ alkyl group, a linear $C_{13-17}$ alkenyl group or a linear $C_{17}$ alkadienyl group,
Rb is a linear $C_{2-9}$ alkyl group,
Rc is a hydrogen atom or a linear $C_{2-9}$ alkyl group,
Rd is a hydrogen atom or a linear $C_{2-9}$ alkyl group,
Re is a linear $C_{2-9}$ alkyl group, and
Rf is a linear $C_{2-9}$ alkyl group,

or a salt thereof.

More preferably, cationic lipids represented by the following structural formulas can be mentioned.

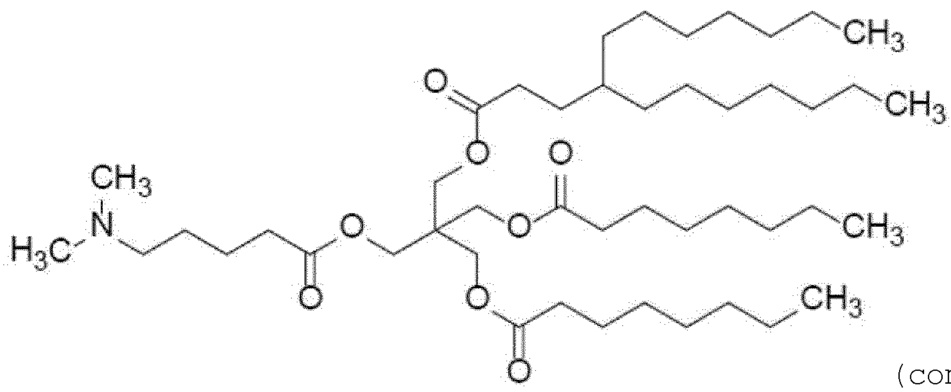

(compound 22)

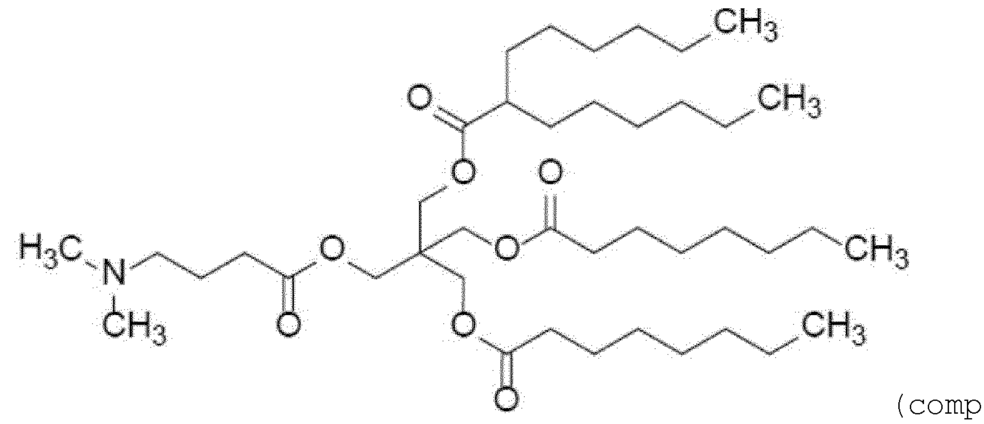

(compound 23)

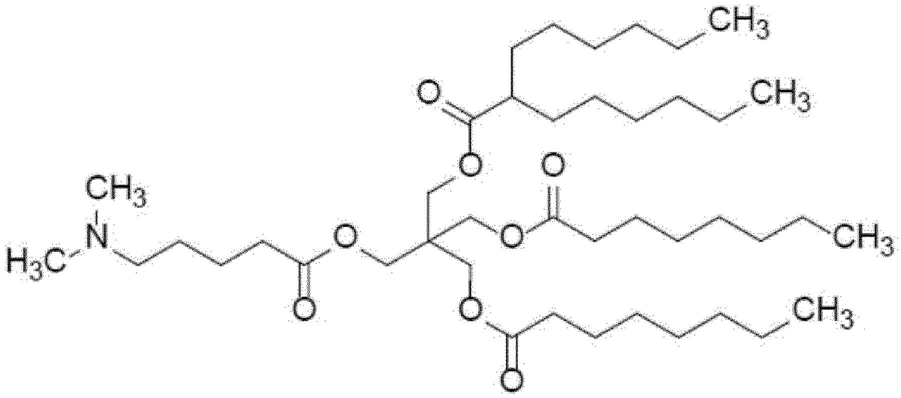

(compound 24)

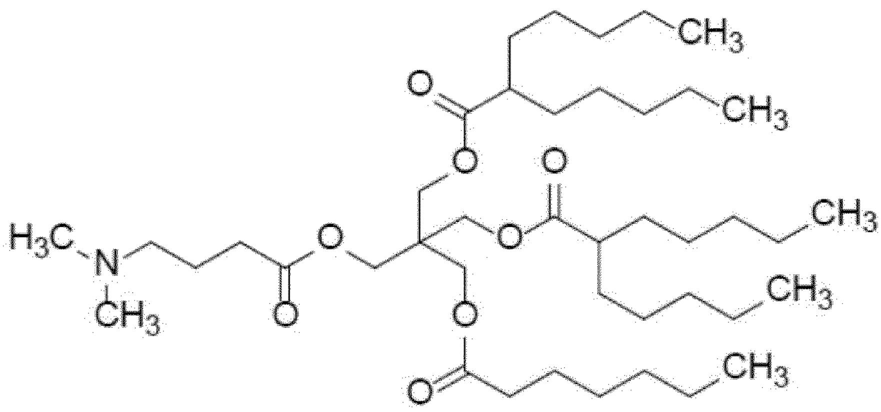

(compound 25)

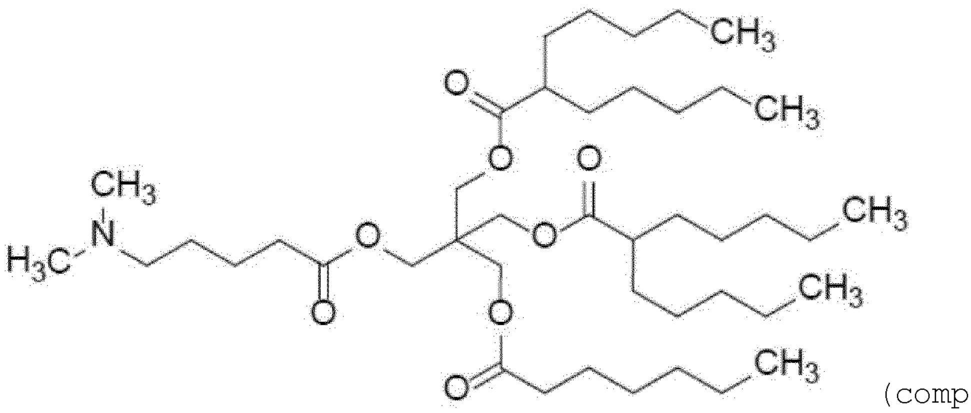

(compound 26)

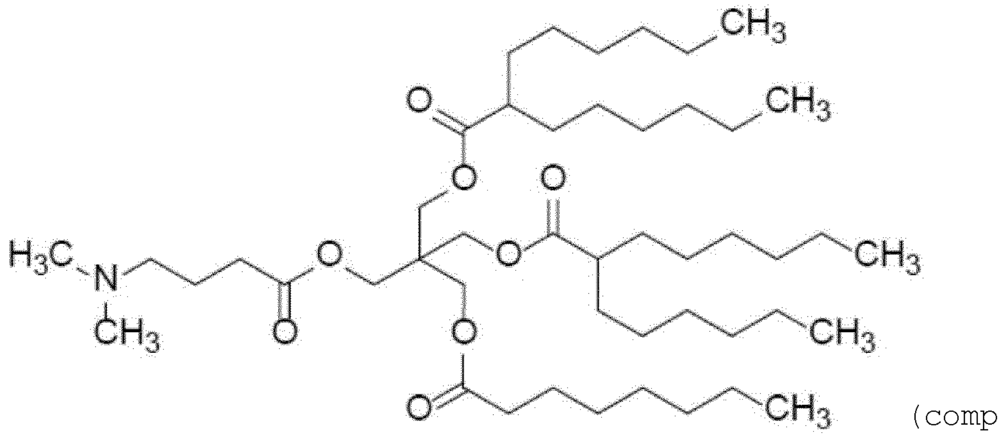

(compound 27)

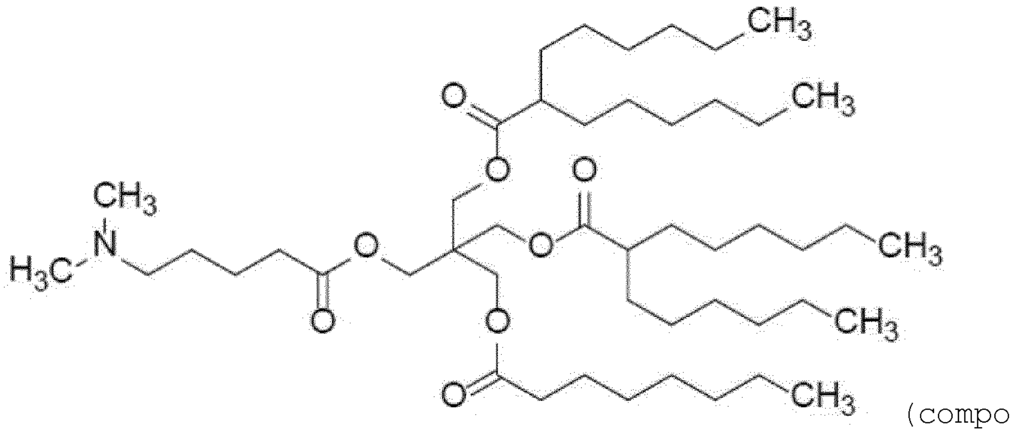

(compound 28)

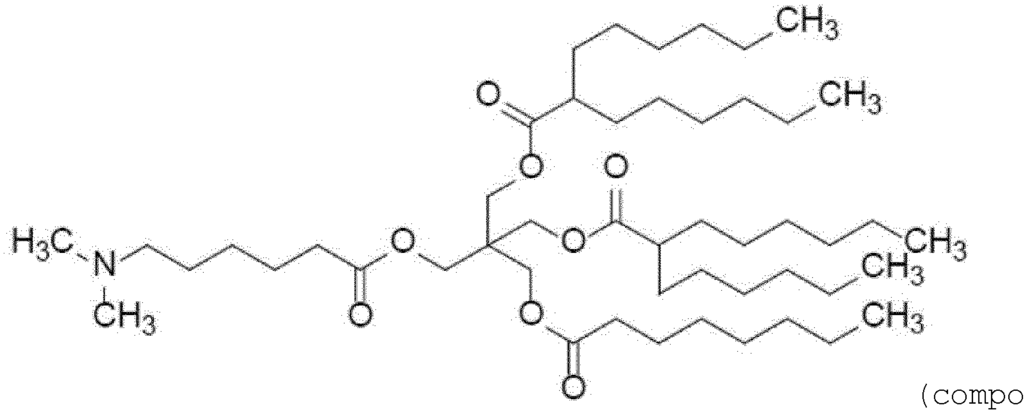

(compound 29)

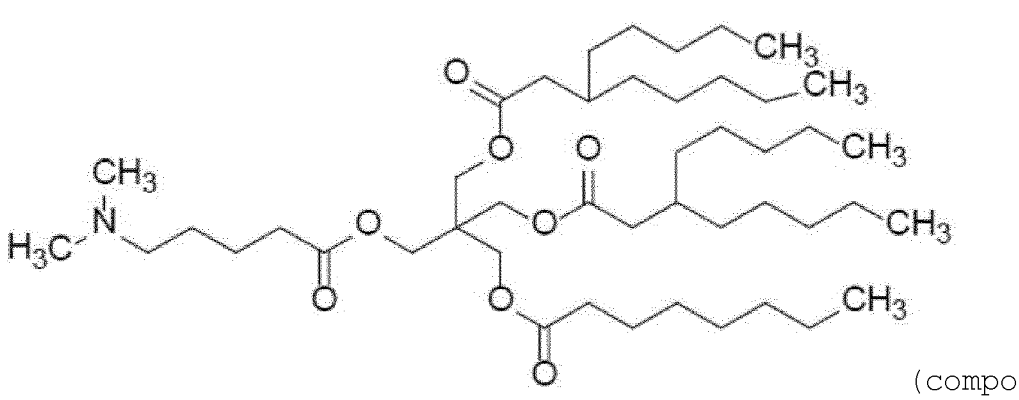

(compound 30)

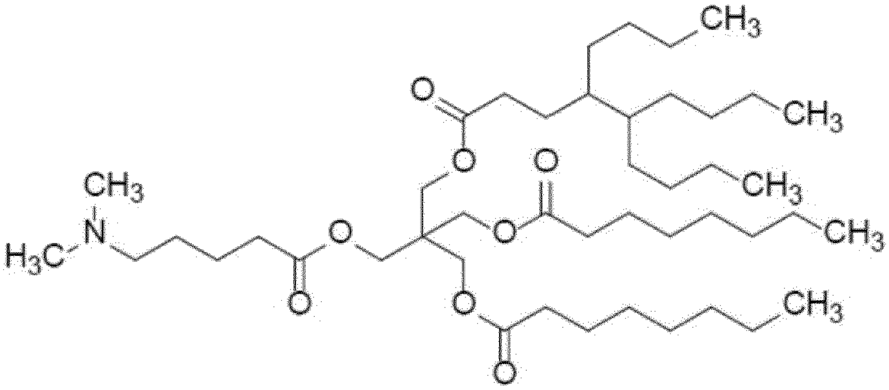

(compound 31)

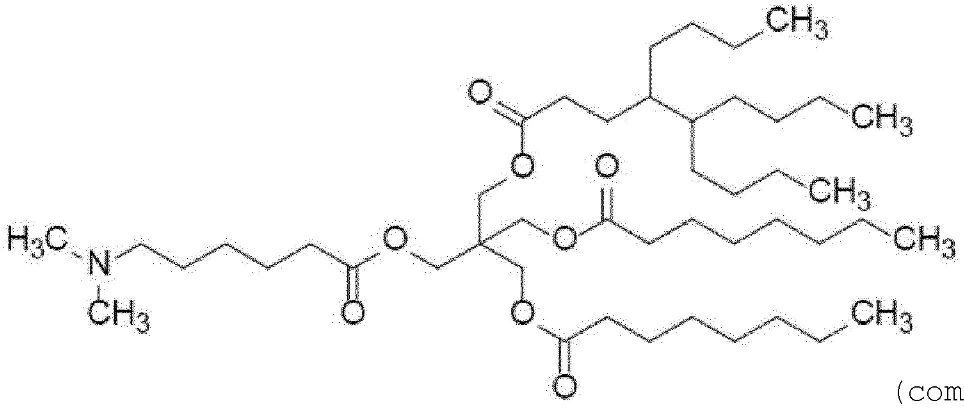

(compound 32)

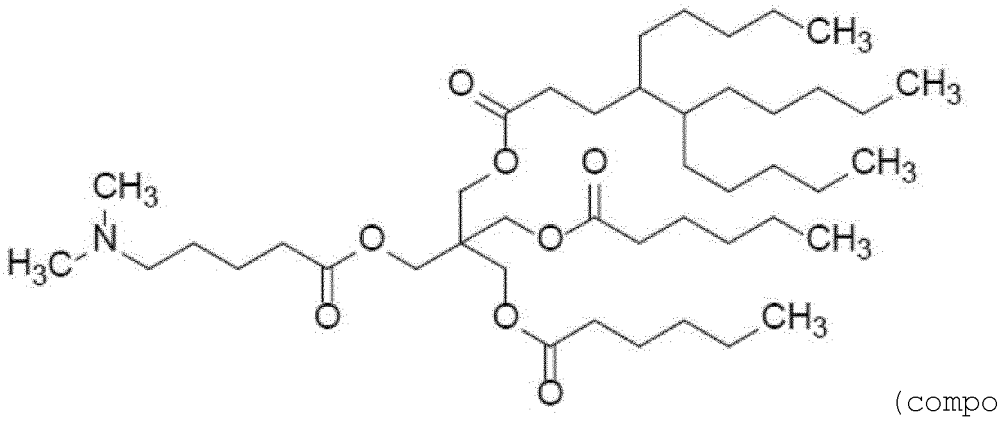

(compound 33)

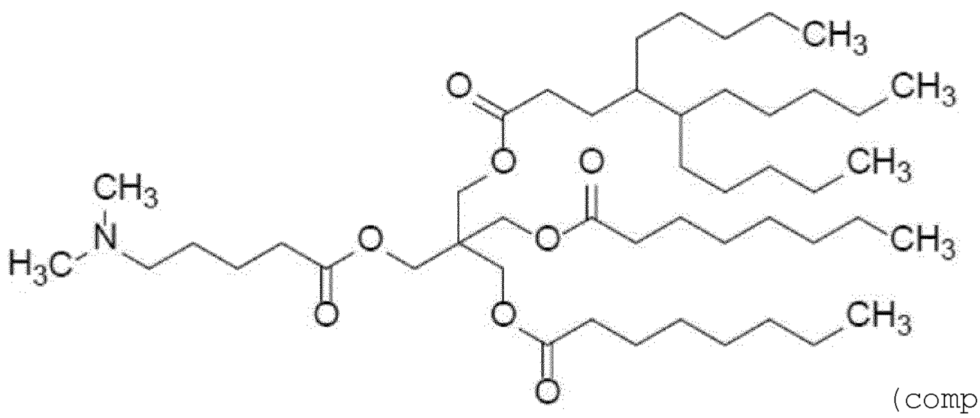

(compound 34)

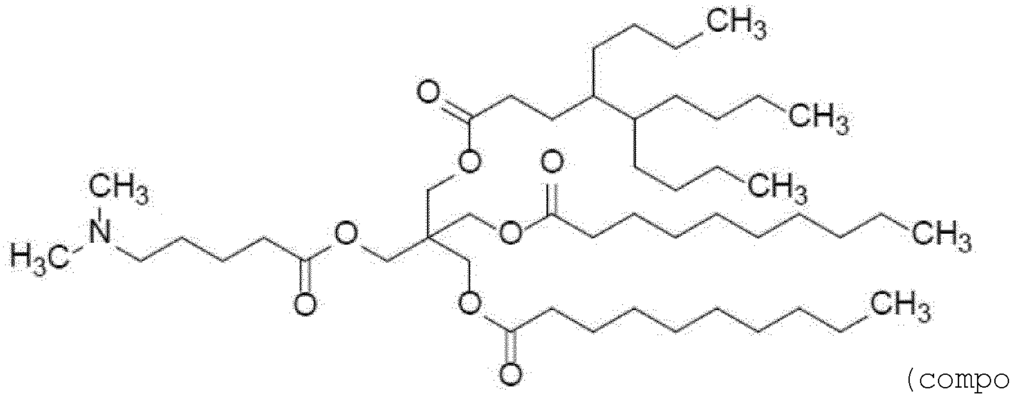

(compound 35)

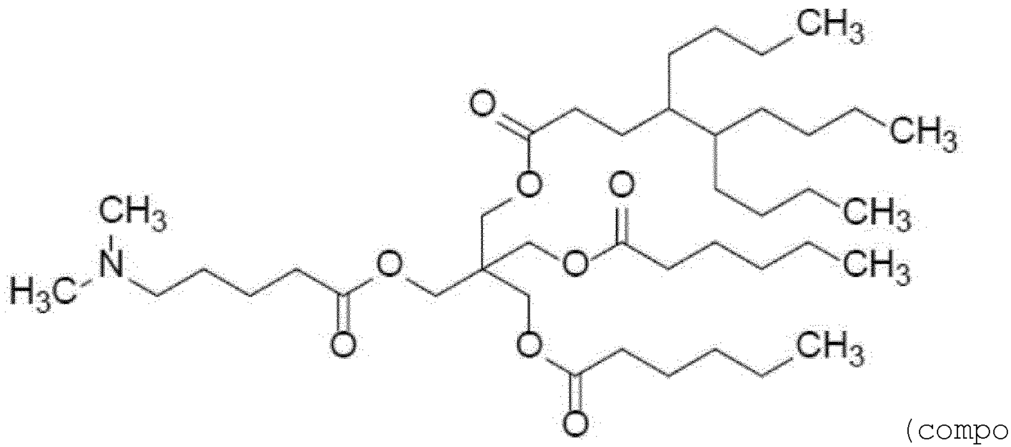

(compound 36)

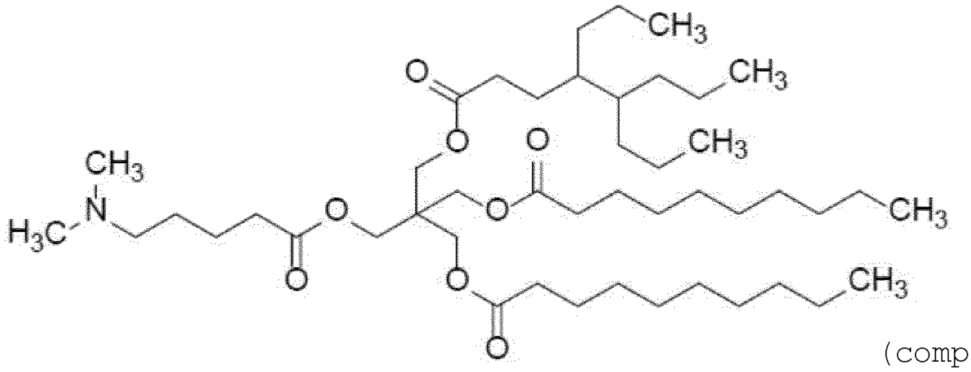

(compound 37)

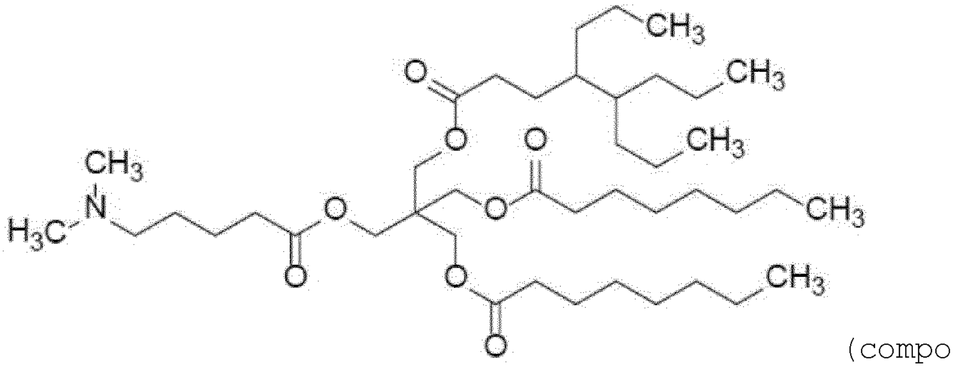

(compound 38)

and

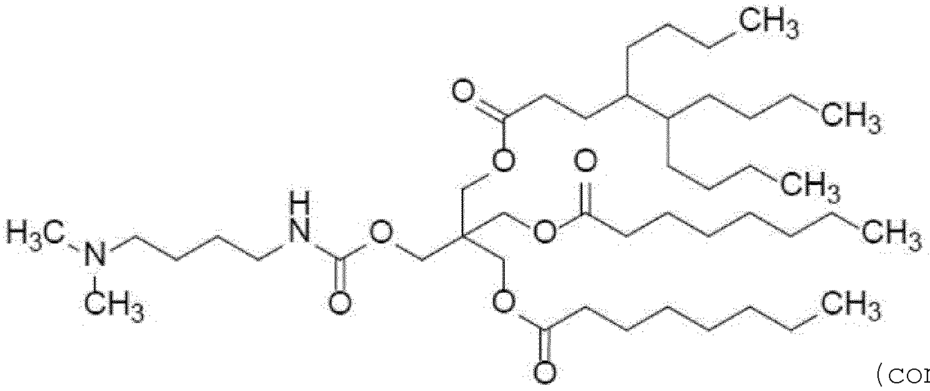

(compound 39)

and salts thereof.

Among the above-mentioned cationic lipids, more preferred are cationic lipids represented by the following structural formulas.

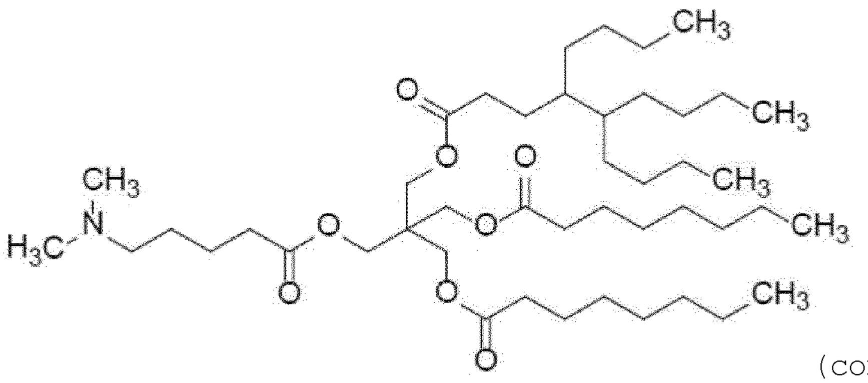

(compound 31)

and

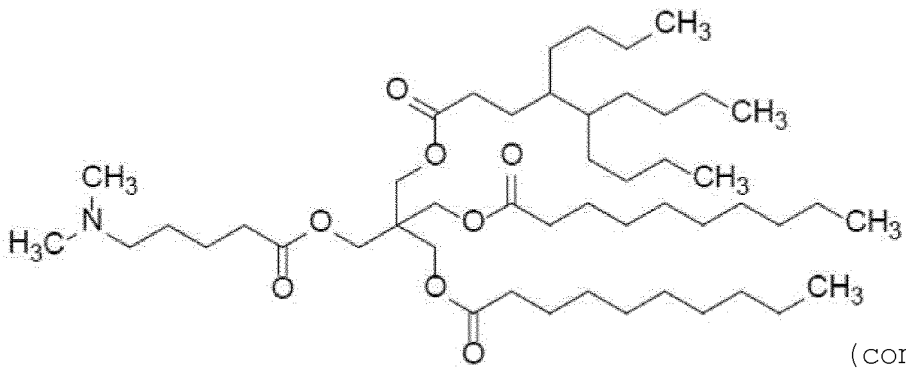

(compound 35)

and salts thereof.

The compound (III) can be produced, for example, by the following production method. In particular, compound (I) with a desired structure can be synthesized using appropriate starting materials according to the structure of the desired compound (III) in the esterification process. The salt of compound (III) can be obtained by appropriately mixing with an inorganic base, an organic base, an organic acid, a basic or an acidic amino acid.

Production method A (L is -C(O)O-)

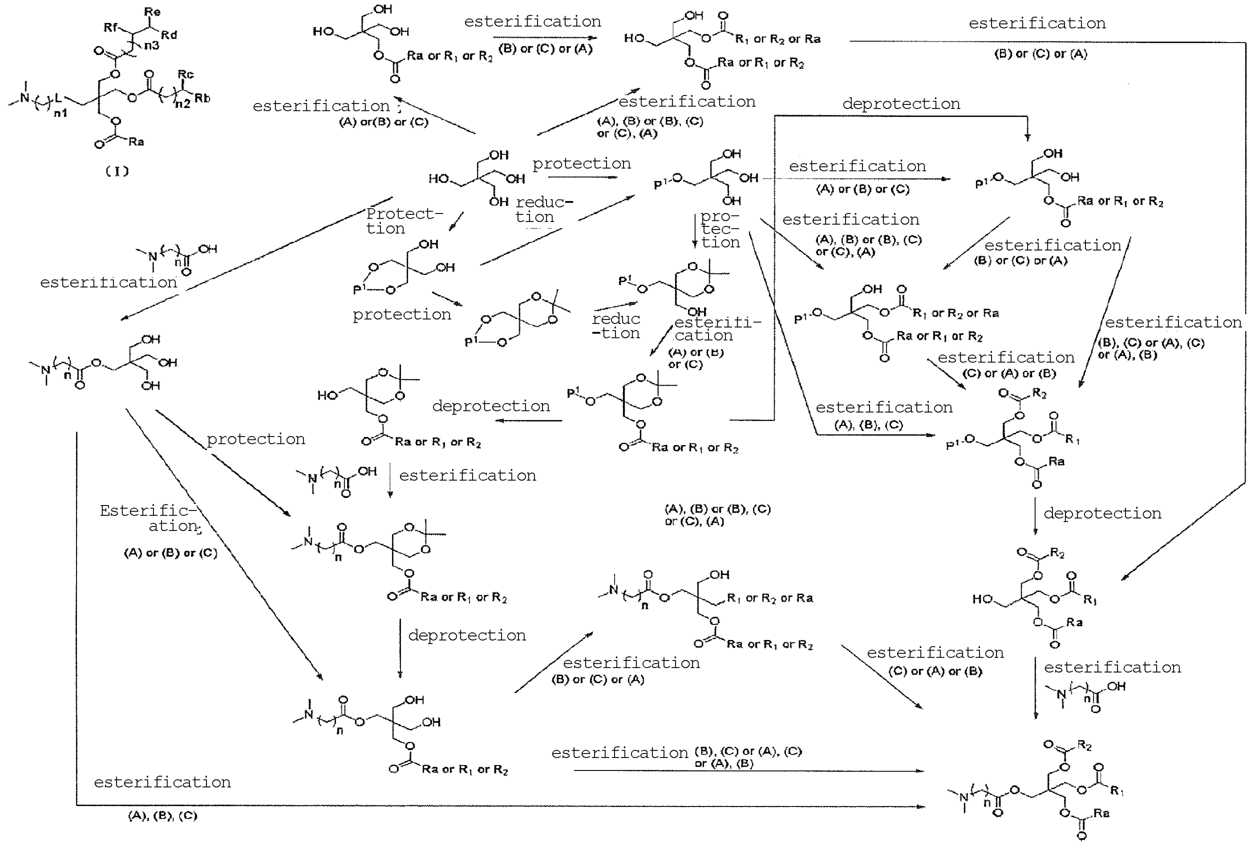


Production method B (L is -NHC(O)O-)

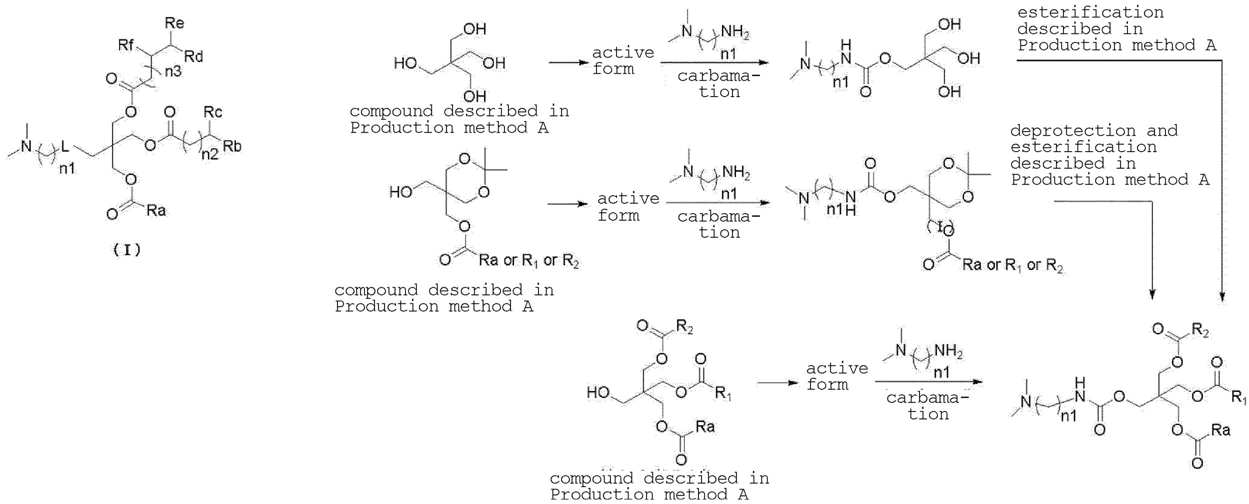


Production method C

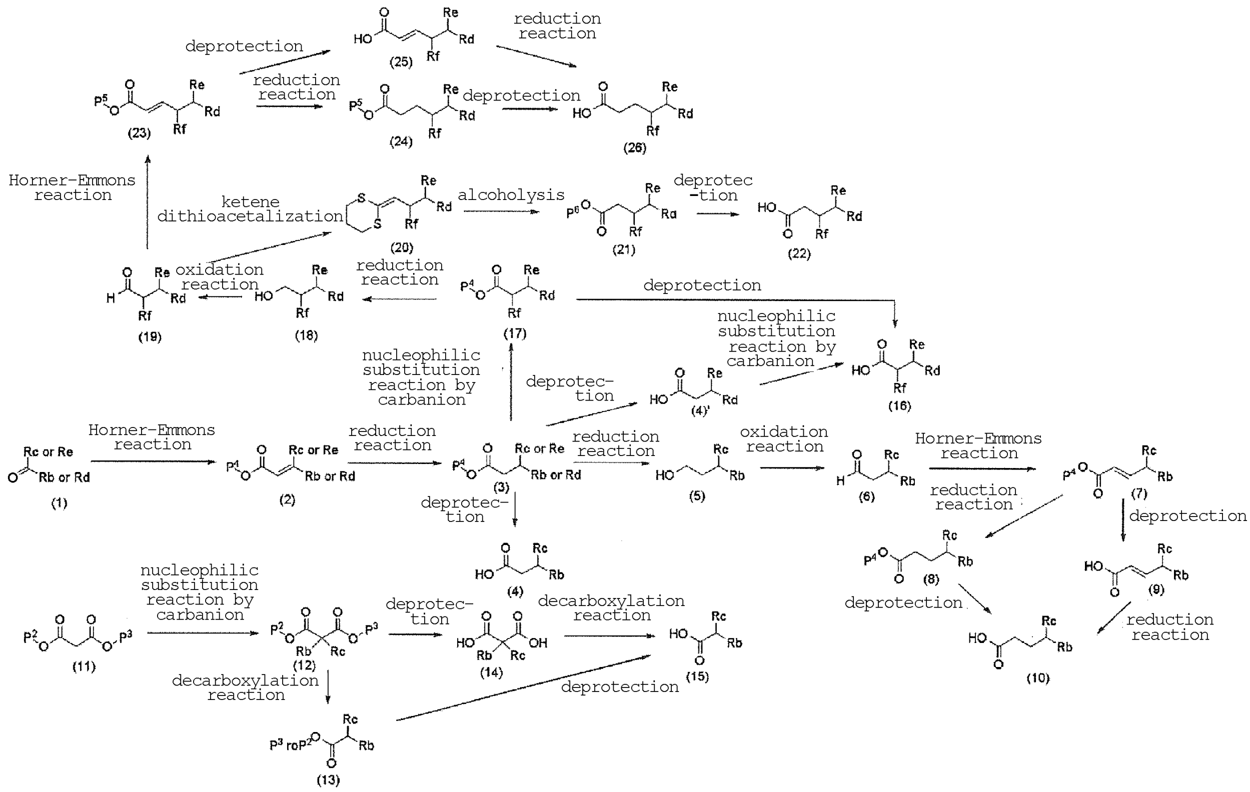


In the above formulas, $P^1$, $P^2$, $P^3$, $P^4$, $P^5$ and $P^6$ are each independently protecting groups, compound (A) is the formula:

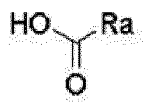 ,

compound (B) is the formula:

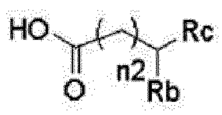 ,

$R^1$ is

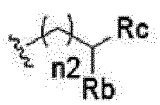 ,

compound (C) is the formula:

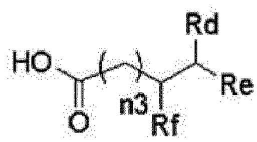

and $R^2$ is

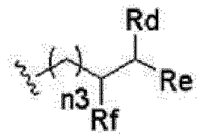

A starting material or a reagent used in each step in the above-mentioned production method, as well as the obtained compound, may each form a salt.

When the compound obtained in each step is a free compound, this compound can be converted to a salt of interest by a method known per se in the art. On the contrary, when the compound obtained in each step is a salt, this salt can be converted to a free form or another type of salt of interest by a method known per se in the art.

The compound obtained in each step may be used in the next reaction directly in the form of its reaction solution or after being obtained as a crude product. Alternatively, the compound obtained in each step can be isolated and/or purified from the reaction mixture by a separation approach such as concentration, crystallization, recrystallization, distillation, solvent extraction, fractionation, or chromatography according to a routine method.

If a starting material or a reagent compound for each step is commercially available, the commercially available product can be used directly.

In the reaction of each step, the reaction time can differ depending on the reagent or the solvent used and is usually 1 min to 48 hr, preferably 10 min to 8 hr, unless otherwise specified.

In the reaction of each step, the reaction temperature can differ depending on the reagent or the solvent used and is usually -78°C to 300°C, preferably -78°C to 150°C, unless otherwise specified.

In the reaction of each step, the pressure can differ depending on the reagent or the solvent used and is usually 1 atm to 20 atm, preferably 1 atm to 3 atm, unless otherwise specified.

In the reaction of each step, for example, a microwave synthesis apparatus such as a Biotage Initiator may be used. The reaction temperature can differ depending on the reagent or the solvent used and is usually room temperature to 300°C, preferably room temperature to 250°C, more preferably 50°C to 250°C, unless otherwise specified. The reaction time can differ depending on the reagent or the solvent used and is usually 1 min to 48 hr, preferably 1 min to 8 hr, unless otherwise specified.

In the reaction of each step, the reagent is used at 0.5 equivalents to 20 equivalents, preferably 0.8 equivalents to 5 equivalents, with respect to the substrate, unless otherwise specified. In the case of using the reagent as a catalyst, the reagent is used at 0.001 equivalents to 1 equivalent, preferably 0.01 equivalents to 0.2 equivalents, with respect to the substrate. When the reagent also serves as a reaction solvent, the reagent is used in the amount for the solvent.

In each step of a reaction, the reaction is carried out without a solvent or by dissolution or suspension in an appropriate solvent, unless otherwise specified. Specific examples of the solvent include the following.

alcohols: methanol, ethanol, isopropanol, isobutanol, tert-butyl alcohol, 2-methoxyethanol and the like;
ethers: diethyl ether, diisopropyl ether, diphenyl ether, tetrahydrofuran, 1,2-dimethoxyethane and the like;
aromatic hydrocarbons: chlorobenzene, toluene, xylene and the like;
saturated hydrocarbons: cyclohexane, hexane, heptane and the like;
amides: N,N-dimethylformamide, N-methylpyrrolidone and the like;
halogenated hydrocarbon s: dichloromethane, carbon tetrachloride and the like;
nitriles: acetonitrile and the like;
sulfoxide: dimethyl sulfoxide and the like;
aromatic organic bases: pyridine and the like;
acid anhydrides: acetic anhydride and the like;
organic acids: formic acid, acetic acid, trifluoroacetic acid and the like;
inorganic acids: hydrochloric acid, sulfuric acid and the like;
esters: ethyl acetate, isopropyl acetate ester and the like;
ketones: acetone, methyl ethyl ketone and the like;
water.

Two or more of these solvents may be used as a mixture at an appropriate ratio.

In each reaction step making use of a base, examples of bases that may be used are those listed below.

inorganic bases: sodium hydroxide, potassium hydroxide, magnesium hydroxide and the like;
basic salts: sodium carbonate, calcium carbonate, sodium hydrogen carbonate and the like;
organic bases: triethylamine, diethylamine, pyridine, 4-dimethylaminopyridine, N,N-dimethylaniline, 1,4-diazabicyclo[2.2.2]octane, 1,8-diazabicyclo[5.4.0]-7-undecene, imidazole, piperidine and the like;
metal alkoxides: sodium ethoxide, potassium tert-butoxide, sodium tert-butoxide and the like;
alkali metal hydrides: sodium hydride and the like;
metal amides: sodium amide, lithium diisopropylamide, lithium hexamethyldisilazide and the like;

organic lithiums: n-butyllithium, sec-butyllithium and the like.

In each reaction step making use of an acid or acid catalyst, the following acids or acid catalysts are used.

inorganic acids: hydrochloric acid, sulfuric acid, nitric acid, hydrobromic acid, phosphoric acid and the like; organic acids: acetic acid, trifluoroacetic acid, citric acid, p-toluenesulfonic acid, 10-camphor sulfonic acid and the like; Lewis acid: boron trifluoride diethyl ether complex, zinc iodide, anhydrous aluminum chloride, anhydrous zinc chloride, anhydrous iron chloride and the like.

Unless stated otherwise, each reaction step may be carried out according to a method known per se in the art, such as those described in Jikken Kagaku Koza (Encyclopedia of Experimental Chemistry in English), 5th Ed., Vol. 13 to Vol. 19 (edited by the Chemical Society of Japan); Shin Jikken Kagaku Koza (New Encyclopedia of Experimental Chemistry in English), Vol. 14 to Vol. 15 (edited by the Chemical Society of Japan); Fine Organic Chemistry, 2nd Ed. Revised (L. F. Tietze, Th. Eicher, Nankodo); Organic Name Reactions; The Reaction Mechanism and Essence, Revised (Hideo Togo, Kodansha); Organic Syntheses Collective Volume I-VII (John Wiley & Sons, Inc.); Modern Organic Synthesis in the Laboratory: A Collection of Standard Experimental Procedures (Jie Jack Li, Oxford University Press); Comprehensive Heterocyclic Chemistry III, Vol. 1 to Vol. 14 (Elsevier Japan KK); Strategic Applications of Named Reactions in Organic Synthesis (translated by Kiyoshi Tomioka, Kagaku-Dojin Publishing); Comprehensive Organic Transformations (VCH Publishers, Inc.), 1989; etc.

In each step, the protection or deprotection reaction of a functional group may be carried out according to a method known per se in the art, for example, a method described in "Protective Groups in Organic Synthesis, 4th Ed." (Theodora W. Greene, Peter G. M. Wuts), Wiley-Interscience, 2007; "Protecting Groups, 3rd Ed." (P.J. Kocienski) Thieme, 2004); etc.

Examples of a protective group for a hydroxy group or a phenolic hydroxy group in alcohols or the like include: ethertype protective groups such as methoxymethyl ether, benzyl ether, p-methoxybenzyl ether, t-butyldimethylsilyl ether, t-butyldiphenylsilyl ether, and tetrahydropyranyl ether; carboxylic acid ester-type protective groups such as acetic acid ester; sulfonic acid ester-type protective groups such as methanesulfonic acid ester; and carbonic acid ester-type protective groups such as t-butyl carbonate.

Examples of a protective group for a carbonyl group in aldehydes include: acetal-type protective groups such as dimethylacetal; and cyclic acetal-type protective groups such as cyclic 1,3-dioxane.

Examples of a protective group for a carbonyl group in ketones include: ketal-type protective groups such as dimethylketal; cyclic ketal-type protective groups such as cyclic 1,3-dioxane; oxime-type protective groups such as O-methyloxime; and hydrazone-type protective groups such as N,N-dimethylhydrazone.

Examples of a protective group for a carboxyl group include: ester-type protective groups such as methyl ester; and amide-type protective groups such as N,N-dimethylamide.

Examples of a protective group for thiol include: ethertype protective groups such as benzyl thioether; and ester-type protective groups such as thioacetic acid ester, thiocarbonate and thiocarbamate.

Examples of a protective group for an amino group or aromatic heterocycle such as imidazole, pyrrole or indole include: carbamate-type protective groups such as benzyl carbamate; amide-type protective groups such as acetamide; alkylamine-type protective groups such as N-triphenylmethylamine; and sulfonamide-type protective groups such as methanesulfonamide.

The protective groups can be removed by use of a method known per se in the art, for example, a method using an acid, a base, ultraviolet light, hydrazine, phenylhydrazine, sodium N-methyldithiocarbamate, tetrabutylammonium fluoride, palladium acetate or trialkylsilyl halide (e.g., trimethylsilyl iodide or trimethylsilyl bromide), or a reduction method.

In each step making use of a reduction reaction, examples of reducing agents that may be used include: metal hydrides such as lithium aluminum hydride, sodium triacetoxyborohydride, sodium cyanoborohydride, diisobutyl aluminum hydride (DIBAL-H), sodium borohydride and tetramethylammonium triacetoxyborohydride; boranes such as borane-tetrahydrofuran complex; Raney nickel; Raney cobalt; hydrogen; and formic acid. For example, Raney-nickel or Raney cobalt can be used in the presence of hydrogen or formic acid. In the case of reducing a carbon-carbon double bond or triple bond, a method using a catalyst such as palladium-carbon or Lindlar's catalyst may be used.

In each step making use of an oxidation reaction, examples of oxidizing agents that may be used include: peracids such as m-chloroperbenzoic acid (MCPBA), hydrogen peroxide and t-butyl hydroperoxide; perchlorates such as tetrabutylammonium perchlorate; chlorates such as sodium chlorate; chlorites such as sodium chlorite; periodates such as sodium periodate; highvalent iodine reagents such as iodosylbenzene; manganese reagents, such as manganese dioxide and potassium permanganate; lead reagents such as lead tetraacetate; chromium reagents, such as pyridinium chlorochromate (PCC), pyridinium dichromate (PDC) and Jones' reagent; halogen compounds such as N-bromosuccinimide (NBS); oxygen; ozone; sulfur trioxide-pyridine complex; osmium tetroxide; selenium dioxide; and 2,3-dichloro-5,6-dicyano-1,4-benzoquinone (DDQ).

In each step making use of a radical cyclization reaction, examples of radical initiators that may be used include: azo compounds such as azobisisobutyronitrile (AIBN); water-soluble radical initiators such as 4-4'-azobis-4-cyanopentanoic acid (ACPA); triethylboron in the presence of air or oxygen; and benzoyl peroxide. Examples of radical initiators to be used include tributylstannane, tristrimethylsilylsilane, 1,1,2,2-tetraphenyldisilane, diphenylsilane and samarium iodide.

In each step making use of a Wittig reaction, examples of Wittig reagents that may be used include alkylidenephosphoranes. The alkylidenephosphoranes can be prepared by a method known per se in the art, for example, the reaction between a phosphonium salt and a strong base.

In each step making use of a Horner-Emmons reaction, examples of reagents that may be used include phosphonoacetic acid esters such as methyl dimethylphosphonoacetate and ethyl diethylphosphonoacetate, and bases such as alkali metal hydrides and organic lithiums.

In each step making use of a Friedel-Crafts reaction, examples of reagents that may be used include a Lewis acid and an acid chloride or alkylating agent (e.g. alkyl halides, alcohols and olefins). Alternatively, an organic or inorganic acid may be used instead of the Lewis acid, and acid anhydrides such as acetic anhydride may be used instead of the acid chloride.

In each step making use of an aromatic nucleophilic substitution reaction, a nucleophile (e.g., amine or imidazole) and a base (e.g., basic salts or organic bases) may be used as reagents.

In each step making use of a nucleophilic addition reaction using a carbanion, nucleophilic 1,4-addition reaction (Michael addition reaction) using a carbanion, or nucleophilic substitution reaction using a carbanion, examples of bases that may be used for generating the carbanion include organolithium reagents, metal alkoxides, inorganic bases and organic bases.

In each step making use of a Grignard reaction, examples of Grignard reagents include aryl magnesium halides such as phenyl magnesium bromide, and alkyl magnesium halides such as methyl magnesium bromide, isopropyl magnesium bromide. The Grignard reagent can be prepared by a method known per se in the art, for example, the reaction between an alkyl halide or aryl halide and magnesium metal in ether or tetrahydrofuran as a solvent.

In each step making use of a Knoevenagel condensation reaction, an active methylene compound flanked by two electronattracting groups (e.g., malonic acid, diethyl malonate or malononitrile) and a base (e.g., organic bases, metal alkoxides or inorganic bases) may be used as reagents.

In each step making use of a Vilsmeier-Haack reaction, phosphoryl chloride and an amide derivative (e.g. N,N-dimethylformamide) may be used as reagents.

In each step making use of an azidation reaction of alcohols, alkyl halides or sulfonic acid esters, examples of azidating agents that may be used include diphenylphosphorylazide (DPPA), trimethylsilylazide and sodium azide. In the case of azidating, for example, alcohols, a method using diphenylphosphorylazide and 1,8-diazabicyclo[5,4,0]undec-7-ene (DBU), a method using trimethylsilylazide and Lewis acid, or the like can be used.

In each step making use of a reductive amination reaction, examples of reducing agents that may be used include sodium triacetoxyborohydride, sodium cyanoborohydride, hydrogen and formic acid. When the substrate is an amine compound, examples of carbonyl compounds that may be used include p-formaldehyde as well as aldehydes such as acetaldehyde and ketones such as cyclohexanone. When the substrate is a carbonyl compound, examples of amines that may be used include primary amines such as ammonia and methylamine, and secondary amines such as dimethylamine.

In each step making use of a Mitsunobu reaction, azodicarboxylic acid esters (e.g. diethyl azodicarboxylate (DEAD) and diisopropyl azodicarboxylate (DIAD)) and triphenylphosphine may be used as reagents.

In each step making use of an esterification, amidation or ureation reaction, examples of reagents that may be used include acyl halides such as acid chlorides or acid bromides, and activated carboxylic acids such as acid anhydrides, active esters or sulfate esters. Examples of the activating agents for carboxylic acids include: carbodiimide condensing agents such as 1-ethyl-3-(3-dimethylaminopropyl)carbodiimide hydrochloride (WSCD); triazine condensing agents such as 4-(4,6-dimethoxy-1,3,5-triazin-2-yl)-4-methylmorpholinium chloride-n-hydrate (DMT-MM); carbonic acid ester condensing agents such as 1,1-carbonyldiimidazole (CDI); diphenylphosphorylazide (DPPA); benzotriazol-1-yloxy-trisdimethylaminophosphonium salt (BOP reagent); 2-chloro-1-methyl-pyridinium iodide (Mukaiyama reagent); thionyl chloride; lower alkyl haloformate such as ethyl chloroformate; O-(7-azabenzotriazol-1-yl)-N,N,N',N'-tetramethyluronium hexafluorophosphate (HATU); sulfuric acid; and combinations thereof. In the case of using a carbodiimide condensing agent, the addition of an additive such as 1-hydroxybenzotriazole (HOBt), N-hydroxysuccinimide (HOSu) or dimethylaminopyridine (DMAP) to the reaction may be beneficial.

In each step making use of a coupling reaction, examples of metal catalysts that may be used include palladium compounds such as palladium(II) acetate, tetrakis(triphenylphosphine)palladium(0) , dichlorobis(triphenylphosphine)palladium(II) , dichlorobis(triethylphosphine)palladium(II), tris(dibenzylideneacetone)dipalladium(0), 1,1'-bis(diphenylphosphino)ferrocene palladium(II) chloride and palladium(II) acetate; nickel compounds such as tetrakis(triphenylphosphine)nickel(0); rhodium compounds such as tris(triphenylphosphine)rhodium(III) chloride; cobalt compounds; copper compounds such as copper oxide and copper(I) iodide; and platinum compounds. Addition of a base to the reaction may also be beneficial. Examples of such bases include inorganic bases and basic salts.

In each step making use of a thiocarbonylation reaction, diphosphorus pentasulfide is typically used as a thiocarbonylating agent. A reagent having a 1,3,2,4-dithiadiphosphetane-2,4-disulfide structure such as 2,4-bis(4-methoxyphenyl-1,3,2,4-dithiadiphosphetane-2,4-disulfide (Lawesson reagent) may be used instead of diphosphorus pentasulfide.

In each step making use of a Wohl-Ziegler reaction, examples of halogenating agents that may be used include N-iodosuccinimide, N-bromosuccinimide (NBS), N- chlorosuccinimide (NCS), bromine and sulfuryl chloride. The reaction can be accelerated by the further addition of a radical initiator such as heat, light, benzoyl peroxide or azobisisobutyronitrile.

In each step making use of a halogenation reaction of a hydroxy group, examples of halogenating agents that may be used include a hydrohalic acid or the acid halide of an inorganic acid; examples include hydrochloric acid, thionyl chloride, and phosphorus oxychloride for chlorination and 48% hydrobromic acid for bromination. In addition, a method for obtaining an alkyl halide from an alcohol by the action of triphenylphosphine and carbon tetrachloride or carbon tetrabromide, etc., may also be used. Alternatively, a method for synthesizing an alkyl halide through a 2-step reaction involving the conversion of an alcohol to a sulfonic acid ester and subsequent reaction with lithium bromide, lithium chloride or sodium iodide may also be used.

In each step making use of an Arbuzov reaction, examples of reagents that may be used include alkyl halides such as ethyl bromoacetate, and phosphites such as triethylphosphite and tri(isopropyl)phosphite.

In each step making use of a sulfone-esterification reaction, examples of the sulfonylating agent used include methanesulfonyl chloride, p-toluenesulfonyl chloride, methanesulfonic anhydride and p-toluenesulfonic anhydride and trifluoromethanesulfonic anhydride.

In each step making use of a hydrolysis reaction, an acid or a base may be used as a reagent. In the case of carrying out the acid hydrolysis reaction of a t-butyl ester, reagents such as formic acid, triethylsilane or the like may be added to reductively trap the by-product t-butyl cation.

In each step making use of a dehydration reaction, examples of dehydrating agents that may be used include sulfuric acid, diphosphorus pentoxide, phosphorus oxychloride, N,N'-dicyclohexylcarbodiimide, alumina and polyphosphoric acid.

A salt of the compound represented by the above-mentioned each structural formula is preferably a pharmacologically acceptable salt. Examples thereof include salts with inorganic bases (e.g., alkali metal salts such as sodium salt, potassium salt and the like; alkaline earth metal salts such as calcium salt, magnesium salt and the like; aluminum salt, ammonium salt), salts with organic bases (e.g., salts with trimethylamine, triethylamine, pyridine, picoline, ethanolamine, diethanolamine, triethanolamine, tromethamine[tris(hydroxymethyl)methylamine], tert-butylamine, cyclohexylamine, benzylamine, dicyclohexylamine, N,N-dibenzyl ethylenediamine), salts with inorganic acids (e.g., salts with hydrofluoric acid, hydrochloric acid, hydrobromic acid, hydroiodic acid, nitric acid, sulfuric acid, phosphoric acid), salts with organic acids (salts with formic acid, acetic acid, trifluoroacetic acid, phthalic acid, fumaric acid, oxalic acid, tartaric acid, maleic acid, citric acid, succinic acid, malic acid, methanesulfonic acid, benzenesulfonic acid, p-toluenesulfonic acid), salts with basic amino acids (salts with arginine, lysine, ornithine) or salts with acidic amino acids (salts with aspartic acid, glutamic acid).

The ratio (mol%) of the cationic lipid to the total lipids present in the lipid nanoparticle is, for example, about 10% to about 80%, preferably about 20% to about 70%, more preferably about 40% to about 60%; however, the ratio is not limited to these.

Only one kind of the above-mentioned cationic lipid may also be used or two or more kinds thereof may be used in combination. When multiple cationic lipids are used, the ratio of the whole cationic lipid is preferably as mentioned above.

(b) Non-cationic lipid

In the present specification, the "non-cationic lipid" means a lipid other than the cationic lipid, and is a lipid that does not have a net positive electric charge at a selected pH such as physiological pH and the like. Examples of the non-cationic lipid used in the lipid nanoparticle of the present invention include phospholipid, steroids, PEG lipid and the like.

To enhance the delivery of nucleic acid into, for example, T cell, the phospholipid is not particularly limited as long as it stably maintains nucleic acid and does not inhibit fusion with cellular membranes (plasma membrane and organelle membrane). For example, phosphatidyl choline, phosphatidyl ethanolamine, phosphatidyl serine, phosphatidyl inositol, phosphatidic acid, palmitoyloleoylphosphatidyl choline, lysophosphatidyl choline, lysophosphatidyl ethanolamine, dipalmitoylphosphatidyl choline, dioleoylphosphatidyl choline, distearoylphosphatidyl choline, dilinolenoylphosphatidyl choline and the like can be mentioned.

Preferred phospholipids include distearoylphosphatidyl choline (DSPC), dioleoylphosphatidyl choline (DOPC), dipalmitoylphosphatidyl choline (DPPC), dioleoylphosphatidyl glycerol (DOPG), palmitoyloleoylphosphatidyl glycerol

(POPG), dipalmitoylphosphatidyl glycerol (DPPG), dioleoyl-phosphatidyl ethanolamine (DOPE), palmitoyloleoylphosphatidyl choline (POPC), palmitoyloleoyl-phosphatidyl ethanolamine (POPE), and dioleoylphosphatidyl ethanolamine 4-(N-maleimide methyl)-cyclohexane-1-carboxylate (DOPE-mal), more preferably DOPC, DPPC, POPC, and DOPE.

The ratio (mol%) of the phospholipid to the total lipids present in the lipid nanoparticle may be, for example, about 0% to about 90%, preferably about 5% to about 30%, more preferably about 8% to about 15%.

Only one kind of the above-mentioned phospholipid may be used or two or more kinds thereof may be used in combination. When multiple phospholipids are used, the ratio of the whole phospholipid is preferably as mentioned above.

As the steroids, cholesterol, 5α-cholestanol, 5β-coprostanol, cholesteryl-(2'-hydroxy)-ethylether, cholesteryl-(4'-hydroxy)-butylether, 6-ketocholestanol, 5α-cholestane, cholestenone, 5α-cholestanone, 5β-cholestanone, and cholesteryl decanoate can be mentioned, preferably cholesterol.

The ratio (mol%) of the steroids to the total lipids present in the lipid nanoparticle when steroids are present may be, for example, about 10% to about 60%, preferably about 12% to about 58%, more preferably about 20% to about 55%.

Only one kind of the above-mentioned steroid may be used or two or more kinds thereof may be used in combination. When multiple steroids are used, the ratio of the whole steroid is preferably as mentioned above.

In the present specification, the "PEG lipid" means any complex of polyethylene glycol (PEG) and lipid. PEG lipid is not particularly limited as long as it has an effect of suppressing aggregation of the lipid nanoparticle. For example, PEG conjugated with dialkyloxypropyl (PEG-DAA), PEG conjugated with diacylglycerol (PEG-DAG) (e.g., SUNBRIGHT GM-020 (NOF CORPORATION)), PEG conjugated with phospholipids such as phosphatidylethanolamine (PEG-PE), PEG conjugated with ceramide (PEG-Cer), PEG conjugated with cholesterol (PEG-cholesterol), or derivatives thereof, or mixtures thereof, mPEG2000-1,2-Di-O-alkyl-sn3-carbomoylglyceride (PEG-C-DOMG), 1-[8'-(1,2-dimyristoyl-3-propanoxy)-carboxamide-3',6-dioxaoctanyl]carbamoyl-ω-methyl-poly(ethylene glycol) (2KPEG-DMG) and the like can be mentioned. Preferred PEG lipid includes PEG-DGA, PEG-DAA, PEG-PE, PEG-Cer, and a mixture of these, more preferably, a PEG-DAA conjugate selected from the group consisting of a PEG-didecyl oxypropyl conjugate, a PEG-dilauryl oxypropyl conjugate, a PEG-dimyristyl oxypropyl conjugate, a PEG-dipalmityl oxypropyl conjugate, a PEG-distearyl oxypropyl conjugate, and mixtures thereof.

In addition to the methoxy group, the maleimide group, N-hydroxysuccinimidyl group and the like for binding the T cell activating ligand can be used as the free end of PEG. For example, SUNBRIGHT DSPE-0201MA (NOF) can be used as a PEG lipid having a functional group for binding a T cell- activating ligand (sometimes to be referred to as "terminal reactive PEG lipid" in the present specification).

The ratio (mol%) of the PEG lipid to the total lipids present in the lipid nanoparticle of the present invention may be, for example, about 0% to about 20%, preferably about 0.1% to about 5%, more preferably about 0.7% to about 2%.

The ratio (mol%) of the terminal reactive PEG lipid in the above-mentioned total PEG lipids may be, for example, about 10% to about 100%, preferably about 30% to about 100%, more preferably about 40% to about 100%.

Only one kind of the above-mentioned PEG lipid may be used or two or more kinds thereof may be used in combination. When multiple PEG lipids are used, the ratio of the whole PEG lipid is preferably as mentioned above.

### 1-1-2. Liposome

As another preferable nucleic acid delivery carrier to be used in the present invention, a liposome can be mentioned. As the liposome, those conventionally used in DDS of nucleic acids to cells can be similarly used. For example, liposomes prepared by mixing various cationic lipids (e.g., DOTMA, DOTAP, DDAB, DMRIE etc.) developed as transfection reagents, and membranefused neutral lipids (e.g., DOPE, cholesterol etc.) that promote release from endosome are widely used. Liposomes in which functional molecules such as PEG, pH-responsive membrane fusion peptide, membrane permeation promoting peptide and the like are added to the surface of the liposome can also be used.

### 1-2. T cell activating ligand

In the nucleic acid delivery carrier of the present invention, a T cell activating ligand is added to the surface of the above-mentioned nucleic acid delivery carrier.

The T cell activating ligand to be used in the present invention is not particularly limited as long as it is a molecule that interacts with the surface molecules of T cells to promote activation and/or proliferation of the T cells. For example, molecules having a function of specifically binding to CD3, which is coupled with TCR and responsible for signal transduction via TCR, and surface molecules CD28, ICOS, CD137, OX40, CD27, GITR, BAFFR, TACI, BMCA, CD40L and the like, which are known as co-stimulation factors of T cell activation, and transducing activation/proliferation signals and co-signals in T cells or antigen-presenting cells can be mentioned. Such molecule may be a physiological ligand (or receptor) for the above-mentioned T cell surface molecule, or may be a non-physiological ligand (or receptor) having

an agonist activity. As the non-physiological ligand, an agonist antibody can be preferably mentioned.

More preferably, the T cell activating ligand to be used in the present invention includes an antibody against CD3 and/or an antibody against CD28. The antibody against CD3 and the antibody against CD28 each specifically bind to CD3 and CD28 expressed on target T cells to be induced to activate and/or proliferate (for example, when target T cell is derived from human, the antibody against CD3 and the antibody against CD28 are desirably anti-human CD3 antibody and anti-human CD28 antibody, respectively), and may be a complete antibody or a fragment thereof (e.g., Fab, $F(ab')_2$, Fab', scFv, Fv, reduced antibody (rIgG), dsFv, sFv, diabody, triabody, etc.) as long as they have the ability to stimulate these surface molecules of T cells and transduce signals in the T cells. The subclass of the antibody is also not particularly limited, but is preferably an IgG antibody.

When an agonist antibody such as an antibody against CD3 or an antibody against CD28 is used as the T cell activating ligand, a commercially available anti-CD3 antibody, anti-CD28 antibody, or the like can also be used as long as it is a complete antibody molecule, or the antibody can also be isolated from the culture medium of the cells that produce the antibody. On the other hand, when the ligand is any of the aforementioned antibody fragments, by treating a complete antibody with a reducing agent (e.g., 2-mercaptoethanol, dithiothreitol) or peptidase (e.g., papain, pepsin, ficin), or by isolating a nucleic acid encoding fragments of anti-CD3 antibody, anti-CD28 antibody and the like in the same manner as in obtaining a nucleic acid to be encapsulated in a nucleic acid delivery carrier to be described later, the antibody fragment can be recombinantly produced using the same.

As the T cell activating ligand, only one kind may be used, or two or more kinds may be used in combination. It is preferable to combine two or more kinds. When two or more kinds of T cell activating ligands are used in combination, at least one kind is preferably an antibody against CD3 or an antibody against CD28, more preferably an antibody against CD3. Particularly preferably, both an antibody against CD3 and an antibody against CD28 can be used as T cell activating ligands.

When at least an antibody against CD3 and an antibody against CD28 are used in combination as the T cell activating ligands, the molar ratio of the two added to the surface of the nucleic acid delivery carrier of the present invention is 1:4 - 4:1, preferably 1:2 - 2:1.

When two or more kinds of T cell activating ligands are used in combination, the T cell activating ligands may be separately added to the surface of the nucleic acid delivery carrier, or they may be complexed and added to the surface of the nucleic acid delivery carrier as long as the T cell activation activity of each is maintained. For example, when the two kinds of the T cell activating ligands are antibodies (e.g., antibody against CD3 and antibody against CD28), they can be provided as bispecific antibodies known per se.

In the nucleic acid delivery carrier of the present invention, the T cell activating ligand may bind to the outer shell in any manner as long as it is present on the surface of the nucleic acid delivery carrier. For example, when a lipid nanoparticle containing a terminally reactive PEG lipid as a non-cationic lipid is used as a nucleic acid delivery carrier, the ligand can be added to the terminal of PEG. For example, lipid nanoparticles labeled with a ligand (antibody) can be prepared by reacting a PEG lipid with a maleimide group introduced into the terminus (e.g., SUNBRIGHT DSPE-0200MA) with the thiol group of the above-mentioned reduced antibody. Even when a liposome modified with PEG is used as a nucleic acid delivery carrier, the T cell activating ligand can be added to the surface of the liposome surface in the same manner.

1-3. Nucleic acid contained in the nucleic acid delivery carrier of the present invention

The nucleic acid delivery carrier of the present invention in a form free of a nucleic acid can also be used to induce activation and/or proliferation of T cells. In one preferred embodiment, activation and/or proliferation of T cells and delivery of the nucleic acid into T cells can be performed simultaneously in one step with the encapsulated nucleic acid. Therefore, in one preferred embodiment, the nucleic acid delivery carrier of the present invention further contains a nucleic acid to be delivered into T cells.

When the nucleic acid delivery carrier of the present invention contains a nucleic acid inside, the nucleic acid is not particularly limited as long as the nucleic acid itself or a transcript or translation product thereof has a function of changing T cells into a desired state within the T cells.

1-3-1. Nucleic acid suppressing expression of T cell activation inhibitory factor

In one preferred embodiment, the nucleic acid delivery carrier of the present invention contains inside a nucleic acid suppressing expression of a T cell activation inhibitory factor. The T cell activation inhibitory factor to be the target is not particularly limited as long as it suppresses activation of T cells. For example, immune checkpoint factors (e.g., CTLA-4, PD-1, TIM-3, LAG-3, TGIT, BTLA, VISTA(PD-1H) etc.) which are cell surface molecules that transmit negative signals to activation and/or proliferation of T cells upon receipt of stimulation from antigen-presenting cells or tumor cells, CD160, Cbl-b, endogenous TCR and the like can be mentioned.

A nucleic acid suppressing expression of a T cell activation inhibitory factor may act at any level from transcription level of gene encoding the factor, posttranscriptional regulation level, protein translation level, post-translational modification level, and the like. Therefore, examples of the nucleic acid suppressing expression of a T cell activation inhibitory factor include a nucleic acid (e.g., antigene) inhibiting transcription of a gene encoding the factor, a nucleic acid inhibiting processing from initial transcripts to mRNA, a nucleic acid inhibiting translation from mRNA to protein (e.g., antisense nucleic acid, miRNA) or degrading mRNA (e.g., siRNA, ribozyme, miRNA) and the like. While the substances acting at any of those levels are preferably used, a substance that complementarily binds to mRNA to inhibit translation into protein or degrades mRNA is preferable. As the nucleic acid,

(a) nucleic acid having RNAi activity against mRNA encoding the factor, or a precursor thereof,
(b) antisense nucleic acid against mRNA encoding the factor,
(c) ribozyme nucleic acid against mRNA encoding the factor, and the like can be mentioned.

The nucleotide sequence of mRNA (cDNA) encoding each T cell activation inhibitory factor is known, and sequence information can be obtained, for example, from public databases (e.g., NCBI, EMBL, DDBJ etc.).

(a) Nucleic acid having RNAi activity against mRNA encoding T cell activation inhibitory factor, or precursor thereof

As the nucleic acid having RNAi activity against mRNA encoding a T cell activation inhibitory factor, double-stranded RNA consisting of an oligo RNA complementary to the target mRNA and a complementary strand thereof, i.e., siRNA can be mentioned. The siRNA can be designed based on the cDNA sequence information of the target gene, for example, according to the rules proposed by Elbashir et al. (Genes Dev., 15, 188-200 (2001)). The short hairpin RNA (shRNA), which is a precursor of siRNA, can be designed by appropriately selecting any linker sequence (for example, about 5-25 bases) capable of forming a loop structure, and linking the sense strand and antisense strand of siRNA via the linker sequence.

The siRNA and/or shRNA sequences can be searched using search software provided free of charge on various websites. Examples of such site include, but are not limited to, siDESIGN Center provided by Dharmacon (http://dharmacon.horizondiscovery.com/jp/designcenter/?rdr=true&LangType=1041&pageid=17179928204), siRNA Target Finder provided by GenScript (https://www.genscript.com/tools/sirna-target-finder) and the like.

In the present specification, microRNA (miRNA) that targets mRNA encoding a T cell activation inhibitory factor is also defined as being included in the nucleic acid having RNAi activity against the mRNA. For miRNA, primary-microRNA (pri-miRNA), which is the primary transcript, is first transcribed from a gene encoding the miRNA, then processed by Drosha into precursor-microRNA (pre-miRNA) of about 70 bases in length having a characteristic hairpin structure, transported from the nucleus to the cytoplasm, and further processed by mediation of Dicer to become mature miRNA, which is taken up by RISC and acts on the target mRNA. Therefore, pre-miRNA or pri-miRNA, preferably pre-miRNA, can also be used as a precursor of miRNA.

miRNA can be searched using target prediction software provided free of charge on various websites. Examples of such site include, but are not limited to, TargetScan (http://www.targetscan.org/vert_72/) published by Whitehead Institute, USA, DIANA-micro-T-CDS(http://diana.imis.athenainnovation.gr/DianaTools/index.php?r=microT_CDS/index) published by Alexander Fleming Biomedical Sciences Research Centre, Greece, and the like. Alternatively, miRNA targeting mRNA encoding a T cell activation inhibitory factor can also be searched using TarBase (http://carolina.imis.athenainnovation.gr/diana_tools/web/index.php?r=tarbasev8/index) which is a database relating to miRNA that has been experimentally proven to act on the target mRNA, and published by the University of Thessaly, Pasteur Institute and the like.

The nucleotide molecules that constitute siRNA and/or shRNA, or miRNA and/or pre-miRNA may be native RNA or DNA. In order to improve stability (chemical and/or against enzyme) and specific activity (affinity for RNA), various chemical modifications known per se can be included.

siRNA can be prepared according a process comprising synthesizing a sense strand and an antisense strand of target sequence on mRNA each with the DNA/RNA automatic synthesizer, denaturing in a suitable annealing-buffer solution at about 90 to 95°C for about 1 minute, and annealing at about 30 to 70°C for about 1 to 8 hours. In addition, siRNA can also be prepared by synthesizing shRNA which is the precursor of siRNA and cleaving the shRNA with the use of a dicer. miRNA and pre-miRNA can be synthesized by a DNA/RNA automatic synthesizer based on the sequence information thereof.

In the present specification, a nucleic acid designed to be able to produce siRNA or miRNA against mRNA encoding a T cell activation inhibitor in vivo is also defined as being included in the nucleic acid having RNAi activity against the mRNA. Examples of such nucleic acid include expression vectors constructed to express the above-mentioned shRNA or siRNA or miRNA or pre-miRNA, and the like. As the promoter, a polII promoter (e.g., CMV early-immediate promoter) can be used. It is a general practice to use a polIII promoter to ensure accurate transcription of

short RNA. Examples of the polIII promoter include mouse and human U6-snRNA promoters, human H1-RNase P RNA promoter, human valine-tRNA promoter and the like. In addition, a sequence in which four or more Ts are continuous is used as the transcription termination signal. An expression cassette of miRNA and pre-miRNA can also be produced in the same manner as in shRNA.

(b) Antisense nucleic acid against mRNA encoding T cell activation inhibitory factor

The antisense nucleic acid against mRNA encoding a T cell activation inhibitory factor is a nucleic acid comprising a nucleotide sequence complimentary to the nucleotide sequence of mRNA or a part thereof, which has a function of inhibiting the protein synthesis by binding specifically with the target mRNA to form a stable duplex. The antisense nucleic acid may be DNA or RNA, or DNA/RNA chimera. When the antisense nucleic acid is DNA, the RNA:DNA hybrid formed by the target RNA and the antisense DNA is recognized by endogenous RNase H, thereby undergoing the selective degradation of the target RNA. The length of the target region of the antisense nucleic acid is not particularly limited as long as the hybridization of the antisense nucleic acid eventually inhibits the translation into protein, and may be the entire sequence of mRNA encoding the protein or a partial sequence thereof. A short one may be about 10 bases, and a long one may be the entire sequence of mRNA or initial transcript. In addition, the antisense nucleic acid may be a nucleic acid that inhibits the translation into a protein by hybridizing with target mRNA or initial transcript, and it may as well as be the nucleic acid capable of forming a triplex by binding with these genes which are the double-stranded DNAs and inhibiting the transcription into RNA (anti-gene).
The nucleotide molecule constituting the antisense nucleic acid may also be modified in the same manner as in the cases of the above-mentioned siRNA and the like in order to improve stability, specific activity and the like.
The antisense oligonucleotide can be prepared by determining a target sequence of mRNA or initial transcript based on the cDNA sequence or genomic DNA sequence of the target gene, and synthesizing its complementary sequence with the use of a commercially available DNA/RNA automatic synthesizer.
In the present specification, a nucleic acid designed to be able to generate an antisense RNA for mRNA encoding a T cell activation inhibitory factor in vivo is also defined to be included in an antisense nucleic acid for the mRNA. Such nucleic acid can be exemplified by an expression vector so constructed as to express the above-mentioned antisense RNA, or the like. As the promoter, a polII promoter or a polIII promoter can be appropriately selected and used according to the length of the antisense RNA to be transcribed.

(c) Ribozyme nucleic acid for mRNA encoding T cell activation inhibitory factor

A ribozyme nucleic acid capable of specifically cleaving the internal coding region of the mRNA encoding a T cell activation inhibitory factor can also be used as a nucleic acid suppressing the expression of the factor. The "ribozyme" is narrowly-defined as RNA having enzymatic activity for cleaving nucleic acid, but the present specification also includes DNA as long as there is a sequence specific enzymatic activity for cleaving nucleic acid. Ribozyme nucleic acid with the broadest utility includes self-splicing RNA which can be found in infectious RNA such as viroid, a virusoid, etc., and hammerhead type or hairpin type are known. When ribozyme is used in the form of an expression vector having DNA which encodes the ribozyme, the ribozyme can be hybrid ribozyme further coupled with the sequence of modified tRNA so as to promote transport to cytoplasm of a transcript.

(d) Nucleic acid suppressing expression of T cell activation inhibitory factor by genome editing

In a preferred embodiment different from the above-mentioned (a) - (c), the nucleic acid suppressing expression of a T cell activation inhibitory factor can be a nucleic acid that can inactivate (knock out) a gene encoding the factor. As such nucleic acid, a nucleic acid encoding an artificial nuclease composed of a nucleic acid sequence recognition module (e.g., CRISPR/Cas9, ZFmotif, TAL effector etc.) capable of specifically recognizing a partial nucleotide sequence in the gene as a target, and a nuclease that introduces double-strand break (DSB) into the gene in the inside of or near the target sequence can be mentioned. After DSB introduction, the gene can be knocked out by insertion or deletion mutation due to a nonhomologous end joining (NHEJ) repair error. Alternatively, gene knockout by homologous recombination (HR) repair can also be performed by combining with a targeting vector in which a marker gene (e.g., reporter gene such as fluorescent protein gene and the like, selection marker gene such as drug resistance gene and the like) is inserted in the gene sequence. In addition, the endogenous TCR gene can also be knocked in by HR repair with an exogenous TCR gene.

1-3-2. Nucleic acid encoding T cell activation promoting factor

In another preferred embodiment, the nucleic acid delivery carrier of the present invention contains inside a

nucleic acid encoding a T cell activation promoting factor. The T cell activation promoting factor of interest includes, for example, T cell surface molecules (e.g., CD28, ICOS, CD137, OX40, CD27, GITR, BAFFR, TACI, BMCA, CD40L etc.) to which the aforementioned T cell activating ligand binds to transduce activation and/or proliferation signals in T cells, and the like.

The nucleotide sequence of mRNA (cDNA) encoding each T cell activation promoting factor is known, and sequence information can be obtained, for example, from public databases (e.g., NCBI, EMBL, DDBJ etc.).

The nucleic acid encoding the T cell activation promoting factor can be encapsulated, in the form of an expression vector containing mRNA or DNA encoding the factor, in the nucleic acid delivery carrier of the present invention. The mRNA encoding the T cell activation promoting factor can be isolated by a method known per se, using RNA extracted from T cells as a template, and using a probe or primer prepared based on the sequence information thereof. The obtained mRNA may be encapsulated as it is in the nucleic acid delivery carrier of the present invention, or can be converted into cDNA and amplified by RT-PCR.

The obtained DNA encoding a T cell activation promoting factor can be inserted into an expression vector, preferably a plasmid vector, containing a functional promoter in T cells, either as is or after adding a suitable linker and/or nuclear translocation signal and the like. Examples of the functional promoter in T cells include, but are not limited to, constitutive SR$\alpha$ promoter, SV40 promoter, LTR promoter, CMV (cytomegalovirus) promoter, RSV (Rous sarcoma virus) promoter, MoMuLV (Moloney mouse leukemia virus) LTR, HSV-TK (herpes simplex virus thymidine kinase) promoter and the like in mammalian cells. In addition, gene promoters such as CD3, CD4, and CD8, which direct specific expression in T cells, can also be used.

The mRNA encoding a T cell activation promoting factor can be prepared by transcription into mRNA in an in vitro transcription system known per se using an expression vector containing DNA encoding the factor as a template.

<u>1-3-3. Nucleic acid encoding chimeric antigen receptor (CAR) or exogenous T cell receptor (TCR)</u>

As mentioned above, the nucleic acid delivery carrier of the present invention encapsulating the nucleic acid makes it possible to perform activation and/or proliferation of T cells, and delivery of the nucleic acid into T cells simultaneously in one step. Therefore, by encapsulating a nucleic acid encoding CAR or TCR in the nucleic acid delivery carrier of the present invention, a step of activation/proliferation of T cells and a step of gene transfer into T cells can be performed simultaneously in one pod.

That is, in one preferred embodiment, the nucleic acid delivery carrier of the present invention contains inside a nucleic acid encoding CAR or TCR.

(a) Nucleic acid encoding CAR

CAR is an artificially constructed hybrid protein containing the antigen-binding domain (e.g., scFv) of an antibody coupled to a T cell signal transduction domain. CAR is characterized by the ability to utilize the antigen-binding property of the monoclonal antibody to redirect the specificity and responsiveness of T cells to a selected target in a non-MHC-restricted manner. Non-MHC-restricted antigen recognition confers on CAR-expressing T cells the ability to recognize antigens independently of antigen processing, thereby bypassing the major mechanism of tumor escape. Furthermore, when expressed in T cells, CAR advantageously does not dimerize with the endogenous TCR $\alpha$ chain and $\beta$ chain.

CAR to be encapsulated in the nucleic acid delivery carrier of the present invention includes an antigen-binding domain of an antibody that can specifically recognize surface antigens (e.g., cancer antigen peptide, surface receptor showing promoted expression in cancer cells, etc.) that the target T cell should recognize, an extracellular hinge domain, a transmembrane domain, and an intracellular T cell signal transduction domain.

Examples of the surface antigens specifically recognized by antigen-binding domains include, but are not limited to, surface receptors showing promoted expression in various cancers (e.g., acute lymphocytic cancer, alveolar rhabdomyosarcoma, bladder cancer, bone cancer, brain cancer (e.g., medulloblastoma), breast cancer, anus, anal canal or anorectal cancer, cancer of the eye, cancer of the interhepatic bile duct, joint cancer, cervical, gallbladder or pleural cancer, nose, nasal cavity or middle ear cancer, oral cancer, vulvar cancer, chronic myelogenous cancer, colorectal cancer, esophageal cancer, cervical cancer, fibrosarcoma, gastrointestinal carcinoid tumor, head and neck cancer (e.g., head and neck squamous cell carcinoma), hypopharyngeal cancer, kidney cancer, laryngeal cancer, leukemia (e.g., acute lymphoblastic leukemia, acute lymphocytic leukemia, chronic lymphocytic leukemia, acute myelogenous leukemia), liquid tumor, liver cancer, lung cancer (e.g., non-small cell lung cancer), lymphoma (e.g., Hodgkin lymphoma, non-Hodgkin lymphoma, diffuse large B cell lymphoma, follicular lymphoma), malignant mesothelioma, mastocytoma, melanoma, multiple myeloma, nasopharyngeal cancer, ovarian cancer, pancreatic cancer; peritoneal, omentum and mesenteric cancer; pharyngeal cancer, prostate cancer, rectal cancer, kidney cancer, skin cancer, small intestinal cancer, soft tissue cancer, solid tumor, gastric cancer, testicular cancer, thyroid cancer, ureteral cancer and the like, for example, CD19, EGF receptor, BCMA, CD30, Her2, ROR1, MUC16, CD20, mesothelin, B-cell mutation antigen, CD123, CD3, prostate

specific membrane antigen (PSMA), CD33, MUC-1, CD138, CD22, GD2, PD-L1, CEA, chondroitin sulfate proteoglycan-4, IL-13 receptor α chain, IgG κ light chain, and cancer antigen peptides (e.g., peptides derived from WT1, GPC3, MART-1, gp100, NY-ESO-1, MAGE-A4, etc.).

The antigen-binding domain used in the present invention is not particularly limited as long as it is an antibody fragment that can specifically recognize the target antigen. Considering the ease of preparation of CAR, a single-chain antibody (scFv) in which a light chain variable region and a heavy chain variable region are linked via a linker peptide is desirable. The configuration of the light chain variable region and heavy chain variable region in single-chain antibody is not particularly limited as long as they can reconstitute a functional antigen-binding domain. They can generally be designed in the order of light chain variable region, linker peptide, and heavy chain variable region from the N-terminal side. As the linker peptide, a known linker peptide typically used for the production of single-chain antibodies can be used. For example, DNA encoding light chain variable region and DNA encoding heavy chain variable region can be prepared by cloning light chain gene and heavy chain gene respectively from antibody-producing cells and performing PCR using them as templates, or the like, or by chemically synthesizing them from the sequence information of existing antibodies. DNA encoding a single-chain antibody can be obtained by ligating each obtained DNA fragment with a DNA encoding linker peptide by an appropriate method. The N-terminal side of the antigen-binding domain is preferably further added with a leader sequence to present CAR to the surface of T cell.

As the extracellular hinge domain and transmembrane domain, T cell surface molecule-derived domains generally used in the relevant technical field can be used as appropriate. For example, they include, but are not limited to, domains derived from CD8α and CD28.

Examples of the intracellular signal transduction domain include, but not limited to, those having a CD3ζ chain, those further having a co-stimulatory motif such as CD28, CD134, CD137, Lck, DAP10, ICOS, 4-1BB, and the like between the transmembrane domain and the CD3ζ chain, those having two or more co-stimulatory motifs and the like. Any domains normally used in the relevant technical field can be used in combination.

Sequence information of nucleic acids encoding extracellular hinge domain, transmembrane domain, and intracellular signaling domain is well known in the relevant technical field. Those of ordinary skill in the art can easily obtain DNA fragments encoding each domain from T cells based on such information.

DNA encoding CAR can be obtained by linking DNA fragments respectively encoding the thus-obtained antigen binding domain, extracellular hinge domain, transmembrane domain, and intracellular signal transduction domain, by a conventional method.

The obtained DNA encoding CAR can be inserted into an expression vector, preferably a plasmid vector, containing a functional promoter in T cells, either as is or after adding a suitable linker and/or nuclear localization signal and the like. Examples of the functional promoter in T cells include, but are not limited to, constitutive SRα promoter, SV40 promoter, LTR promoter, CMV (cytomegalovirus) promoter, RSV (Rous sarcoma virus) promoter, MoMuLV (Moloney mouse leukemia virus) LTR, HSV-TK (herpes simplex virus thymidine kinase) promoter and the like in mammalian cells. In addition, gene promoters such as CD3, CD4, and CD8, which direct specific expression in T cells, can also be used.

RNA encoding a CAR, preferably mRNA, can be prepared by transcription into mRNA in an in vitro transcription system known per se using an expression vector containing DNA encoding the above-mentioned CAR as a template.

(b) Nucleic acid encoding exogenous TCR

In the present specification, the "T cell receptor (TCR)" means a receptor that consists of dimers of the TCR chain (α-chain, β-chain) and recognizes an antigen or the antigen-HLA (human leukocyte type antigen) (MHC; major histocompatibility complex) complex and transduces a stimulatory signal to T cells. Each TCR chain consists of a variable region and a constant region, and the variable region contains three complementarity determining regions (CDR1, CDR2, CDR3). The TCR used in the present invention includes not only those in which the α and β chains of the TCR constitute a heterodimer but also those in which they constitute a homodimer. Furthermore, the TCR includes those with a part or all of the constant regions deleted, those with recombined amino acid sequence, and those with soluble TCR, and the like.

The "exogenous TCR" means being exogenous to T cell, which is the target cell of the nucleic acid delivery carrier of the present invention. The amino acid sequence of the exogenous TCR may be the same as or different from that of the endogenous TCR expressed by T cell, which is the target cell of the nucleic acid delivery carrier of the present invention.

The nucleic acid encoding TCR encapsulate in the nucleic acid delivery carrier of the invention is a nucleic acid encoding the α chain and β chain of TCR that can specifically recognize surface antigens (e.g., cancer antigen peptide etc.) to be recognized by the target T cell.

The nucleic acid can be prepared by a method known per se. When the amino acid sequence or nucleic acid sequence of the desired TCR is known, a DNA encoding the full-length or a part of the TCR of the present invention can be constructed based on the sequence by, for example, chemically synthesizing a DNA strand or an RNA strand, or

connecting a synthesized, partially overlapping oligo-DNA short strand by the PCR method or the Gibson assembly method.

When the sequence of the desired TCR is not known, for example, T cell of interest is isolated from a population of cells containing the T cell expressing a TCR of interest, and a nucleic acid encoding the TCR can be obtained from the T cell. Specifically, a cell population (e.g., PBMC) containing T cells is collected from an living organism (e.g., human), the cell population is cultured in the presence of epitopes of cell surface antigens recognized by the TCR of interest while stimulating the cell population, and T cell that specifically recognizes cells expressing the cell surface antigen can be selected from the cell population by a known method and using, as indices, specificity for cells expressing the cell surface antigen and cell surface antigens such as CD8 and CD4. The specificity for cells expressing the cell surface antigen of T cells can be measured, for example, by dextromer assay, ELISPOT assay, cytotoxic assay, or the like. The aforementioned cell population containing T cells is preferably collected from, for example, an organism having a large number of cells expressing a cell surface antigen recognized by the TCR of interest (e.g., patient with a disease such as cancer, or T cell-containing population contacted with an epitope of the antigen or dendritic cells pulsed with the epitope).

The nucleic acid of the present invention can be obtained by extracting DNA from the aforementioned isolated T cell by a conventional method, amplifying and cloning the TCR gene based on the nucleic acid sequence of the constant region of the TCR by using the DNA as a template. It can also be prepared by extracting RNA from a cell and synthesizing cDNA by a conventional method, and performing 5'-RACE (rapid amplification of cDNA ends) with the cDNA as templates using antisense primers complementary to the nucleic acids respectively encoding the constant regions of the TCR α chain and β chain. 5'-RACE may be performed by a known method and can be performed, for example, using a commercially available kit such as SMART PCR cDNA Synthesis Kit (manufactured by clontech). The DNA encoding the α chain and β chain of the obtained TCR can be inserted into an appropriate expression vector in the same way as the DNA encoding the above-mentioned CAR. The DNA encoding α chain and the DNA encoding β chain may be inserted into the same vector or separate vectors. When inserted into the same vector, the expression vector may express both strands in a polycistronic or monocistronic manner. In the former case, an intervening sequence that permits polyscystronic expression, such as IRES or FMV 2A, is inserted between the DNA encoding both strands.

In addition, RNA encoding each strand of the TCR, preferably mRNA, can be prepared in the same way as the above-mentioned RNA encoding CAR, for example, by using the expression vector as a template.

## 1-4. T cell-targeting ligand

The nucleic acid delivery carrier of the present invention is desirably used to activate and/or proliferate T cells preferably ex vivo. It also includes an embodiment of in vivo administration to a subject. In this case, a ligand capable of targeting the nucleic acid delivery carrier to T cells (hereinafter to be also referred to as "T cell targeting ligand") is further added to the surface of the nucleic acid delivery carrier of the present invention, whereby the targeting efficiency to T cells can be enhanced.

The T cell-targeting ligand is not particularly limited as long as it can specifically recognize surface molecules that are specifically or highly expressed in T cells. Preferably, it includes one containing an antigen binding domain of an antibody against CD3, CD4 or CD8, more preferably, an anti-CD3 antibody. Here, the "antigen-binding domain" is synonymous with the antigen-binding domain that constitutes the above-mentioned CAR. However, since CAR needs to be prepared as a nucleic acid encoding same, restrictions occur and single-chain antibodies are generally used in many cases. Since the antigen-binding domain as a T cell targeting ligand is contained in a protein state in the lipid nanoparticle of the present invention, not only single-chain antibodies, but also any other antibody fragments, such as complete antibody molecules, Fab, $F(ab')_2$, Fab', Fv, reduced antibody (rIgG), dsFv, sFv, diabody, triabody, and the like, can also be used preferably. These antibody fragments can be prepared by treating the complete antibody (e.g., IgG) with a reduced agent (e.g., 2-mercaptoethanol, dithiothreitol) or peptidase (e.g., papain, pepsin, ficin), or by using a genetic recombination operation.

When the T-cell targeting ligand is a complete antibody molecule, commercially available anti-CD3, CD4, CD8 antibodies, etc. can be used, or the ligand can be isolated from the culture of the cells producing the antibody. On the other hand, when the ligand is any one of the aforementioned antigen-binding domain (antibody fragment), the nucleic acid encoding the antigen-binding domain, such as anti-CD3, CD4, CD8 antibodies, etc., is isolated in the same way as in the nucleic acid encoding the antigen-binding domain constituting the said CAR is obtained, and the antigen-binding domain can be recombinantly produced using the same.

## 2. Production of the nucleic acid delivery carrier of the present invention

As a representative example of the nucleic acid delivery carrier of the present invention, a production example of the nucleic acid delivery carrier of the present invention using lipid nanoparticles as a carrier (hereinafter to be also

referred to as "the lipid nanoparticle of the present invention") is explained in the following. Even when other carriers such as liposomes and the like are used, the nucleic acid delivery carrier of the present invention can be obtained in the same manner by appropriately making changes according to the carrier used.

The lipid nanoparticle of the present invention can be produced, for example, by forming lipid nanoparticles by the method described in US9,404,127, and chemically binding the T cell-activating ligand. Alternatively, as described in WO 2016/021683, for example, an organic solvent solution dissolving cationic lipid and non-cationic lipid is prepared, the organic solvent solution is mixed with water or a buffer solution dissolving the nucleic acid to be encapsulated in the lipid nanoparticles to prepare lipid nanoparticles, and T cell activating ligand (further, T cell-targeting ligand as necessary when the lipid nanoparticle of the present invention is used in vivo) is chemically bound, whereby the lipid nanoparticle can be produced. The mixing ratio (molar ratio) of cationic lipid, phospholipid, cholesterol, and PEG lipid is, for example, 40 to 60:0 to 20:0 to 50:0 to 5, but the ratio is not limited thereto. When PEG lipid is blended as a non-cationic lipid and a T cell-activating ligand is added to the terminal of PEG, the mixing ratio (molar ratio) of the PEG lipid and the ligand may be, for example, 20:1 to 1:20. The above-mentioned PEG lipid may contain terminal reactive PEG at a ratio (mol%) of about 10% to about 100%. The above-mentioned mixing can be conducted using a pipette, a micro fluid mixing system (e.g., Asia microfluidic system (Syrris)). The obtained lipid particles may be subject to purification by gel filtration, dialysis or sterile filtration.

The concentration of the total lipid component in the organic solvent solution is preferably 0.5 to 100 mg/mL.

As the organic solvent, for example, methanol, ethanol, 1-propanol, 2-propanol, 1- butanol, tert-butanol, acetone, acetonitrile, N,N-dimethylformamide, dimethyl sulfoxide, or a mixture thereof can be recited. The organic solvent may contain 0 to 20% of water or a buffer solution. As the buffer solution, acidic buffer solutions (e.g., acetate buffer solution, citrate buffer solution) or neutral buffer solutions (e.g., 4-(2-hydroxyethyl)-1-piperazineethanesulfonic acid, (HEPE) buffer solution, tris(hydroxymethyl)aminomethane (Tris) buffer solution, a phosphate buffer solution, phosphate buffered saline (PBS)) can be recited.

In the case where a micro fluid mixing system is used for mixing, preference is given to mixing 1 part by volume of an organic solvent solution with 1 to 5 parts by volume of water or a buffer solution. In addition, in said system, the flow rate of the mixture (a mixture solution of an organic solvent solution and water or a buffer solution) is preferably 0.1 to 10 mL/min, and the temperature preferably is 15 to 45°C.

When a lipid particle dispersion is produced as described above, the dispersion containing cationic lipid, non-cationic lipid, nucleic acid and T cell activating ligand can be produced by adding a nucleic acid to be encapsulated in lipid nanoparticles to water or buffer. Addition of the nucleic acid in a manner to render the concentration thereof in water or a buffer solution 0.05 to 2.0 mg/mL is preferable.

In addition, the lipid nanoparticle can also be produced by admixing a lipid particle dispersion with the nucleic acid by a method known per se.

In the lipid nanoparticle of the present invention, the content of the nucleic acid is preferably 1 - 20 wt%. The content can be measured using Quant-iT™Ribogreen® (Invitrogen). In the lipid nanoparticle of the present invention, the encapsulation ratio of the nucleic acid can be calculated based on the difference in fluorescence intensity in the presence or absence of the addition of a surfactant (e.g., Triton-X100).

A dispersion medium can be substituted with water or a buffer solution by dialysis. For the dialysis, ultrafiltration membrane of molecular weight cutoff 10 to 20K is used to carry out at 4°C to room temperature. The dialysis may repeatedly be carried out. For the dialysis, tangential flow filtration may be used.

The ratio (weight ratio) of the nucleic acid and the lipid in the lipid nanoparticle of the present invention obtained as mentioned above is about 0.01 to about 0.2.

The average particle size of the lipid nanoparticle of the present invention is preferably 10 to 200 nm. The average particle size of the lipid particles can be calculated using, for example, Zetasizer Nano ZS (Malvern Instruments) on cumulant analysis of an autocorrelation function.

## 3. Activation/proliferation method of T cells, delivery method of nucleic acid into T cells, and production method of medicament containing T cells, using T cell activating ligand and nucleic acid delivery carrier

The present invention also provides an activation and/or proliferation method of T cells using the nucleic acid delivery carrier of the present invention obtained as mentioned above (hereinafter to be also referred to as "the activation/proliferation method of the present invention"). The method includes a step of contacting a cell population containing T cells with the nucleic acid delivery carrier of the present invention. As used herein, the T cell may be a T cell collected from a living organism (to be also referred to as "ex vivo T cell" in the present specification"), or T cell in a living organism (to be also referred to as "in vivo T cell" in the present specification), with preference given to ex vivo T cell.

In a different aspect, when the nucleic acid delivery carrier of the present invention contains a nucleic acid in the inside, the activation/proliferation method of the present invention can simultaneously deliver the nucleic acid into T cells. Therefore, the present invention also provides a delivery method of nucleic acid into T cells, including a step of

contacting a cell population containing T cells with the nucleic acid delivery carrier of the present invention. In another embodiment, the present invention provides a delivery method of nucleic acid into T cells, including a step of contacting a cell population containing T cells simultaneously with at least one kind of T cell activating ligand, and any of the above-mentioned nucleic acid delivery carriers without a T cell activating ligand added to the surface (hereinafter the above-mentioned two embodiments are also to be collectively referred to as "the nucleic acid delivery method of the present invention"). In the present specification, when a free T cell activating ligand that is not bound to a nucleic acid delivery carrier is used, the ligand may be used alone, or as a complex in which the ligand is bound to a carrier (e.g., Dynabeads(R) (Thermo Fisher Scientific company)). As such complex, a commercially available product (e.g., TransAct (Milteny Biotech company), Dynabeads Human T-Activator CD3/CD28 (ThermoFisher Scientific company)) may also be used.

In another different aspect, T cells activated and/or proliferated using the nucleic acid delivery carrier of the present invention, or T cells into which a nucleic acid is delivered can be used as an agent for immune cell therapy. Therefore, the present invention also provides a production method of a medicament containing T cells, including a step of contacting a cell population containing T cells with the nucleic acid delivery carrier of the present invention. In another embodiment, the present invention provides a production method of a medicament containing T cells, including a step of contacting a cell population containing T cells simultaneously with at least one kind of T cell activating ligand, and any of the above-mentioned nucleic acid delivery carriers without a T cell activating ligand added to the surface.

A cell population containing T cells to be brought into contact with the nucleic acid delivery carrier of the present invention, or a T cell activating ligand and a nucleic acid delivery carrier without a T cell activating ligand added to the surface may be an isolated T cell or, for example, a nonuniform cell population such as progenitor cells of lymphocytes including lymphocytes and pluripotent cells as long as it is a cell population containing T cell or a progenitor cell thereof. In the present invention, the "lymphocyte" means one of the subtypes of leukocyte in the immune system of vertebrates. Examples of the lymphocyte include T cell, B cell, and natural killer cell (NK cell), preferably, isolated and purified T cell. In the present invention, the "T cell" is one type of leukocyte found in lymphatic organs, peripheral blood, and the like, and refers to one category of lymphocyte characterized by differentiation and maturation mainly in the thymus gland and expression of TCR. Examples of the T cell that can be used in the present invention include cytotoxic T cell (CTL), which is a CD8-positive cell, helper T cell, which is a CD4-positive cell, regulatory T cell, and effector T cell, and preferably, cytotoxic T cell.

The aforementioned lymphocyte can be collected from, for example, peripheral blood, bone marrow, and umbilical cord blood of a human or non-human mammal. When ex vivo T cell contacted with the nucleic acid delivery carrier of the present invention is used for the treatment of diseases such as cancer, the cell population is preferably collected from the person to be treated or a donor with the HLA type matching with that of the subject to be treated.

A cell population containing T cells to be brought into contact with the nucleic acid delivery carrier of the present invention, or a T cell activating ligand and a nucleic acid delivery carrier without a T cell activating ligand added to the surface may be a cell population of T cells obtained by differentiation induction from progenitor cells of lymphocytes containing pluripotent cells. Examples of the lymphocyte progenitor cell, including pluripotent cell, include embryonic stem cell (ES cell), induced pluripotent stem cell (iPS cell), embryonic cancer cell (EC cell), embryonic germ cell (EG cell), hematopoietic stem cell, pluripotent progenitor cell that has lost self-renewal potential (multipotent progenitor: MMP), common myelo-lymphoid progenitor cell (MLP), myeloid progenitor cell (MP), granulocyte mononuclear progenitor cell (GMP), macrophage-dendritic cell progenitor cell (MDP), dendritic cell progenitor cell (DCP) and the like. Undifferentiated cells such as pluripotent cell and the like can be differentiated into T cell by a method known per se.

There is no particular limitation on the method of contacting the nucleic acid delivery carrier of the present invention, or T cell activating ligand and a nucleic acid delivery carrier without a T cell activating ligand added to the surface with ex vivo T cells, and, for example, the nucleic acid delivery carrier of the present invention, or T cell activating ligand and a nucleic acid delivery carrier without a T cell activating ligand added to the surface may be added to a typical medium for T cells. Therefore, in another aspect, the present invention also provides a cell culture containing a cell population containing T cells, the nucleic acid delivery carrier of the present invention, or at least one kind of T cell activating ligand and a nucleic acid delivery carrier without a T cell activating ligand added to the surface, and a medium.

In the nucleic acid delivery method of the present invention, to increase the efficiency of the delivery nucleic acid, for example, the calcium phosphate co-precipitation method, PEG method, electroporation method, microinjection method, lipofection method, and the like may be used in combination.

In the nucleic acid delivery method of the present invention, when the nucleic acid delivery carrier of the present invention, or a nucleic acid delivery carrier without a T cell activating ligand added to the surface particularly contains a nucleic acid encoding exogenous TCR in the inside, the expression of endogenous TCR $\alpha$ chain and TCR $\beta$ chain that are inherently expressed by the T cell may be suppressed by siRNA from the viewpoint of an increase in the expression of exogenous TCR, inhibition of the appearance of mispaired TCR, or inhibition of self-reactivity. When the above-mentioned nucleic acid is applied to the method, to avoid the effect of siRNA on exogenous TCR, the base sequence of a nucleic acid encoding the TCR is preferably a sequence (codon conversion type sequence) different from the base sequence corresponding to RNA on which siRNA, which suppresses the expression of endogenous TCR$\alpha$ and TCR$\beta$

chains, acts. The method therefor is described, for example, in WO 2008/153029. The aforementioned base sequence can be produced by introducing a silent mutation into a naturally acquired nucleic acid encoding TCR or chemically synthesizing an artificially designed nucleic acid. Alternatively, to avoid mispair with the endogenous TCR chain, a part or all of the constant regions of the nucleic acid encoding the exogenous TCR may be replaced with a constant region derived from an animal other than human, for example, a mouse.

Alternatively, as mentioned above, endogenous TCR gene may also be knocked out using a genome editing technique.

## 4. Nucleic acid delivery system containing at least one kind of T cell activating ligand, a nucleic acid delivery carrier without a T cell activating ligand added to the surface in combination

As mentioned above, a nucleic acid can be delivered into T cells by contacting a cell population contacting T cells simultaneously with at least one kind of T cell activating ligand, and any of the above-mentioned nucleic acid delivery carriers without a T cell activating ligand added to the surface. Therefore, the present invention also provides a nucleic acid delivery system containing at least one kind of T cell activating ligand, and a nucleic acid delivery carrier without a T cell activating ligand added to the surface in combination. In the nucleic acid delivery system, the T cell activating ligand and the nucleic acid delivery carrier without a T cell activating ligand added to the surface may be provided as a composition containing the both, or in the form of a kit containing the both as separate components. The nucleic acid delivery kit may further contain, in addition to the above-mentioned both components, for example, a medium to be used in contacting a cell population containing T cells with such components, and the like, though not limited to these.

## 5. Medicament containing ex vivo T cells activated and/or proliferated by T cell activating ligand and nucleic acid delivery carrier, or delivered with nucleic acid

The present invention also provides ex vivo T cells activated and/or proliferated by the activation/proliferation method of the present invention, ex vivo T cells having a nucleic acid delivered by the nucleic acid delivery method of the present invention (including cell culture containing medium), and a medicament containing them.

The ex vivo T cells activated and/or proliferated by the activation/proliferation method of the present invention specifically recognize cells expressing surface antigen specifically recognized by TCR expressed in the T cells and kill them (e.g., induction of apoptosis). In addition, the ex vivo T cells into which a nucleic acid encoding CAR or exogenous TCR is delivered by the nucleic acid delivery method of the present invention express the CAR or exogenous TCR, specifically recognize cells expressing surface antigen specifically recognized by the CAR or exogenous TCR and can kill them (e.g., induction of apoptosis). Therefore, ex vivo T cells in which T cells that express TCR recognizes a surface molecule that is specifically expressed as a surface antigen or showing enhanced expression in a disease cells, such as a cancer cell and the like, are activated and/or proliferated, and ex vivo T cells introduced with a nucleic acid encoding CAR or exogenous TCR that recognizes the surface molecule can be used for the prophylaxis or treatment of diseases such as cancer and the like, and can be safely administered to mammals (human or other mammal (e.g., mouse, rat, hamster, rabbit, cat, dog, bovine, sheep, monkey, preferably human)).

In a medicament containing, as an active ingredient, ex vivo T cells contacted with the nucleic acid delivery carrier of the present invention, or T cell activating ligand and a nucleic acid delivery carrier without a T cell activating ligand added to the surface, the T cells may be cultured using an appropriate medium before administration to a subject. In addition, the activation and/or proliferation of T cells can also be maintained or extended by adding a stimulation molecule to the medium. Furthermore, serum or plasma may be added to the medium. While the amount of addition to these media is not particularly limited, 0% by volume - 20% by volume can be mentioned. Moreover, the amount of serum or plasma to be used can be changed according to the culturing stage. For example, serum or plasma concentration can be reduced stepwise. The origin of serum or plasma may be either autologous or nonautologous, and autologous one is preferable from the aspect of safety.

The medicament containing, as an active ingredient, ex vivo T cells contacted with the nucleic acid delivery carrier of the present invention, or T cell activating ligand and a nucleic acid delivery carrier without a T cell activating ligand added to the surface is preferably used by parenteral administration to the subject. Examples of the method for parenteral administration include intravenous, intraarterial, intramuscular, intraperitoneal, and subcutaneous administration and the like. While the dose is appropriately selected according to the condition, body weight, age and the like of the subject, the medicament is generally administered such that the cell number is generally $1\times10^6$ - $1\times10^{10}$ cells, preferably $1\times10^7$ - $1\times10^9$ cells, more preferably $5\times10^7$ - $5\times10^8$ cells, per dose to a subject with body weight 60 kg. The medicament may be administered once, or in multiple divided portions. The medicament containing, as an active ingredient, ex vivo T cells contacted with the nucleic acid delivery carrier of the present invention can be formulated into a known form suitable for parenteral administration, for example, agent for injection or infusion. The medicament of the present invention may contain pharmacologically acceptable excipients as appropriate. The pharmacologically acceptable excipient includes those described above. The medicament may contain saline, phosphate buffered saline (PBS), medium and the like to maintain the cells stably. Examples of the medium include, but are not limited to, media such as RPMI, AIM-V, X-VIVO10 and the like. The medicament may be supplemented with a pharmaceutically acceptable carrier (e.g., human serum albumin), preservative and the like for stabilizing purposes.

The medicament containing, as an active ingredient, ex vivo T cells contacted with the nucleic acid delivery carrier of the present invention, or T cell activating ligand and a nucleic acid delivery carrier without a T cell activating ligand added to the surface can be a prophylactic or therapeutic drug for cancer. The cancer to be the application target for the medicament of the present invention is not particularly limited. Examples thereof include, but are not limited to, acute lymphocytic cancer, alveolar rhabdomyosarcoma, bladder cancer, bone cancer, brain cancer (e.g., medulloblastoma), breast cancer, anus, anal canal or anorectal cancer, cancer of the eye, cancer of the interhepatic bile duct, joint cancer, cervical, gallbladder or pleural cancer, nose, nasal cavity or middle ear cancer, oral cancer, vulvar cancer, chronic myelogenous cancer, colon cancer, esophageal cancer, cervical cancer, fibrosarcoma, gastrointestinal carcinoid tumor, head and neck cancer (e.g., head and neck squamous cell carcinoma), hypopharyngeal cancer, kidney cancer, laryngeal cancer, leukemia (e.g., acute lymphoblastic leukemia, acute lymphocytic leukemia, chronic lymphocytic leukemia, acute myeloid leukemia), liquid tumor, liver cancer, lung cancer (e.g., non-small cell lung cancer), lymphoma (e.g., Hodgkin lymphoma, non-Hodgkin lymphoma, diffuse large B cell lymphoma, follicular lymphoma), malignant mesothelioma, mastocytoma, melanoma, multiple myeloma, nasopharyngeal cancer, ovarian cancer, pancreatic cancer; peritoneal, omentum and mesenteric cancer; pharyngeal cancer, prostate cancer, rectal cancer, renal cancer, skin cancer, small intestine cancer, soft tissue cancer, solid tumor, gastric cancer, testicular cancer, thyroid cancer, ureteral cancer and the like.

6. Medicament containing the nucleic acid delivery carrier of the present invention

The nucleic acid delivery carrier of the present invention can induce activation and/or proliferation of T cells in living organisms by in vivo administration to mammals such as human and the like. In addition, by in vivo administration of the nucleic acid delivery carrier of the present invention containing inside a nucleic acid encoding CAR or exogenous TCR to mammals such as human and the like, the nucleic acid is delivered and expressed in T cells in the living organism, which in turn can impart the T cells with an ability to specifically recognize cells (e.g., cancer cells) expressing surface antigen (e.g., cancer antigen) specifically recognized by CAR or exogenous TCR and kill them (e.g., induction of apoptosis). Therefore, the present invention also provides a medicament containing the nucleic acid delivery carrier of the present invention.

A medicament containing the nucleic acid delivery carrier of the present invention is preferably prepared as a pharmaceutical composition by mixing the nucleic acid delivery carrier with known pharmaceutically acceptable carriers (including excipient, diluent, bulking agent, binder, lubricant, flow aid, disintegrant, surfactant, and the like) and conventional additives, and the like. The excipients are well known to those of ordinary skill in the art and include, for example, phosphate-buffered saline (e.g., 0.01M phosphate, 0.138M NaCl, 0.0027M KCl, pH 7.4), aqueous solutions containing mineral acid salts such as hydrochloride, hydrobromate, phosphate, sulfate, and the like, saline solutions, solutions of glycol, ethanol, and the like, and salts of organic acids such as acetate, propionate, malonate, benzoate, and the like. In addition, adjuvants such as wetting agent or emulsifier, and pH buffering agents can also be used. In addition, preparation adjuvants such as suspension agent, preservative, stabilizer and dispersing agent may also be used. Alternatively, the above-mentioned pharmaceutical composition may be in a dry form which is reconstituted with a suitable sterile liquid prior to use. The pharmaceutical composition may be orally or parenterally administered systemically or topically, depending on the form in which it is prepared (oral agents such as tablet, pill, capsule, powder, granule, syrup, emulsion, suspension and the like; parenteral agents such as injection, drip transfusion, external preparation, suppository and the like). For parenteral administration, intravenous administration, intradermal administration, subcutaneous administration, rectal administration, transdermal administration and the like are available. When used in an injectable form, acceptable buffering agent, solubilizing agent, isotonic agent and the like can also be added.

The dosage of the medicament of the present invention containing the nucleic acid delivery carrier of the present invention is, for example, in the range of 0.001 mg to 10 mg as the amount of a nucleic acid encoding CAR or exogenous TCR, per 1 kg body weight per dose. For example, when administered to a human patient, the dosage is in the range of 0.0001 to 50 mg for a patient weighing 60 kg. The above-mentioned dosage is an example, and the dosage can be appropriately selected according to the type of nucleic acid to be used, administration route, age, weight, symptoms, etc. of the subject of administration or patient.

By administration to a mammal (e.g., human or other mammal (e.g., mouse, rat, hamster, rabbit, cat, dog, bovine, sheep, monkey), preferably, human), a medicament containing the nucleic acid delivery carrier of the present invention can induce the expression of CAR or exogenous TCR in T cell (in vivo T cell) in the body of the animal. The in vivo T cells kill diseased cells such as cancer cells and the like expressing surface antigen targeted by CAR or exogenous TCR, thereby demonstrating a prophylactic or therapeutic effect against the disease.

A medicament containing the nucleic acid delivery carrier of the present invention may be a prophylactic or therapeutic drug for cancer. The cancer to be the application target of the medicament of the present invention is not particularly limited. Examples thereof include, but are not limited to, acute lymphocytic cancer, alveolar rhabdomyosarcoma, bladder cancer, bone cancer, brain cancer (e.g., medulloblastoma), breast cancer, anus, anal canal or anorectal cancer, cancer of the eye, cancer of the interhepatic bile duct, joint cancer, cervical, gallbladder or pleural cancer, nose, nasal cavity or middle ear cancer, oral cancer, vulvar cancer, chronic myelogenous cancer, colorectal cancer, esophageal cancer, cervical cancer, fibrosarcoma, gastrointestinal carcinoid tumor, head and neck cancer (e.g., head and neck squamous cell carcinoma), hypopharyngeal cancer, kidney cancer, laryngeal cancer, leukemia (e.g., acute lymphoblastic leukemia, acute lymphocytic leukemia, chronic lymphocytic leukemia, acute myelogenous leukemia), liquid tumor, liver cancer, lung cancer (e.g., non-small cell lung cancer), lymphoma (e.g., Hodgkin lymphoma, non-Hodgkin lymphoma, diffuse large B cell lymphoma, follicular lymphoma), malignant mesothelioma, mastocytoma, melanoma, multiple myeloma, nasopharyngeal cancer, ovarian cancer, pancreatic cancer; peritoneal, omentum and mesenteric cancer; pharyngeal cancer, prostate cancer, rectal cancer, kidney cancer, skin cancer, small intestinal cancer, soft tissue cancer, solid tumor, gastric cancer, testicular tumor, thyroid cancer, ureteral cancer and the like.

The present invention is explained in more detail in the following by referring to Examples which are mere exemplifications and do not limit the present invention.

[Example]

## 1. Reduction treatment of antibody

9.21 mg/ml anti-CD3 antibody and anti-CD28 antibody (Bio X Cell company) solutions (each 111 μl) were mixed with 10 mM DTT aqueous solution (12.3 μl). The mixture of each antibody and DTT was mixed by vortex to carry out reaction at room temperature for 30 min. The reaction mixture was fractionated by HPLC (column: TSKgel G2000SWXL 7.8 mm×30 cm, TOSOH, mobile phase: PBS) to give a fraction solution containing the reduced antibody. The fraction solution was concentrated by ultrafiltration using Amicon 0.5 ml-10K. The concentrations of the antibody protein and thiol group in the concentrates were measured by absorbance at 230 nm and a fluorescence colorimetric reaction with N-(7-dimethylamino-4-methylcoumarin-3-yl)maleimide (DACM), respectively.

## 2. Preparation of maleimide-lipid nanoparticles

A lipid mixture containing compound 7, 11, 12, 21, 31 or 35 as cationic lipid (cationic lipid:DPPC:Cholesterol:SUNBRIGHT GM-020:SUNBRIGHT DSPE-020MA=60:10.6:28:1.4:1, molar ratio) was dissolved in 90% EtOH, 10% water to give a 7.0 mg/ml lipid solution. On the other hand, luciferase mRNA (TriLink company) was dissolved in 2-morpholinoethanesulfonic acid (MES) buffer (pH 5.0) to give 0.2 mg/ml mRNA solution. The obtained lipid solution and mRNA solution were mixed at room temperature by a Nanoassemblr apparatus (Precision Nanosystems) at a flow rate ratio of 3 ml/min:6 ml/min to give a dispersion containing the composition. The obtained dispersion was dialyzed using Slyde-A-Lyzer (20k fraction molecular weight, Thermo Scientific) against water at room temperature for 1 hr, and against PBS at 4°C for 48 hr. Successively, the dialysate was filtered through a 0.2 μm syringe filter (Iwaki) and stored at 4°C.

## 3. Binding reaction of reduced antibody and Maleimide-lipid nanoparticles

Maleimide-lipid nanoparticle dispersion was mixed with reduced antibody solution such that the molar concentration of reduced antibody was 1/20 of that of maleimide, and allowed to stand at room temperature for 4 hr. Thereafter, the mixture was stored at 4°C until the purification step.

## 4. Gel filtration purification of antibody-lipid nanoparticles

A reaction mixture of a reduced antibody and Maleimide-lipid nanoparticles was loaded on a gel filtration column Sepharose CL-4B (Cat No. 17-0150-01/GE Healthcare), and fractionated with D-PBS(-) as a mobile phase. Successively, the protein concentration of each fraction was measured to identify the fraction containing the antibody-lipid nanoparticles of interest, whereby antibody-lipid nanoparticles stock solution was obtained. The antibody-lipid nanoparticles were filtered through a 0.2 μm syringe filter and stored at 4°C.

## 5. Particle size measurement of antibody-lipid nanoparticles

To 1 μl of an antibody-lipid nanoparticle stock solution was added 99 μl of phosphate buffered saline (137 mM NaCl, 7.99 mM $Na_2HPO_4$, 2.7 mM KCl, 1.47 mM $KH_2PO_4$, pH 7.4). The obtained dispersion was subjected to dynamic

light scattering measurement using Zetasizer Nano ZS (Malvern instruments), and the cumulant analysis of the autocorrelation function was performed to measure the Z average particle size and the polydispersity index (PDI).

6. ζ electric potential measurement of antibody-lipid nanoparticles

To 50 μl of an antibody-lipid nanoparticle stock solution was added 700 μl of HEPES buffer (10 mM HEPES-NaOH, pH 7.3). The obtained dispersion was subjected to electrophoretic light scattering measurement using Zetasizer Nano ZS (Malvern instruments) to measure the ζ electric potential.

7. Measurement of mRNA encapsulation rate and concentration of antibody-lipid nanoparticles

An antibody-lipid nanoparticle stock solution was diluted with TE buffer to adjust the mRNA concentration to about 4 μg/ml. As an mRNA concentration standard solution, naked mRNA was diluted with TE buffer to 4 μg/ml. The diluted antibody-lipid nanoparticles and naked mRNA concentration standard solution (each 60 μl) were each mixed with 60 μl of TE buffer or TE buffer containing 2% Triton-X100. The mixture was allowed to stand at room temperature for 5 min, mixed with 120 μl of Quant-iT™ RiboGreen (registered trade mark), and the mixture was further allowed to stand for 5 min. The fluorescence intensity of the mixture was measured using Envision microplate reader (Perkin-Elmer company). The mRNA encapsulation rate and mRNA concentration were calculated by the following formulas.

$$\%\ \text{mRNA encapsulation rate} = (1-F_{TE}/F_{Triton})\times 100$$

$$\text{mRNA concentration} = (F_{Triton}-b)\times d/m$$

(wherein $F_{TE}$ shows RiboGreen fluorescence intensity of lipid nanoparticles mixed with TE buffer, $F_{Triton}$ shows RiboGreen fluorescence intensity of lipid nanoparticles mixed with TE buffer containing 2% Triton-X100, b and m show y-intercept and slope obtained from the calibration curve of the concentration standard siRNA, and d is the dilution rate of lipid nanoparticles)

A list of the analysis results of the prepared antibody-lipid nanoparticles is shown in the following Table.

[Table 1]

| | particle size (nm) | polydispersity index | mRNA encapsulation rate (%) | mRNA concentration (μg/ml) | antibody concentration (μg/ml) |
|---|---|---|---|---|---|
| anti-CD3-compound 7 | 78 | 0.114 | 95 | 94.1 | 20.4 |
| anti-CD3-compound 11 | 78 | 0.071 | 97 | 87.0 | 29.4 |
| anti-CD3-compound 12 | 99 | 0.134 | 96 | 93.9 | 27.6 |
| anti-CD3-compound 31 | 135 | 0.121 | 90 | 92.3 | 49.6 |
| anti-CD3-compound 21 | 89 | 0.095 | 96 | 73.1 | 72.7 |
| anti-CD3-compound 35 | 108 | 0.129 | 97 | 72.1 | 65.3 |
| anti-CD28-compound 12 | 93 | 0.137 | 94 | 128 | 10.1 |
| anti-CD3/anti-CD28-compound 12 | 97 | 0.068 | 98 | 30.7 | 55.1 |

8. mRNA transfection into human primary cultured T cells with lipid nanoparticles bound to anti-CD3 antibody

Human peripheral blood CD3 positive pan T cells (Precision Bioservices) were seeded on a round-bottomed 96-well plate (Corning) at a cell density of $1\times10^5$ cells/well. A serumfree hematopoietic cell medium X-VIVO10 (Lonza) supplemented with 30 ng/ml recombinant IL-2 (Thermo Fisher Scientific) was used as the medium. Subsequently, anti-CD3 antibody-bound lipid nanoparticles encapsulating luciferase mRNA (TriLink) were added to the medium such that the concentration of luciferase mRNA in the medium was 1 $\mu$g/ml, and the mixture was stood in a 5% $CO_2$ incubator at 37°C for 72 hr. Luciferase expressed in T cells was measured using Bright-Glo Luciferase Assay System Kit (Promega). The relative luciferase luminescence intensity of T cells supplemented with each anti-CD3 antibody-lipid nanoparticle is shown in Fig. 1.

9. mRNA transfection into human primary cultured T cells with lipid nanoparticles bound to anti-CD3 antibody and/or anti-CD28 antibody

Human peripheral blood CD3 positive pan T cells (Precision Bioservices) were seeded on a round-bottomed 96-well plate (Corning) at a cell density of $1\times10^5$ cells/well. A serumfree hematopoietic cell medium X-VIVO10 (Lonza) supplemented with 30 ng/ml recombinant IL-2 (Thermo Fisher Scientific) was used as the medium. Subsequently, anti-CD3 antibody-bound lipid nanoparticles encapsulating luciferase mRNA (TriLink), anti-CD28 antibody-bound lipid nanoparticles, a mixture of anti-CD3 antibody-bound lipid nanoparticles and anti-CD28 antibody-bound lipid nanoparticles, and lipid nanoparticles bound with a mixture of anti-CD3 antibody and anti-CD28 antibody were added to the medium such that the concentration of luciferase mRNA in the medium was 1 $\mu$g/ml, and the mixture was stood in a 5% $CO_2$ incubator at 37°C for 72 hr. Luciferase expressed in T cells was measured using Bright-Glo Luciferase Assay System Kit (Promega). The relative luciferase luminescence intensity of T cells supplemented with each antibody-lipid nanoparticle is shown in Fig. 2.

10. mRNA transfection into human primary cultured T cells with lipid nanoparticles bound to anti-CD3/anti-CD28 antibody and T cell activation stimulation

Human peripheral blood CD3 positive pan T cells (Precision Bioservices) were seeded on a round-bottomed 96-well plate (Corning) at a cell density of $1\times10^5$ cells/well. A serumfree hematopoietic cell medium X-VIVO10 (Lonza) supplemented with 30 ng/ml recombinant IL-2 (Thermo Fisher Scientific) was used as the medium. Subsequently, lipid nanoparticles encapsulating luciferase mRNA (TriLink) and bound to anti-CD3 antibody and anti-CD28 antibody were added to the medium such that the concentration of luciferase mRNA in the medium was 0.3 or 1 $\mu$g/ml, and the mixture was stood in a 5% $CO_2$ incubator at 37°C for 72 hr. As a control sample of T cell activation stimulation, T cells supplemented with TransAct (Miltenyi Biotec company) and Dynabeads (Thermo Fisher Scientific company) were also prepared. Luciferase expressed in T cells was measured using Bright-Glo Luciferase Assay System Kit (Promega). The number of viable T cells was measured using CellTiter-Glo kit (Promega). The expression level of Luciferase is shown in Fig. 3(I), and the survival number of T cells is shown in Fig. 3(II) and (III).

11. Preparation of Luc mRNA-encapsulating lipid nanoparticles

A lipid mixture containing compound 35 as cationic lipid (compound 35:DPPC:Cholesterol:SUNBRIGHT GM-020:SUNBRIGHT = 60:10.6:28:1.4 molar ratio) was dissolved in 90% EtOH, 10% water to give a 8.1 mg/ml lipid solution. On the other hand, luciferase mRNA (Luc mRNA) (TriLink company) was dissolved in 2-morpholinoethanesulfonic acid (MES) buffer (pH 5.0) to give 0.18 mg/ml mRNA solution. The obtained lipid solution and mRNA solution were mixed at room temperature by a Nanoassemblr apparatus (Precision Nanosystems) at a flow rate ratio of 3 ml/min:6 ml/min to give a dispersion of Luc mRNA-encapsulating lipid nanoparticles. The obtained dispersion was dialyzed using Slyde-A-Lyzer (molecular weight 20k for fraction, Thermo Scientific) against water at room temperature for 1 hr, and against PBS at 4°C for 48 hr. Successively, the dialysate was filtered through a 0.2 $\mu$m syringe filter (Iwaki) and stored at 4°C.

12. Particle size measurement of Luc mRNA-encapsulating lipid nanoparticles

To 1 $\mu$l of a Luc mRNA-encapsulating lipid nanoparticles stock solution was added 99 $\mu$l of phosphate buffered saline (137 mM NaCl, 7.99 mM $Na_2HPO_4$, 2.7 mM KCl, 1.47 mM $KH_2PO_4$, pH 7.4). The obtained dispersion was subjected to dynamic light scattering measurement using Zetasizer Nano ZS (Malvern instruments), and the cumulant analysis of the autocorrelation function was performed to measure the Z average particle size and the polydispersity index.

13. ζ potential measurement of Luc mRNA-encapsulating lipid nanoparticles

To 50 μl of a Luc mRNA-encapsulating nanoparticle stock solution was added 700 μl of HEPES buffer (10 mM HEPES-NaOH, pH 7.3). The obtained dispersion was subjected to electrophoretic light scattering measurement using Zetasizer Nano ZS (Malvern instruments) to measure the ζ potential.

14. Measurement of encapsulation rate and concentration of mRNA for Luc mRNA-encapsulating lipid nanoparticles

A Luc mRNA-encapsulating nanoparticle stock solution was diluted with TE buffer to adjust the mRNA concentration to about 4 μg/ml. As an mRNA concentration standard solution, naked mRNA was diluted with TE buffer to 4 μg/ml. The diluted Luc mRNA-encapsulating nanoparticles and naked mRNA concentration standard solution (each 60 μl) were each mixed with 60 μl of TE buffer or TE buffer containing 2% Triton-X100. The mixture was allowed to stand at room temperature for 5 min, mixed with 120 μl of Quant-iT™ RiboGreen (registered trade mark), and the mixture was further allowed to stand for 5 min. The fluorescence intensity of the mixture was measured using Envision microplate reader (Perkin-Elmer company). The mRNA encapsulation rate and mRNA concentration were calculated by the following formulas.

$$\%\ \text{mRNA encapsulation rate} = (1-F_{TE}/F_{Triton})\times 100$$

$$\text{mRNA concentration} = (F_{Triton}-b)\times d/m$$

(wherein $F_{TE}$ shows RiboGreen fluorescence intensity of lipid nanoparticles mixed with TE buffer, $F_{Triton}$ shows RiboGreen fluorescence intensity of lipid nanoparticles mixed with TE buffer containing 2% Triton-X100, b and m show y-intercept and slope obtained from the calibration curve of the concentration standard siRNA, and d is the dilution rate of lipid nanoparticles)

The analysis results of the Luc mRNA-encapsulating lipid nanoparticles are shown in Table 2.

[Table 2]

| composition | Z-average particle size | polydispersity index | zeta potential | mRNA encapsulation rate |
|---|---|---|---|---|
| compound 35-luc mRNA | 88 nm | 0.029 | -0.2 mV | 96% |

15. Highly efficient mRNA transfection into human peripheral blood CD3 positive pan T cells by co-addition of activation stimulant and lipid nanoparticles

Human peripheral blood CD3 positive pan T cells (Precision Bioservices) were seeded on a round-bottomed 96-well plate (Corning) at a cell density of $1\times10^5$ cells/well. A serumfree hematopoietic cell medium X-VIVO10 (Lonza) supplemented with 30 ng/ml recombinant IL-2 (Thermo Fisher Scientific), and with TransAct (Milteny Biotech) or Dynabeads Human T-Activator CD3/CD28 (ThermoFisher Scientific), which stimulates activation of T cells, according to the protocol recommended by each manufacturer, was used as the medium. Subsequently, lipid nanoparticle compound 35-luc mRNA encapsulating luciferase mRNA (TriLink) were added to the medium such that the concentration of luciferase mRNA in the medium was 0.1, 0.3 or 1 μg/ml, and the mixture was stood in a 5% $CO_2$ incubator at 37°C for 72 hr. Luciferase expressed in T cells was measured using Bright-Glo Luciferase Assay System Kit (Promega). The survival and proliferation rate of T cells was measured using CellTiter-Glo Luminescent Cell Viability Assay kit (Promega KK). The obtained results are shown in Figs. 4 and 5. It was shown that addition of lipid nanoparticles encapsulating Luc mRNA to T cells under activation stimulation dramatically improves transfection activity (Fig. 4). In addition, the survival and proliferation rate of T cells was maintained at a high level (Fig. 5).

16. Highly efficient luc mRNA transfection into human CD4/CD8 positive T cells by co-addition of activation stimulant and lipid nanoparticles

Human peripheral blood leukocyte fraction Leukopak (HemaCare) was washed with LOVO Cell Processing System (Fresenius), and CD4 and CD8 positive cells were collected with cell processing system CliniMACS (Milteny Biotec). The obtained CD4/CD8 positive cells were seeded on a flat-bottomed 96-well plate (Corning) at a cell density of $1\times10^5$ cells/well. For cell culture, a medium supplemented with 26 mL of OPTmizer CTS T-cell Expansion Supplement,

20 mL of CTS Immune SR, 10 mL of L-Glutamine 200 mM (all ThermoFischer Scientific), 10 mL of Streptomycin Sulfate 10 mg/ml (MEIJI company), and 4.2 ng/ml MACS GMP Recombinant Human IL-2 (Milteny Biotec), per 1 L of OPTmizer CTS T-Cell Expansion Basal medium, was used. In addition, as a T cell activation stimulant, TransAct (Milteny Biotech) was added according to the protocol recommended by the manufacturer. Subsequently, lipid nanoparticle compound 35-luc mRNA encapsulating luciferase mRNA (TriLink) were added to the medium such that the concentration of luciferase mRNA in the medium was 1, 3 or 10 μg/ml, and the mixture was stood in a 5% $CO_2$ incubator at 37°C for 72 hr. Luciferase expressed in T cells was measured using Bright-Glo Luciferase Assay System Kit (Promega). The survival and proliferation rate of T cells was measured using CellTiter-Glo Luminescent Cell Viability Assay kit (Promega KK). The obtained results are shown in Fig. 6. It was shown that addition of lipid nanoparticles encapsulating Luc mRNA to T cells under activation stimulation dramatically improves transfection activity. In addition, the survival and proliferation rate of T cells was maintained at a high level.

[Industrial Applicability]

Using the nucleic acid delivery carrier of the present invention or the nucleic acid delivery method of the present invention, a step of activating/proliferating T cells and a step of introducing a gene into T cells can be performed simultaneously in one pod. As a result, an agent for immune cell therapy can be provided in a short period of time at a low production cost, and the present invention is extremely useful since an immunocyte therapy can be provided at a lower cost.

This application is based on a patent application No. 2018-197069 filed in October 18, 2018 and a patent application No. 2019-124629 filed in July 3, 2019, the contents of which are hereby incorporated by reference in full herein.

## Claims

1. A method for activating/proliferating T cells, comprising a step of contacting a cell population containing T cells with a nucleic acid delivery carrier having at least one kind of T cell activating ligand added to its surface.

2. The method according to claim 1, wherein the aforementioned T cell activating ligand includes an antibody to CD3 and/or an antibody to CD28.

3. The method according to claim 1, wherein the aforementioned nucleic acid delivery carrier has two or more kinds of T cell activating ligands added to a surface thereof.

4. The method according to claim 1, wherein the aforementioned nucleic acid delivery carrier is a lipid nanoparticle or a liposome.

5. The method according to claim 1, wherein the aforementioned nucleic acid delivery carrier comprises, in its inside, a nucleic acid that suppresses expression of a T cell activation inhibitory factor and/or a nucleic acid encoding a T cell activation promoting factor.

6. The method according to claim 1, wherein the aforementioned nucleic acid delivery carrier comprises a nucleic acid encoding CAR or TCR.

7. The method according to claim 1, wherein the method is performed ex vivo.

8. A method for delivering a nucleic acid into T cells, comprising a step of contacting a cell population containing T cells with a nucleic acid delivery carrier having at least one kind of T cell activating ligand added to its surface and containing a nucleic acid inside, wherein the nucleic acid does not comprise a nucleic acid encoding CAR or TCR.

9. The method according to claim 8, wherein the aforementioned T cell activating ligand includes an antibody to CD3 and/or an antibody to CD28.

10. The method according to claim 8, wherein the aforementioned nucleic acid delivery carrier has two or more kinds of T cell activating ligands added to a surface thereof.

11. The method according to claim 8, wherein the aforementioned nucleic acid delivery carrier is a lipid nanoparticle or a liposome.

**12.** The method according to claim 8, wherein the aforementioned nucleic acid comprises a nucleic acid suppressing expression of a T cell activation inhibitory factor and/or a nucleic acid encoding a T cell activation promoting factor.

**13.** The method according to claim 8, wherein the method is performed ex vivo.

**14.** A method for delivering a nucleic acid into T cells, comprising a step of contacting a cell population containing T cells simultaneously with at least one kind of T cell activating ligand, and a nucleic acid delivery carrier containing a nucleic acid inside and free of a T cell activating ligand added to its surface.

**15.** The method according to claim 14, wherein the aforementioned T cell activating ligand includes an antibody to CD3 and/or an antibody to CD28.

**16.** The method according to claim 14, wherein two or more kinds of T cell activating ligands are contacted.

**17.** The method according to claim 14, wherein the aforementioned nucleic acid delivery carrier is a lipid nanoparticle or a liposome.

**18.** The method according to claim 14, wherein the aforementioned nucleic acid includes a nucleic acid suppressing expression of a T cell activation inhibitory factor and/or a nucleic acid encoding a T cell activation promoting factor.

**19.** The method according to claim 14, wherein the aforementioned nucleic acid comprises a nucleic acid encoding CAR or TCR.

**20.** The method according to claim 14, wherein the method is performed ex vivo.

**21.** A method for producing a medicament comprising T cells, comprising a step of contacting a cell population containing T cells simultaneously with at least one kind of T cell activating ligand, and a nucleic acid delivery carrier containing a nucleic acid inside and free of a T cell activating ligand added to its surface.

**22.** The method according to claim 21, wherein the aforementioned T cell activating ligand includes an antibody to CD3 and/or an antibody to CD28.

**23.** The method according to claim 21, wherein two or more kinds of T cell activating ligands are contacted.

**24.** The method according to claim 21, wherein the aforementioned nucleic acid delivery carrier is a lipid nanoparticle or a liposome.

**25.** The method according to claim 21, wherein the aforementioned nucleic acid delivery carrier comprises a nucleic acid suppressing expression of a T cell activation inhibitory factor and/or a nucleic acid encoding a T cell activation promoting factor.

**26.** The method according to claim 21, wherein the aforementioned nucleic acid comprises a nucleic acid encoding CAR or TCR.

**27.** The method according to claim 21, wherein the method is performed ex vivo.

**28.** A T cell into which a nucleic acid has been delivered by the method according to claim 14.

**29.** A medicament comprising the T cell according to claim 28.

**30.** A cell culture comprising a cell population containing T cells, at least one kind of T cell activating ligand, a nucleic acid delivery carrier without a T cell activating ligand added to the surface, and a medium.

**31.** A composition for delivering a nucleic acid to T cells, comprising at least one kind of T cell activating ligand, and a nucleic acid delivery carrier without a T cell activating ligand added to the surface.

**32.** A kit for delivering a nucleic acid into T cells, comprising at least one kind of T cell activating ligand, and a nucleic acid delivery carrier without a T cell activating ligand added to the surface.

Fig. 1

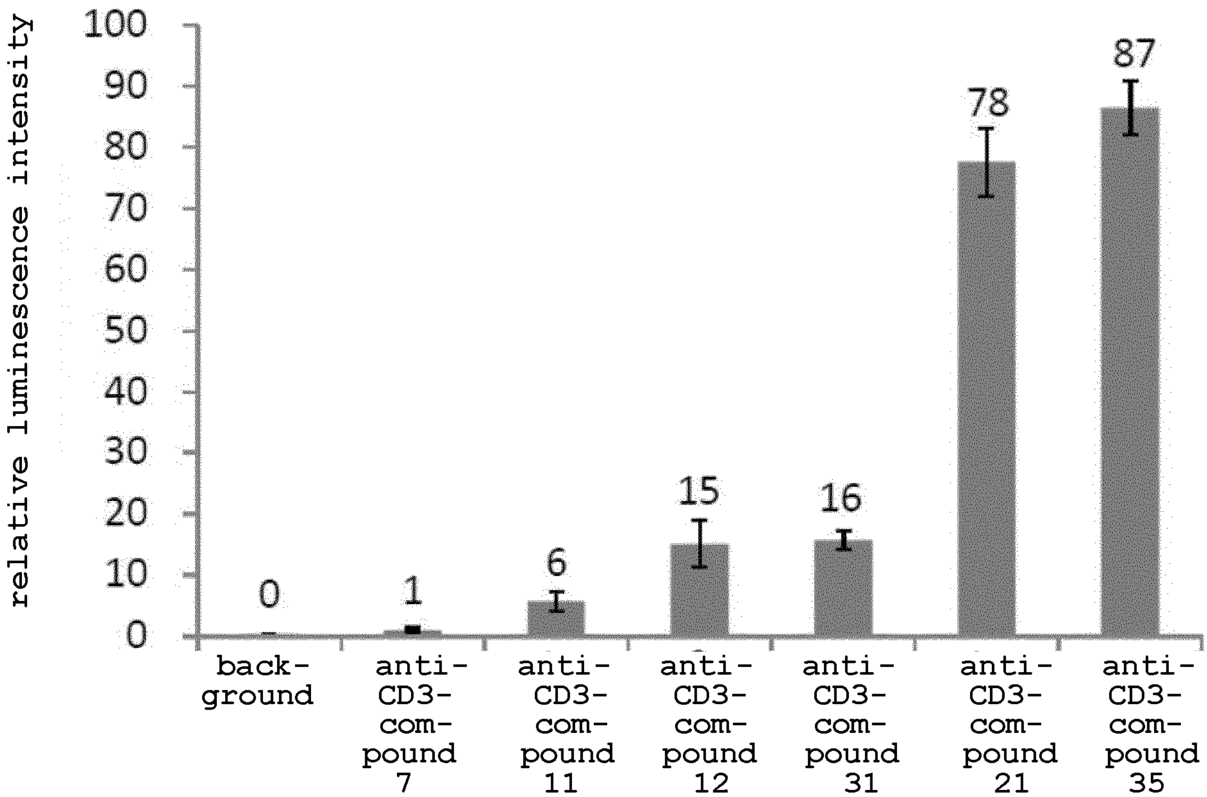
relative luminescence intensity
100
90
80
70
60
50
40
30
20
10
0
0
1
6
15
16
78
87
back-ground
anti-CD3-com-pound 7
anti-CD3-com-pound 11
anti-CD3-com-pound 12
anti-CD3-com-pound 31
anti-CD3-com-pound 21
anti-CD3-com-pound 35

Fig. 2

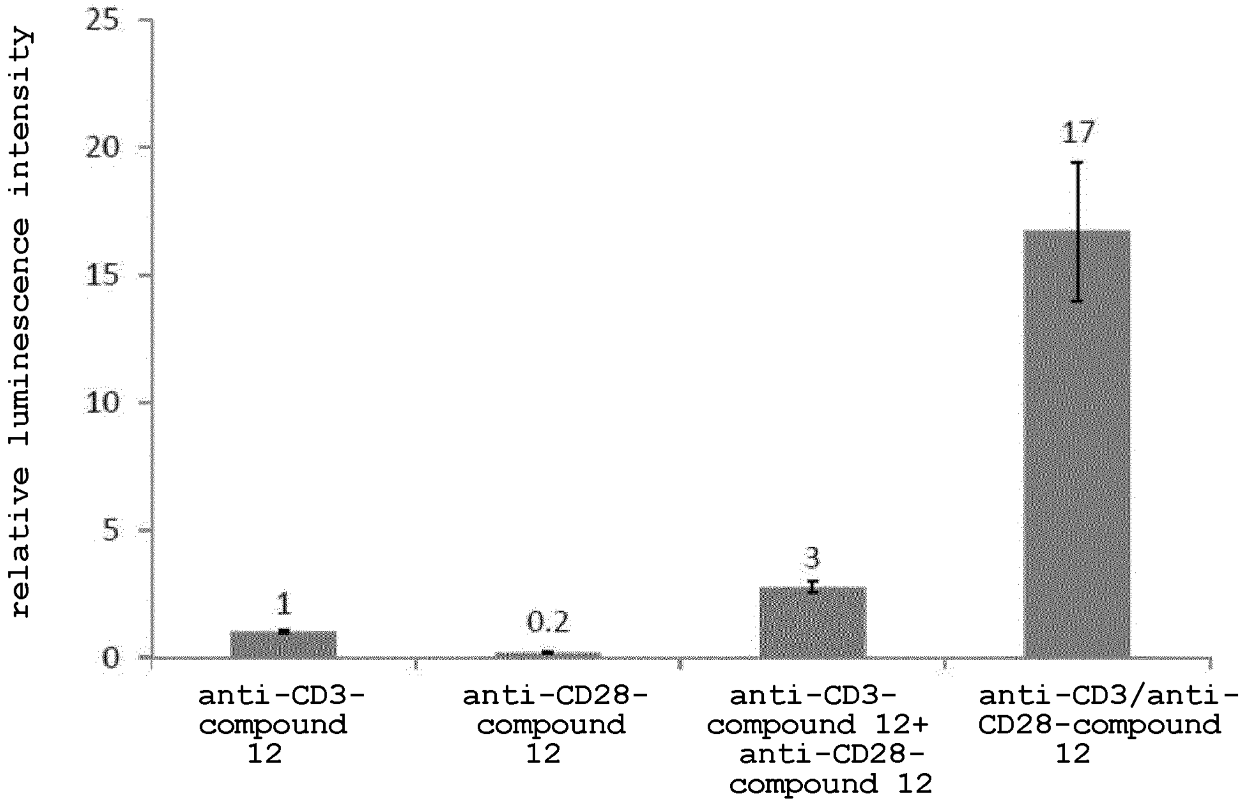
relative luminescence intensity
25
20
15
10
5
0
1
0.2
3
17
anti-CD3-compound 12
anti-CD28-compound 12
anti-CD3-compound 12+ anti-CD28-compound 12
anti-CD3/anti-CD28-compound 12

Fig. 3

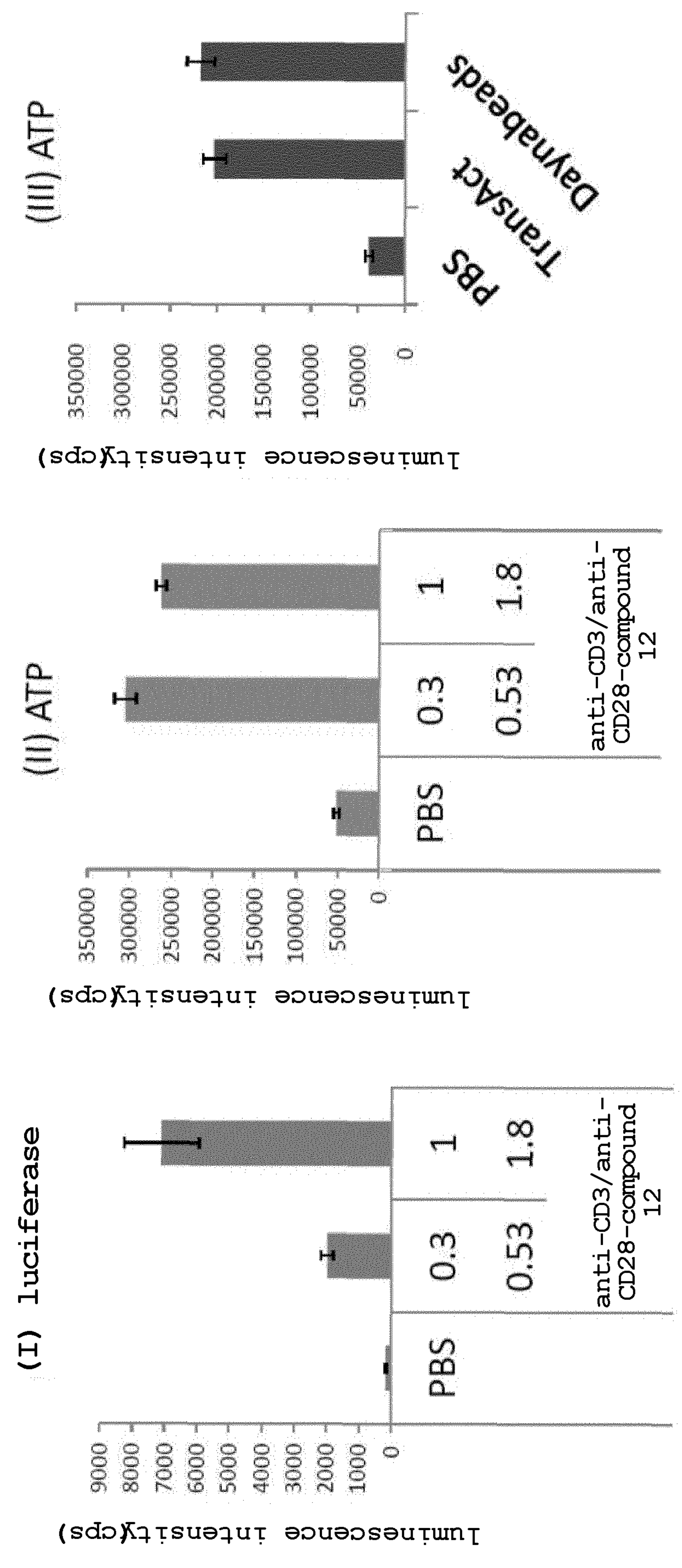
(I) luciferase
Luminescence intensity(cps)
9000
8000
7000
6000
5000
4000
3000
2000
1000
0
PBS
0.3
1
0.53
1.8
anti-CD3/anti-CD28-compound 12
(II) ATP
Luminescence intensity(cps)
350000
300000
250000
200000
150000
100000
50000
0
PBS
0.3
1
0.53
1.8
anti-CD3/anti-CD28-compound 12
(III) ATP
Luminescence intensity(cps)
350000
300000
250000
200000
150000
100000
50000
0
PBS
TransAct
Daynabeads

Fig. 4

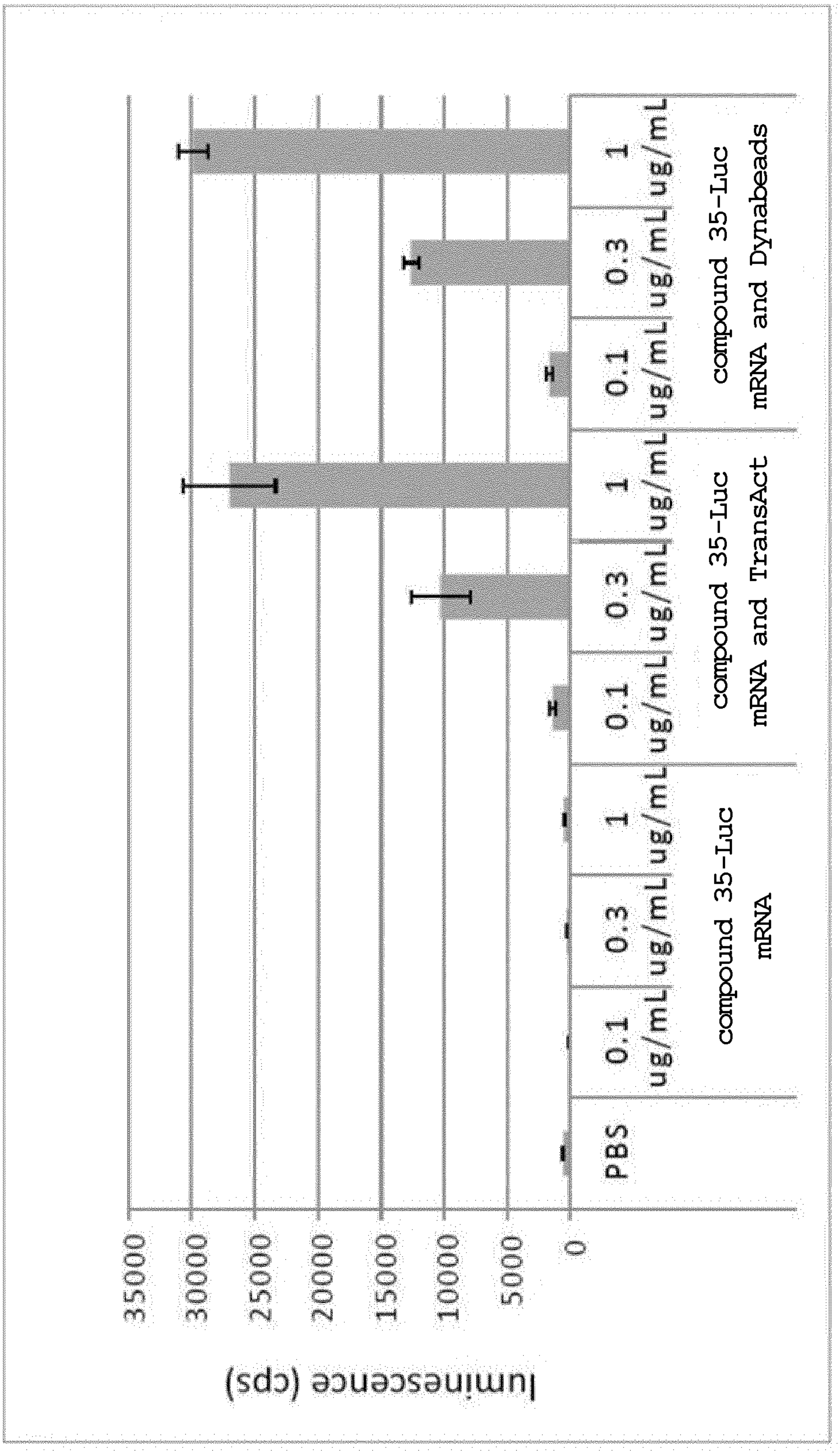
luminescence (cps)
35000
30000
25000
20000
15000
10000
5000
0
PBS
0.1 ug/mL
0.3 ug/mL
1 ug/mL
compound 35-Luc mRNA
0.1 ug/mL
0.3 ug/mL
1 ug/mL
compound 35-Luc mRNA and TransAct
0.1 ug/mL
0.3 ug/mL
1 ug/mL
compound 35-Luc mRNA and Dynabeads

Fig. 5

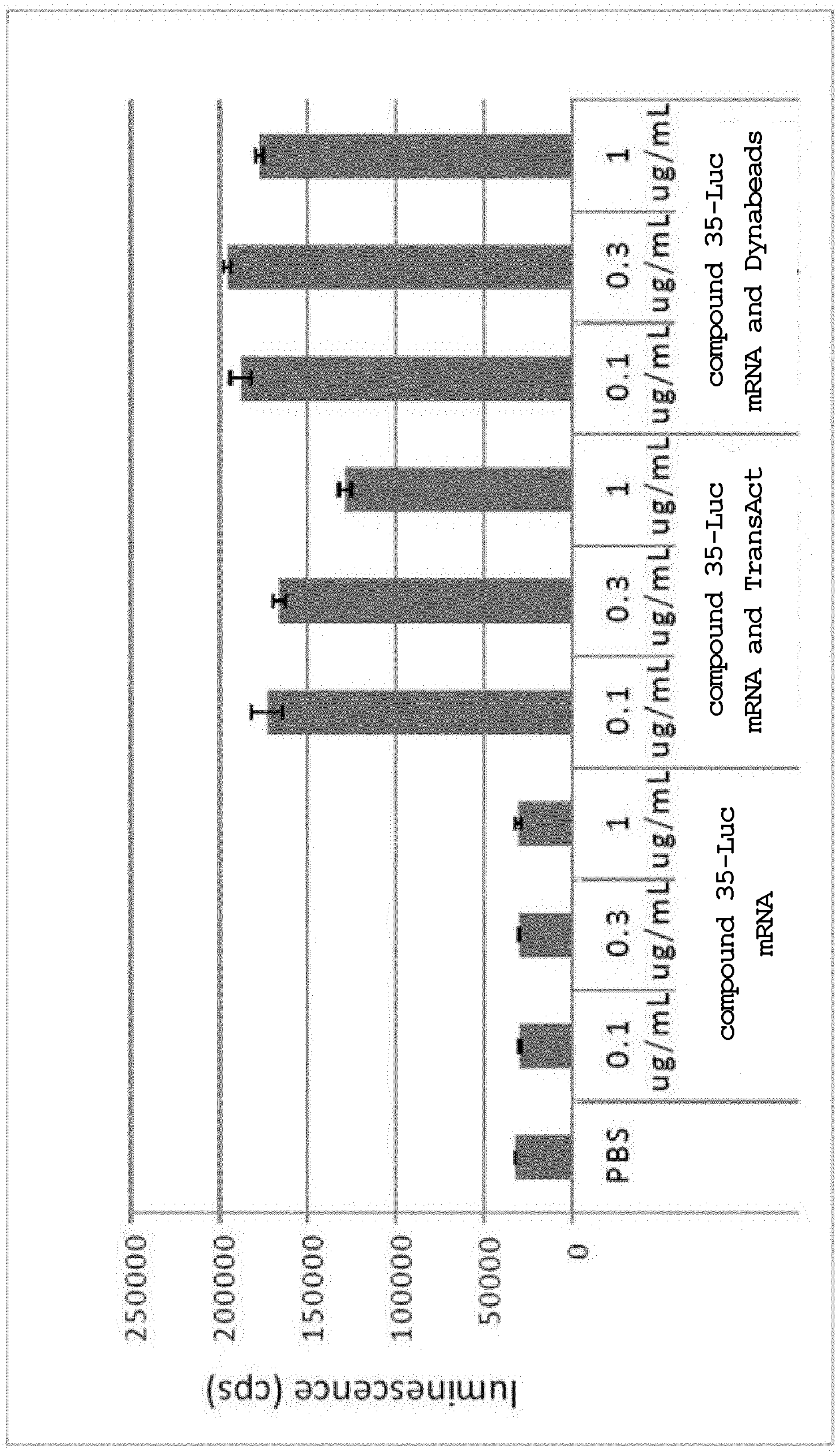
luminescence (cps)
250000
200000
150000
100000
50000
0
PBS
0.1 ug/mL
0.3 ug/mL
1 ug/mL
compound 35-Luc mRNA
0.1 ug/mL
0.3 ug/mL
1 ug/mL
compound 35-Luc mRNA and TransAct
0.1 ug/mL
0.3 ug/mL
1 ug/mL
compound 35-Luc mRNA and Dynabeads

Fig. 6

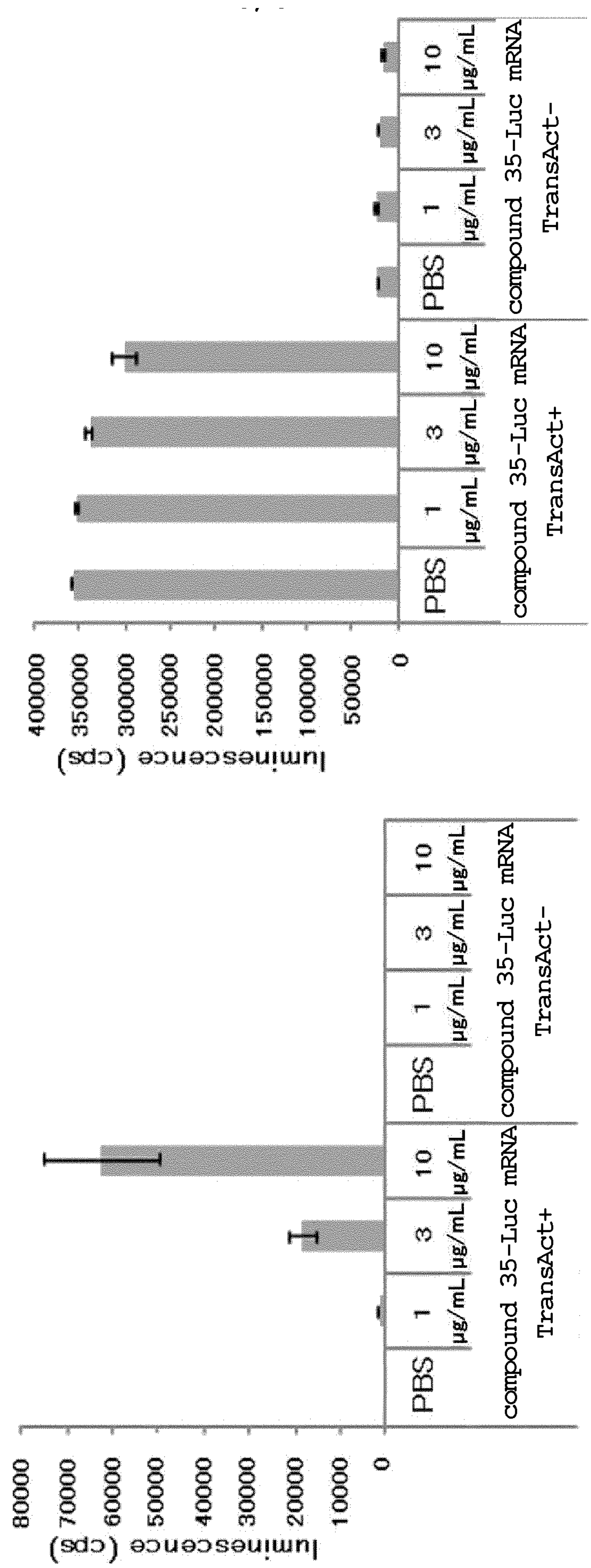
luminescence (cps)
400000
350000
300000
250000
200000
150000
100000
50000
0
PBS
1 μg/mL
3 μg/mL
10 μg/mL
compound 35-Luc mRNA
TransAct+
TransAct-
luminescence (cps)
80000
70000
60000
50000
40000
30000
20000
10000
0

## INTERNATIONAL SEARCH REPORT

International application No. PCT/JP2019/040937

A. CLASSIFICATION OF SUBJECT MATTER
Int.Cl. See extra sheet

According to International Patent Classification (IPC) or to both national classification and IPC

B. FIELDS SEARCHED

Minimum documentation searched (classification system followed by classification symbols)
Int.Cl. C12N15/88, A61K9/127, A61K9/14, A61K31/7088, A61K35/17, A61K39/395, A61K45/00, A61K47/68, A61K48/00, A61P43/00, C12N5/0783, A61P35/00, C07K16/28, C12N15/113, C12N15/12

Documentation searched other than minimum documentation to the extent that such documents are included in the fields searched

| | |
|---|---|
| Published examined utility model applications of Japan | 1922-1996 |
| Published unexamined utility model applications of Japan | 1971-2019 |
| Registered utility model specifications of Japan | 1996-2019 |
| Published registered utility model applications of Japan | 1994-2019 |

Electronic data base consulted during the international search (name of data base and, where practicable, search terms used)
JSTPlus/JMEDPlus/JST7580 (JDreamIII)
CAplus/MEDLINE/EMBASE/BIOSIS/WPIDS (STN)

C. DOCUMENTS CONSIDERED TO BE RELEVANT

| Category* | Citation of document, with indication, where appropriate, of the relevant passages | Relevant to claim No. |
|---|---|---|
| X<br>-<br>Y | US 2016/0145348 A1 (FRED HUTCHINSON CANCER RESEARCH CENTER) 26 May 2016, claims, examples & EP 2970985 A1 | 1-13, 28-29<br>-<br>19, 26 |
| X<br>-<br>Y | US 2017/0296676 A1 (FRED HUTCHINSON CANCER RESEARCH CENTER) 19 October 2017, claims, examples, paragraph [0361] & EP 3442585 A1 | 1-13, 28-29<br>-<br>19, 26 |

☒ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 12 December 2019 (12.12.2019) | 24 December 2019 (24.12.2019) |

| Name and mailing address of the ISA/ | Authorized officer |
|---|---|
| Japan Patent Office<br>3-4-3, Kasumigaseki, Chiyoda-ku,<br>Tokyo 100-8915, Japan | Telephone No. |

Form PCT/ISA/210 (second sheet) (January 2015)

**INTERNATIONAL SEARCH REPORT**

International application No.
PCT/JP2019/040937

C (Continuation). DOCUMENTS CONSIDERED TO BE RELEVANT

| Category* | Citation of document, with indication, where appropriate, of the relevant passages | Relevant to claim No. |
|---|---|---|
| X<br>-<br>Y | JP 2017-125034 A (MASSACHUSETTS INSTITUTE OF TECHNOLOGY et al.) 20 July 2017, claims, paragraphs [0011], [0224] & WO 2009/051837 A2, claims, paragraphs [0010], [0240] & US 2010/0092425 A1 & EP 2217269 A2 | 14-18, 20-25, 27-32<br>19, 26 |

Form PCT/ISA/210 (continuation of second sheet) (January 2015)

**INTERNATIONAL SEARCH REPORT**

International application No.
PCT/JP2019/040937

**Box No. II Observations where certain claims were found unsearchable (Continuation of item 2 of first sheet)**

This international search report has not been established in respect of certain claims under Article 17(2)(a) for the following reasons:

1. ☐ Claims Nos.:
because they relate to subject matter not required to be searched by this Authority, namely:

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

**Box No. III Observations where unity of invention is lacking (Continuation of item 3 of first sheet)**

This International Searching Authority found multiple inventions in this international application, as follows:
See extra sheet

1. ☐ As all required additional search fees were timely paid by the applicant, this international search report covers all searchable claims.
2. ☒ As all searchable claims could be searched without effort justifying additional fees, this Authority did not invite payment of additional fees.
3. ☐ As only some of the required additional search fees were timely paid by the applicant, this international search report covers only those claims for which fees were paid, specifically claims Nos.:

4. ☐ No required additional search fees were timely paid by the applicant. Consequently, this international search report is restricted to the invention first mentioned in the claims; it is covered by claims Nos.:

**Remark on Protest**

☐ The additional search fees were accompanied by the applicant's protest and, where applicable, the payment of a protest fee.

☐ The additional search fees were accompanied by the applicant's protest but the applicable protest fee was not paid within the time limit specified in the invitation.

☐ No protest accompanied the payment of additional search fees.

Form PCT/ISA/210 (continuation of first sheet (2)) (January 2015)

**INTERNATIONAL SEARCH REPORT**

International application No.
PCT/JP2019/040937

CLASSIFICATION OF SUBJECT MATTER
C12N15/88(2006.01)i, A61K9/127(2006.01)i, A61K9/14(2006.01)i, A61K31/7088(2006.01)i A61K35/17(2015.01)i, A61K39/395(2006.01)i, A61K45/00(2006.01)i, A61K47/68(2017.01)i A61K48/00(2006.01)i, A61P43/00(2006.01)i, C12N5/0783(2010.01)i, A61P35/00(2006.01)n C07K16/28(2006.01)n, C12N15/113(2010.01)n, C12N15/12(2006.01)n

Form PCT/ISA/210 (extra sheet) (January 2015)

INTERNATIONAL SEARCH REPORT

International application No.
PCT/JP2019/040937

<Continuation of Box No. III>

This application includes two or more inventions.

(Invention 1) Claims 1-13

Document 1 discloses producing chimeric antigen receptor (CAR)-expressing cells by using lipid-coated synthetic nanoparticles containing a polynucleotide targeting the CAR, and document 2 discloses a method for preparing selected cell populations derived from the hematopoietic system, the method including a step for modifying T cells etc. using nanoparticles coated with liposomes. Claims 1-13 lack novelty in light of document 1 or 2, and thus do not have a special technical feature.

(Invention 2) Claims 14-32

Claim 14 shares the technical feature of a "cell population containing T cells" with claim 1 classified as invention 1. However, said technical feature does not make a contribution over the prior art in light of the disclosures of documents 1 and 2, and thus cannot be said to be a special technical feature. In addition, there do not exist other identical or corresponding special technical features between these inventions.

In addition, claims 14-32 are not dependent on claim 1. In addition, claims 14-32 are not substantially identical or equivalent to any of the claims classified as invention 1.

Thus, claims 14-32 cannot be classified as invention 1.

In addition, claims 14-32 have the special technical feature of a "method for delivering nucleic acids into T cells, the method including simultaneously contacting a cell population containing T cells with at least one T cell activating ligand and a nucleic acid delivery carrier containing a nucleic acid therein and having no T cell activating ligand added to the surface," and are thus classified as invention 2.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 20170296676 A
- US 20160145348 A
- WO 2016189532 A
- WO 2016021683 A
- US 6352694 B
- US 20140087462 A
- WO 2015011633 A
- WO 2011153493 A
- WO 2013126803 A
- WO 2010054401 A
- WO 2010042877 A
- WO 2016104580 A
- WO 2015005253 A
- WO 2014007398 A
- WO 2017117528 A
- WO 2017075531 A
- WO 201700414 A
- WO 2015199952 A
- US 20150239834 A
- WO 2019131839 A
- US 9404127 B
- WO 2008153029 A
- WO 2018197069 A
- WO 2019124629 A


### Non-patent literature cited in the description


- *Nature Nanotechnology,* 2017, vol. 12, 813-820
- *ACS Nano,* 2015, vol. 9 (7), 6706-6716
- **DONG et al.** *Proc Natl Acad Sci U S A.,* 15 April 2014, vol. 111 (15), 5753
- **LOVE KT et al.** *Proc Natl Acad Sci U S A.,* 25 May 2010, vol. 107 (21), 9915
- Jikken Kagaku Koza (Encyclopedia of Experimental Chemistry in English). vol. 13-19
- Shin Jikken Kagaku Koza (New Encyclopedia of Experimental Chemistry in English). vol. 14-15
- Fine Organic Chemistry. **L. F. TIETZE ; TH. EICHER.** Revised. Nankodo
- Organic Name Reactions. **HIDEO TOGO.** The Reaction Mechanism and Essence, Revised. Kodansha
- Organic Syntheses Collective. John Wiley & Sons, Inc, vol. I-VII
- **JIE JACK LI.** Modern Organic Synthesis in the Laboratory: A Collection of Standard Experimental Procedures. Oxford University Press
- Comprehensive Heterocyclic Chemistry III. Elsevier Japan KK, vol. 1-14
- **KIYOSHI TOMIOKA.** Strategic Applications of Named Reactions in Organic Synthesis. Kagaku-Dojin Publishing
- Comprehensive Organic Transformations. VCH Publishers, Inc, 1989
- **THEODORA W. GREENE ; PETER G. M. WUTS.** Protective Groups in Organic Synthesis. Wiley-Interscience, 2007
- **P.J. KOCIENSKI.** Protecting Groups. Thieme, 2004
- **ELBASHIR et al.** *Genes Dev.,* 2001, vol. 15, 188-200